# EUROPEAN PATENT SPECIFICATION

(11) **EP 4 196 575 B1**
(45) Date of publication and mention of the grant of the patent: **14.10.2026**
(21) Application number: 21766397.0
(22) Date of filing: 13.08.2021
(51) Int. Cl.: C12N 9/16, C12N 15/82, A23K 10/30, A23K 20/147

(54) **PHYTASE VARIANTS AND POLYNUCLEOTIDES ENCODING SAME**
PHYTASEVARIANTEN UND POLYNUKLEOTIDE ZUR CODIERUNG DAVON
VARIANTS DE PHYTASE ET POLYNUCLÉOTIDES LES CODANT

(30) Priority: 13.08.2020 EP 20190917; 12.10.2020 EP 20201328; 18.03.2021 WO PCT/CN2021/081613; 07.05.2021 EP 21172706
(43) Date of publication of application: 21.06.2023
(73) Proprietor: Novozymes A/S, 2880 Bagsvaerd (DK)
(72) Inventor: VIND, Jesper, 2880 Bagsvaerd (DK); SKOV, Lars, Kobberøe, 2880 Bagsvaerd (DK); ZHAI, Hengxiao, 4303 Kaiseraugst (CH); ZHANG, Qian, 4303 Kaiseraugst (CH); SANTIGOSA, Ester, 4303 Kaiseraugst (CH); SORBARA, Jose-Otavio, 4303 Kaiseraugst (CH); SÉON, Aurélia, Anne, Catherine, Charlotte, 4303 Kaiseraugst (CH); WALK, Carrie, Louise, 4303 Kaiseraugst (CH)
(74) Representative: NVS EPO Representatives
(86) International application number: PCT/EP2021/072591
(87) International publication number: WO 2022/034211

(56) References cited:
- WO-A1-2019/228441
- WO-A2-2011/117396
- TAN HAO ET AL: "Enhancing the Thermal Resistance of a Novel Acidobacteria-Derived Phytase by Engineering of Disulfide Bridges", JOURNAL OF MICROBIOLOGY AND BIOTECHNOLOGY, vol. 26, no. 10, 28 October 2016 (2016-10-28), pages 1717 - 1722, XP055866457, ISSN: 1017-7825, Retrieved from the Internet <URL:http://dx.doi.org/10.4014/jmb.1604.04051> DOI: 10.4014/jmb.1604.04051

## Description

### REFERENCE TO A SEQUENCE LISTING

This application contains a Sequence Listing in computer readable form, which is incorporated herein by reference.

### REFERENCE TO ATOMIC COORDINATES

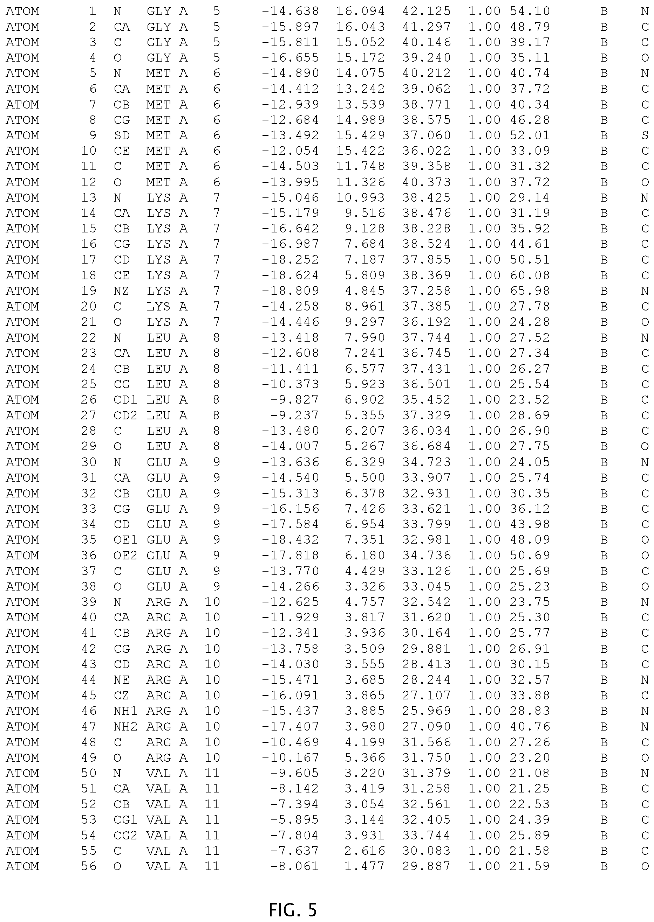

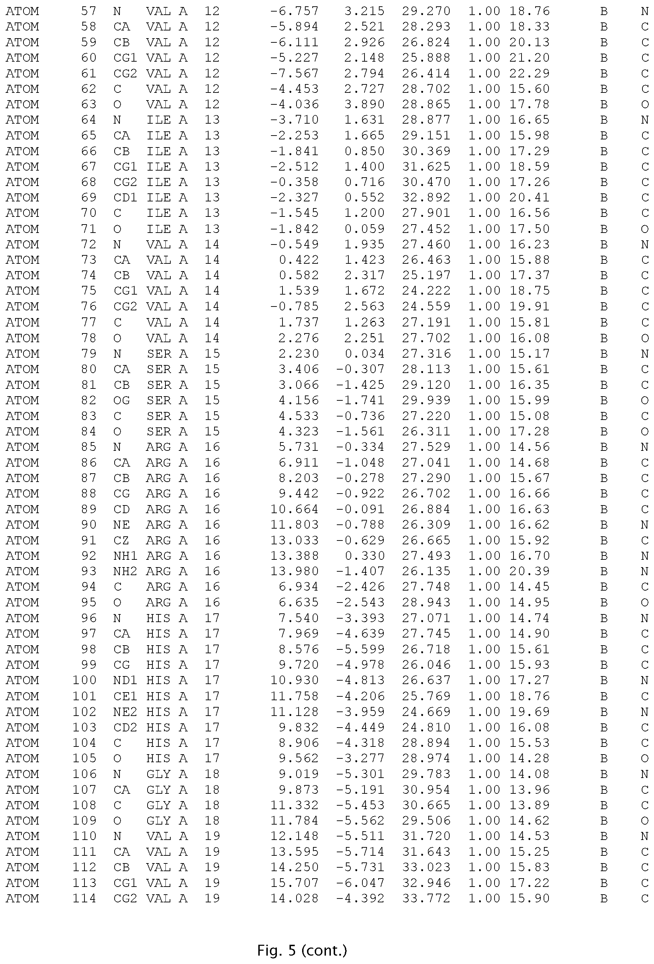

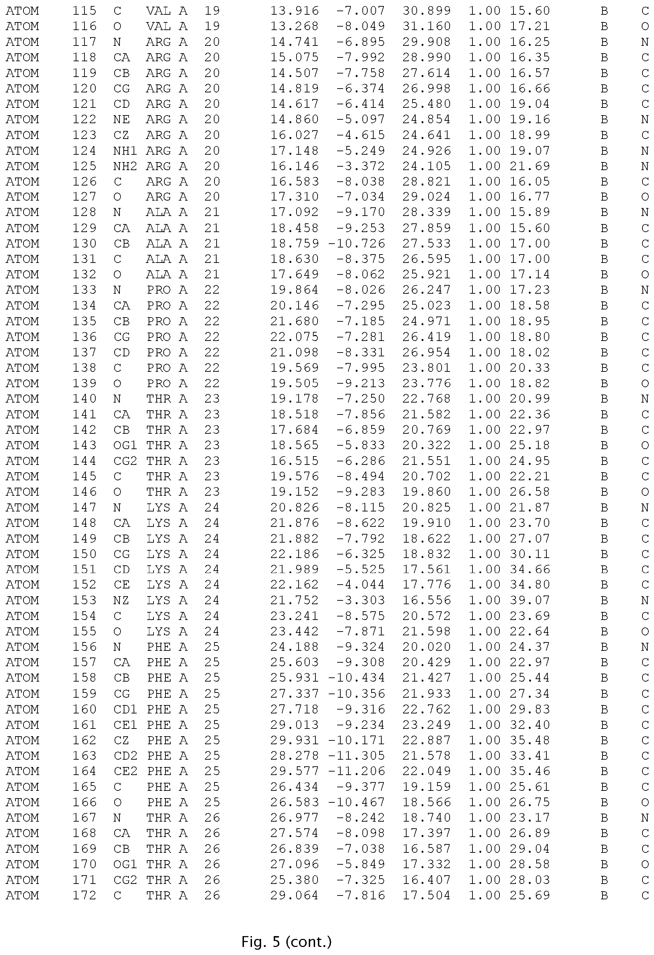

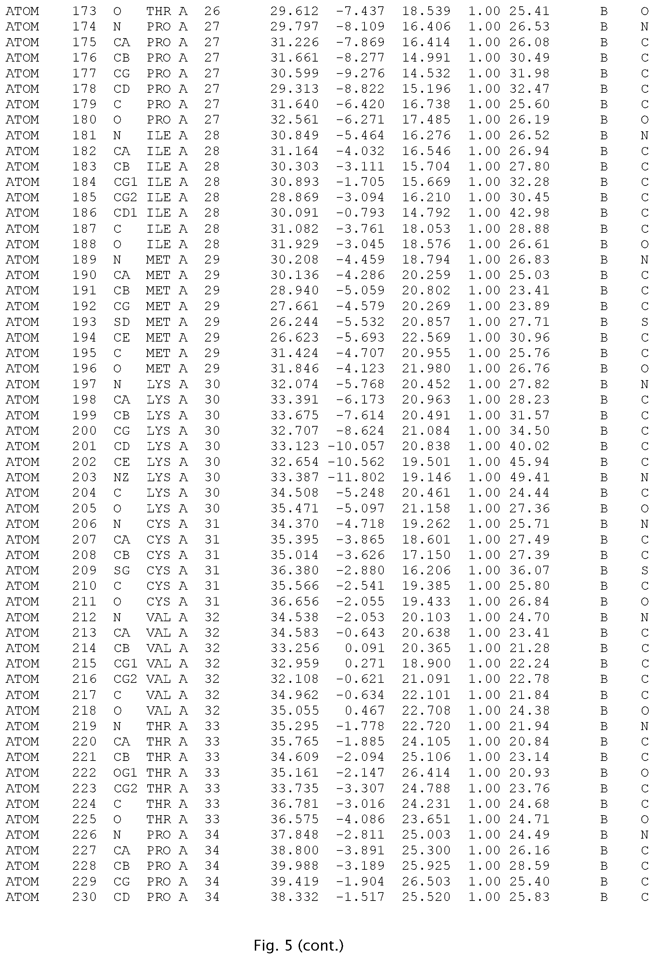

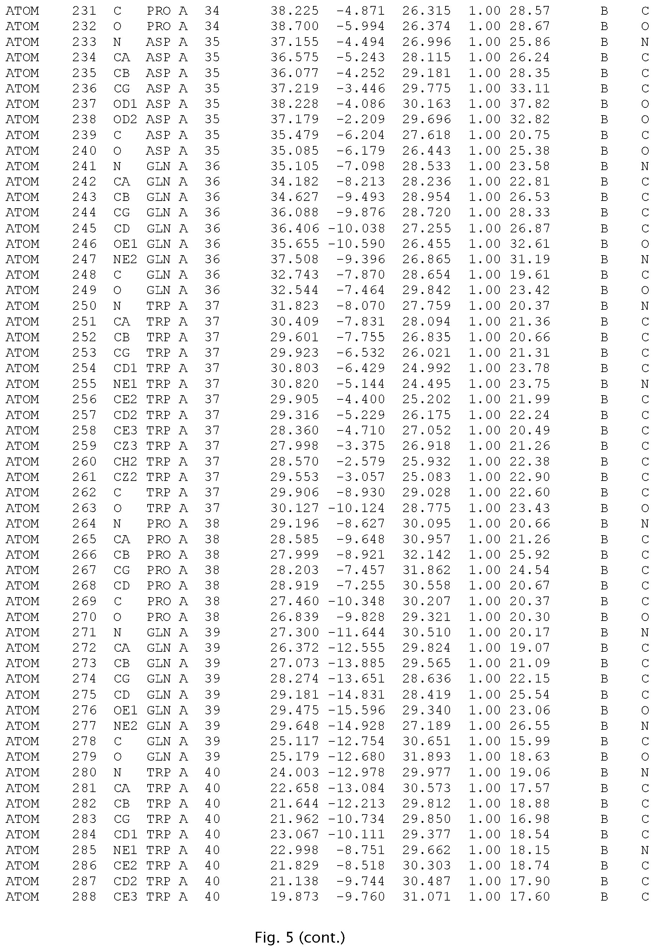

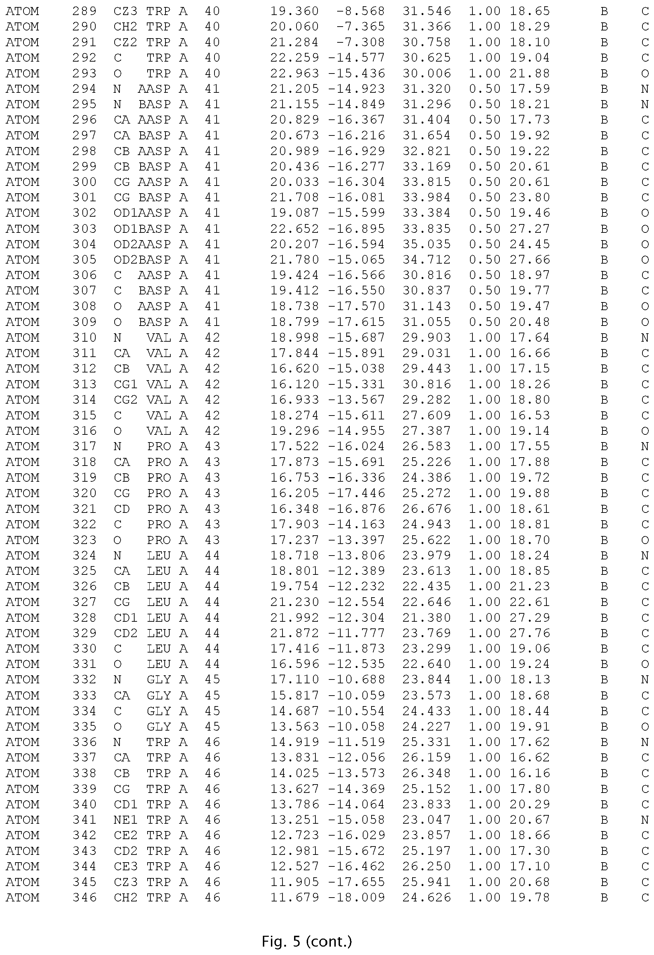

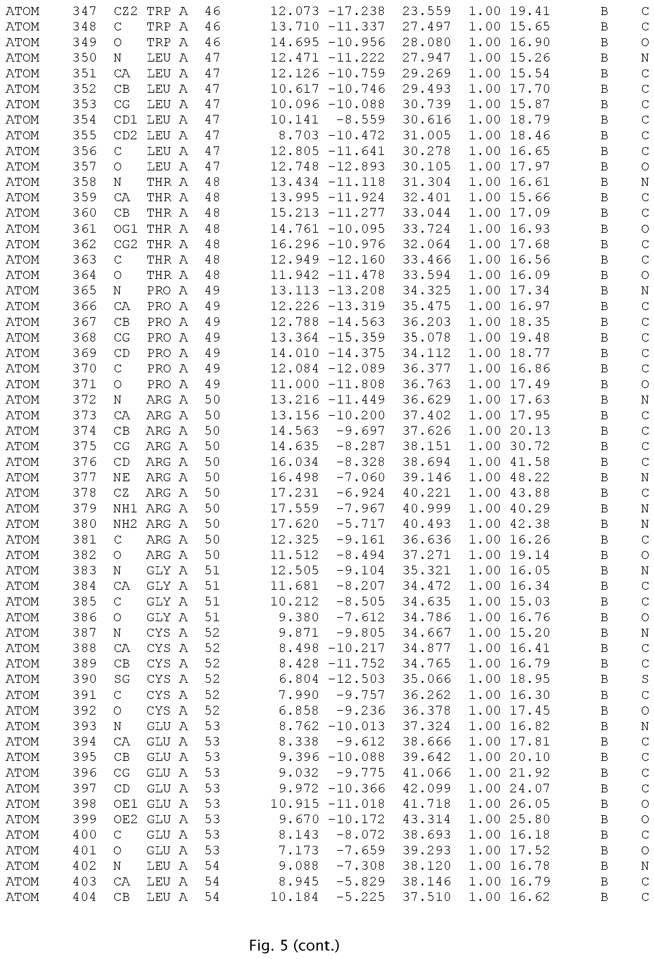

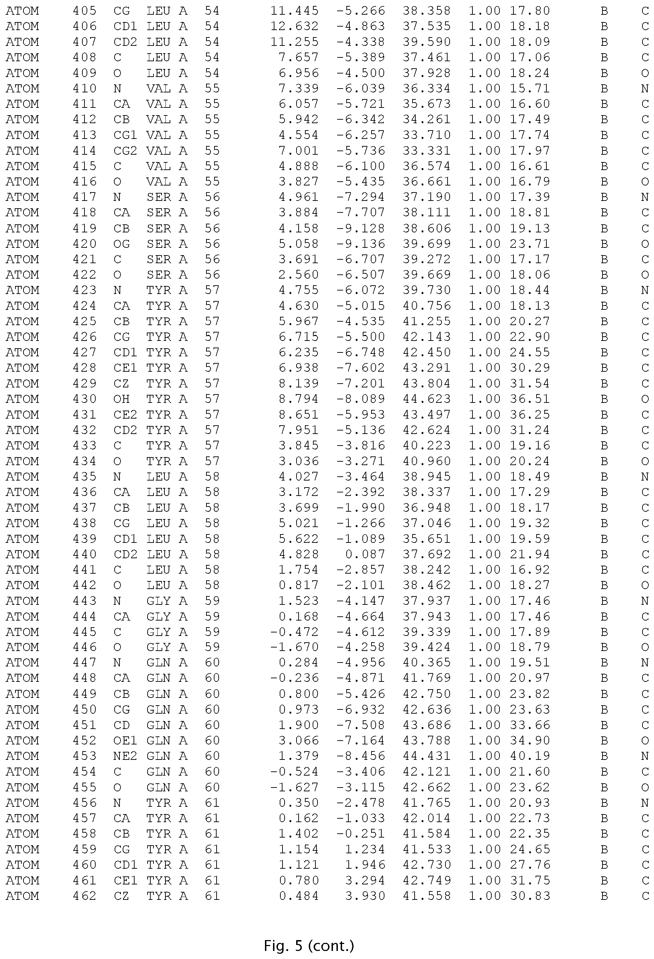

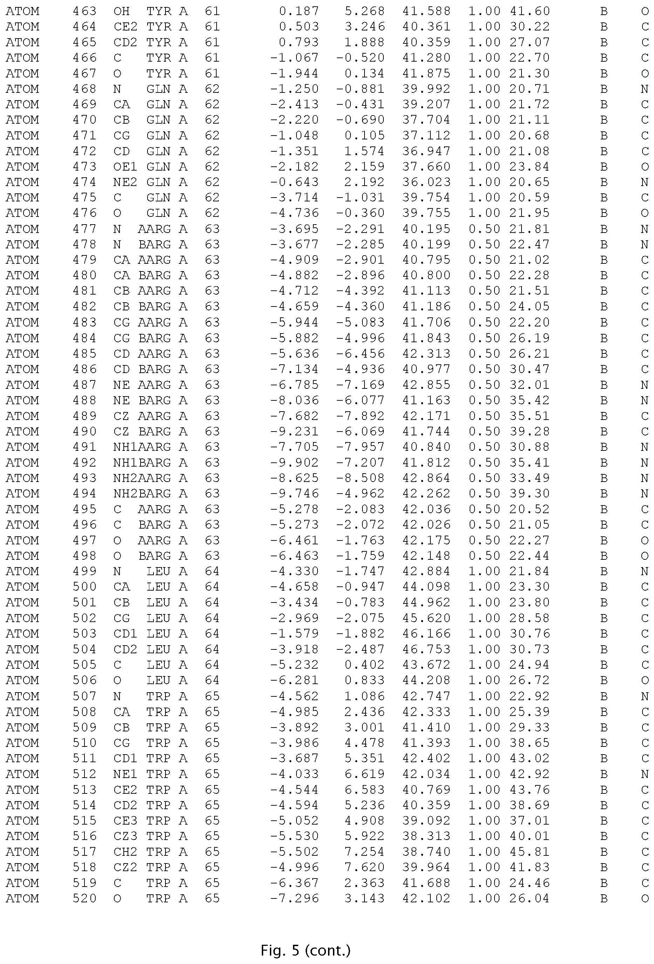

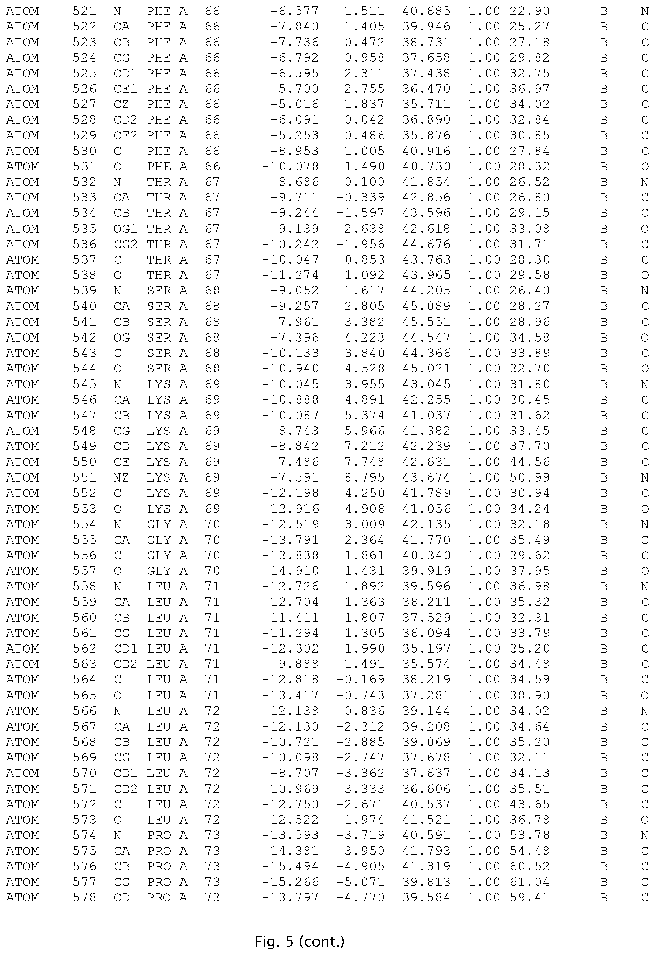

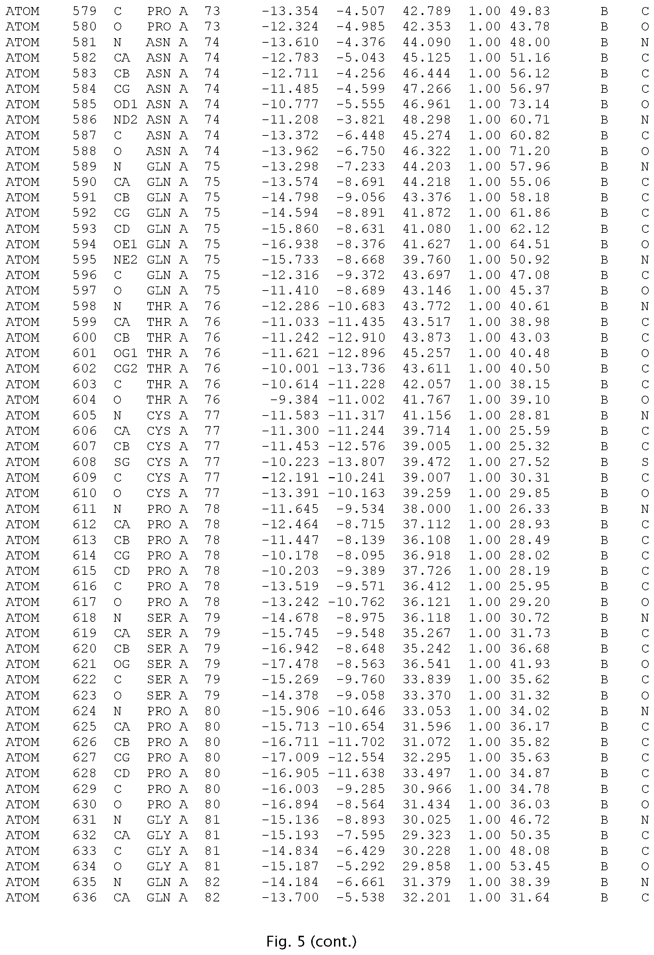

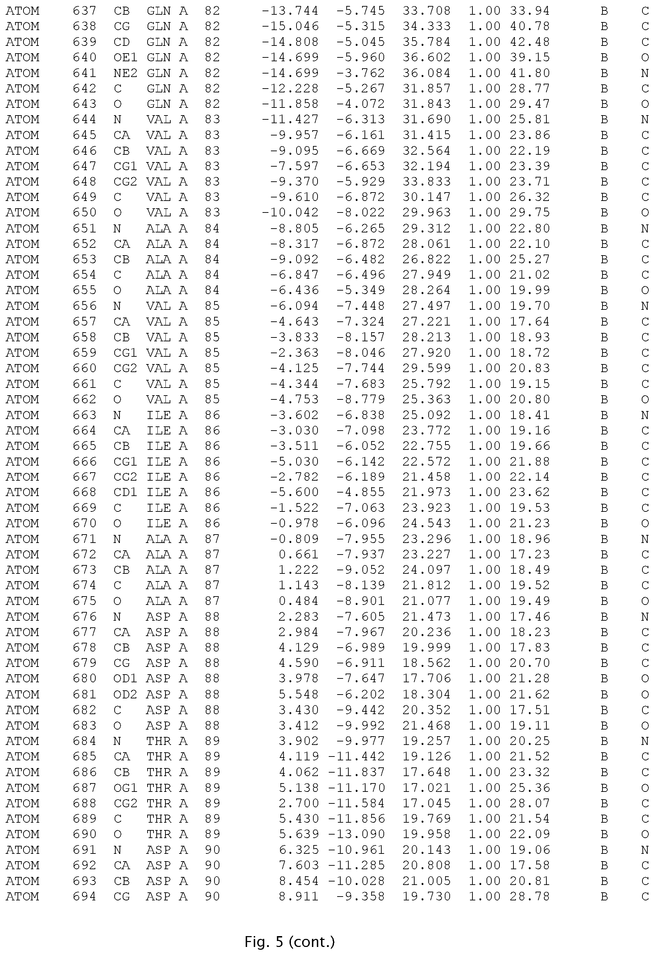

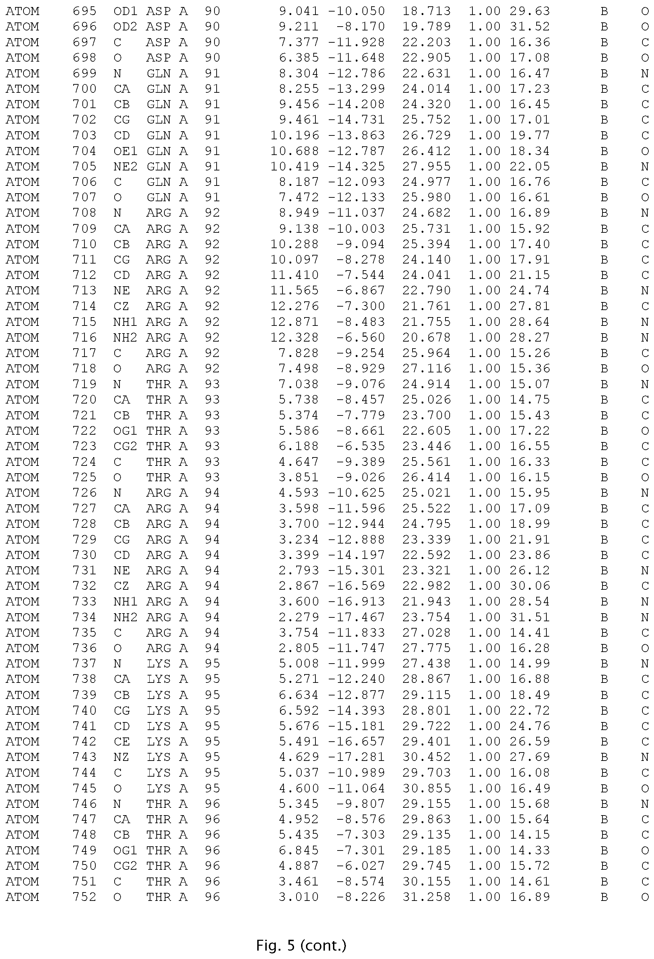

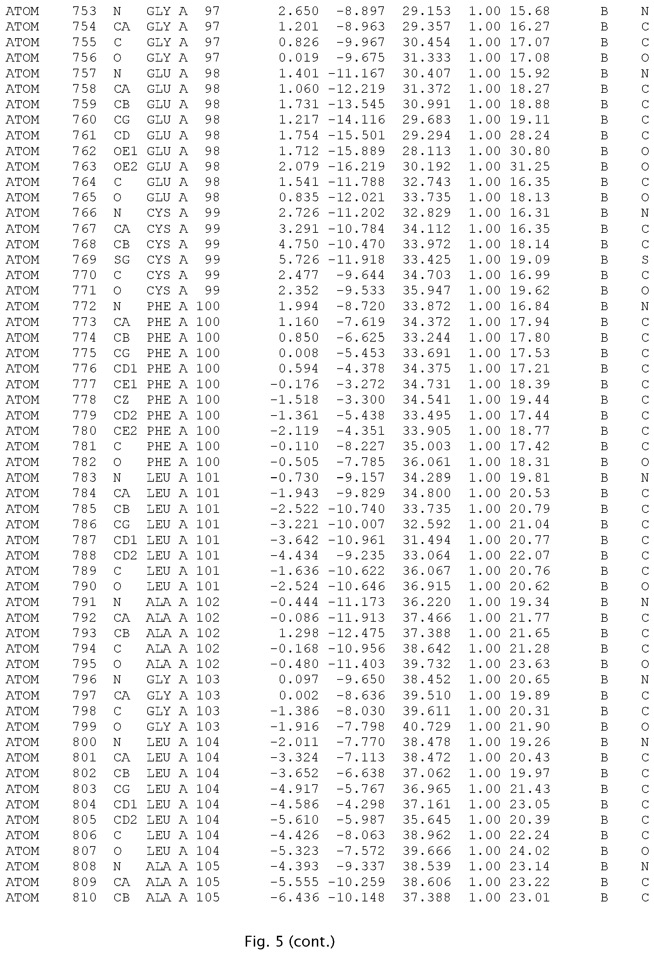

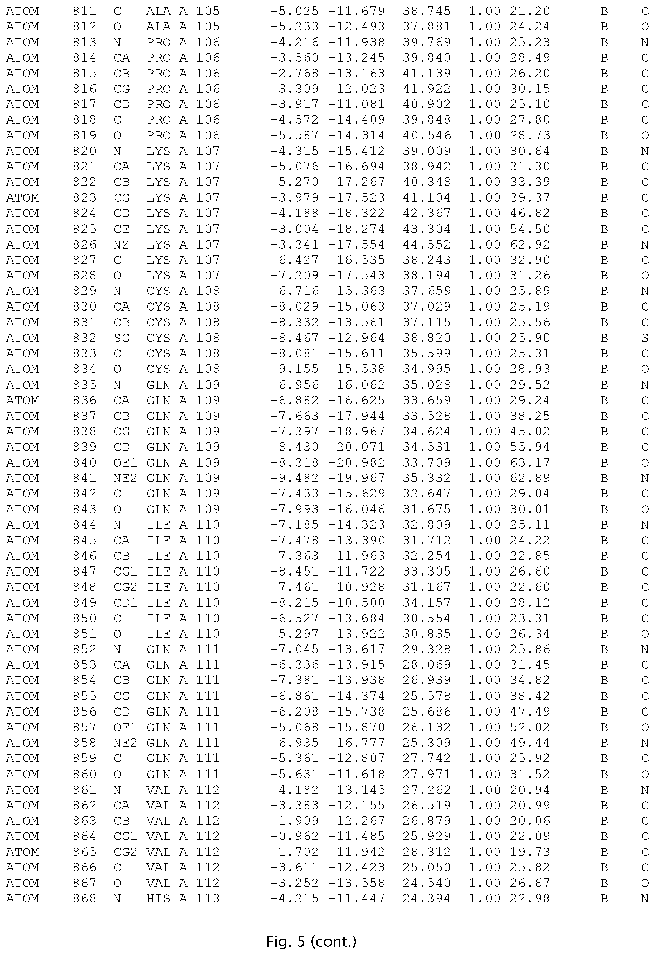

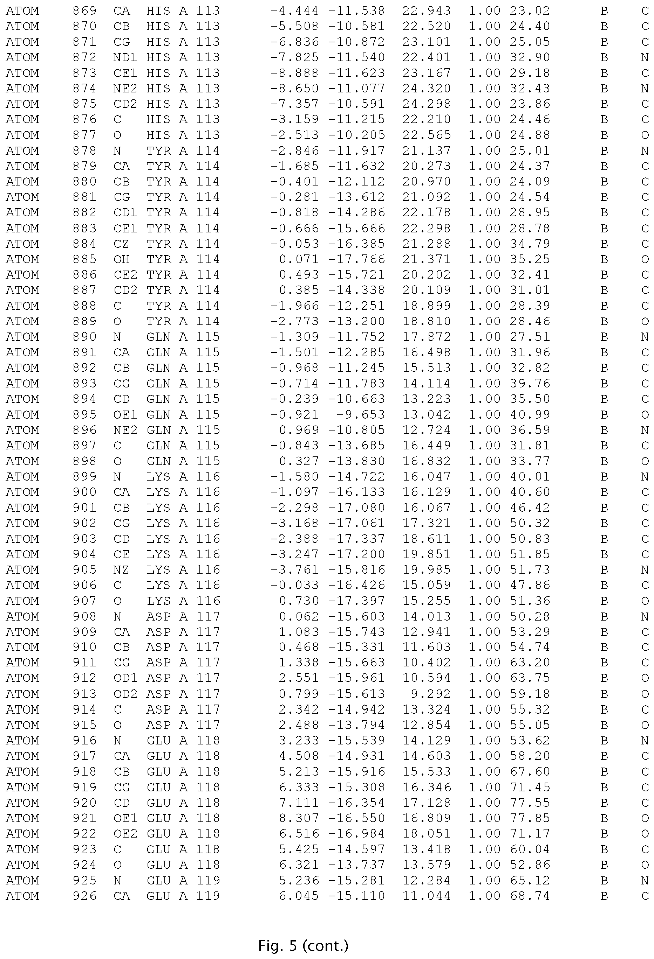

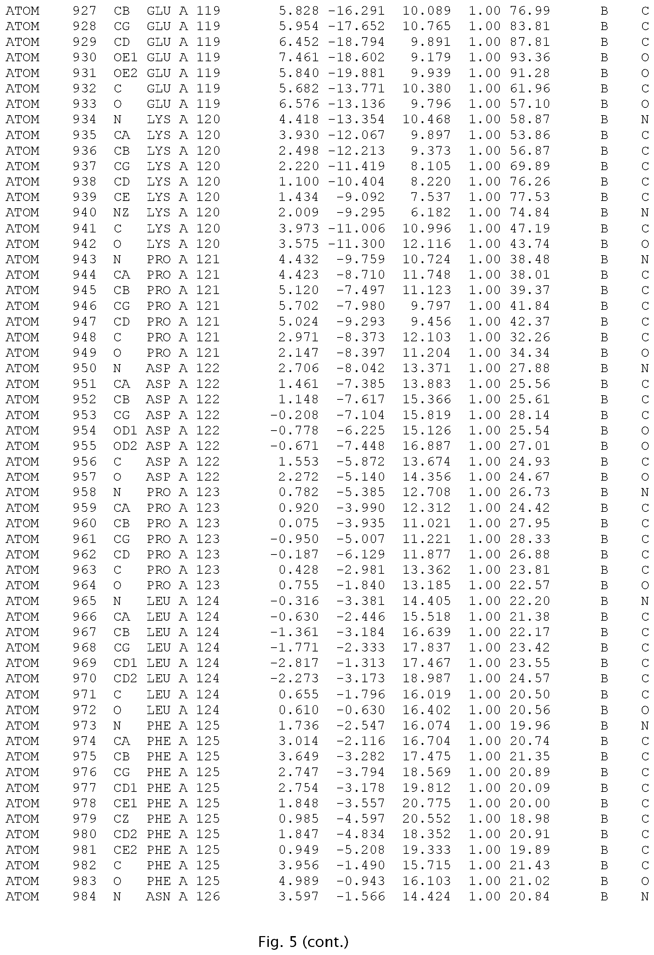

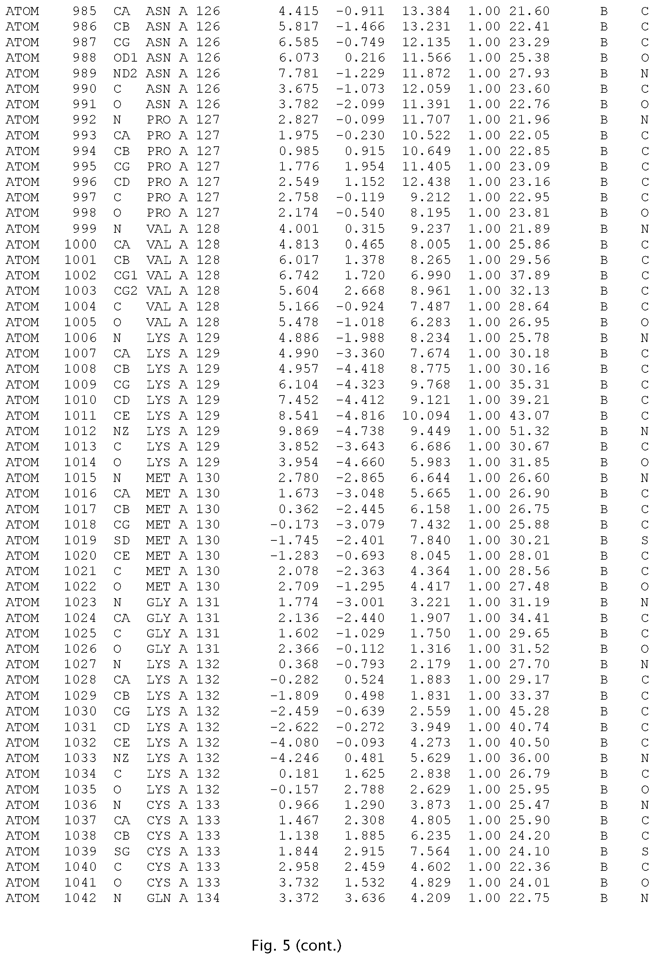

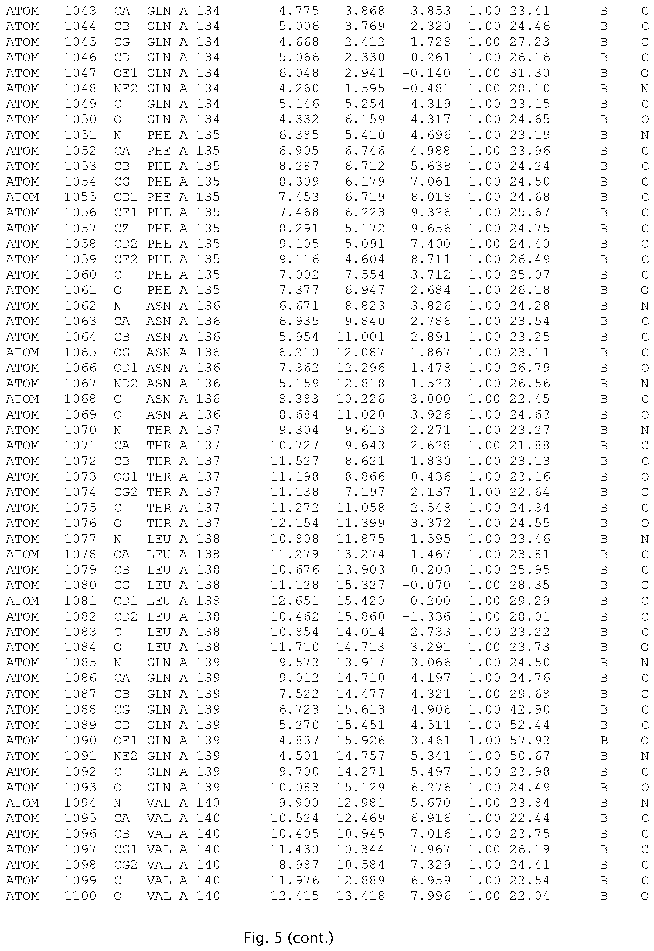

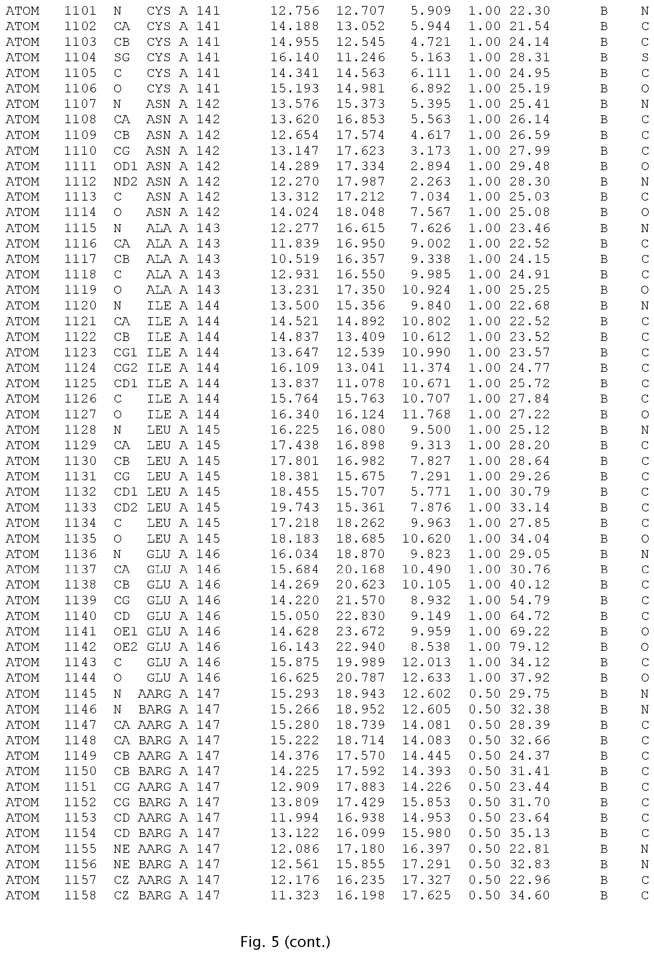

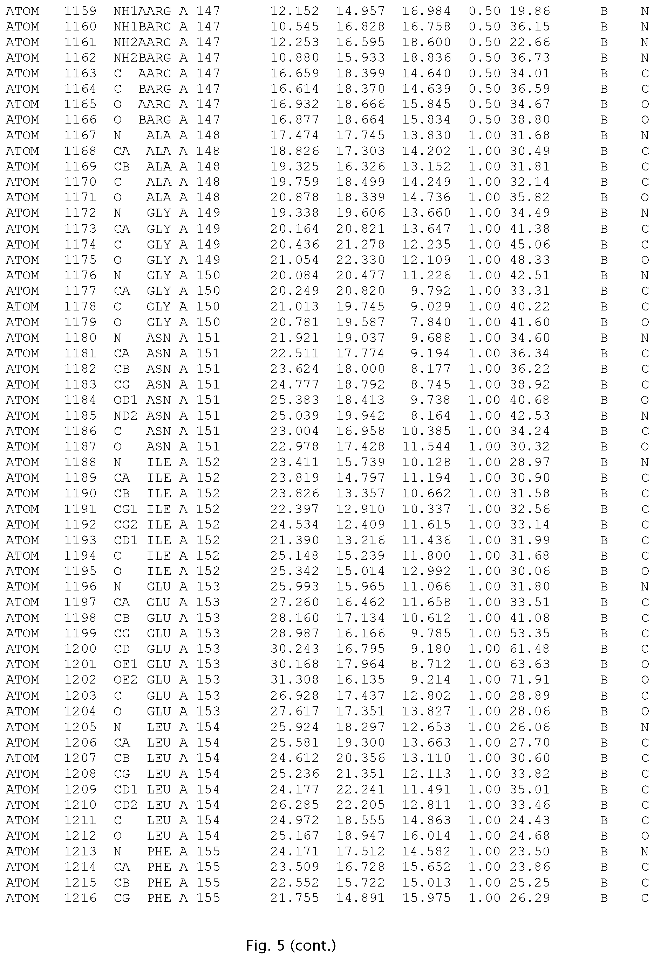

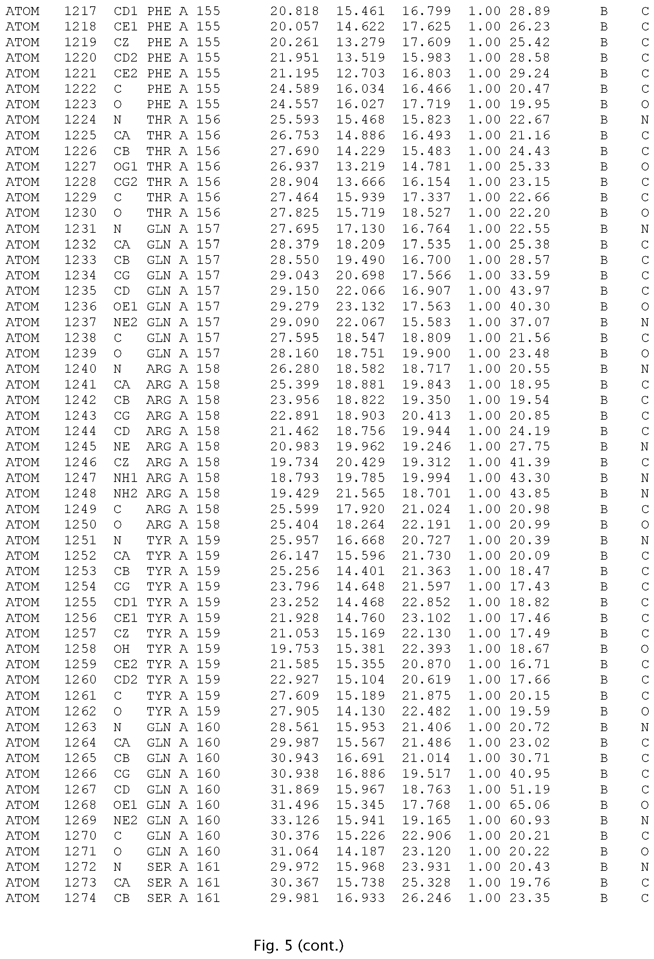

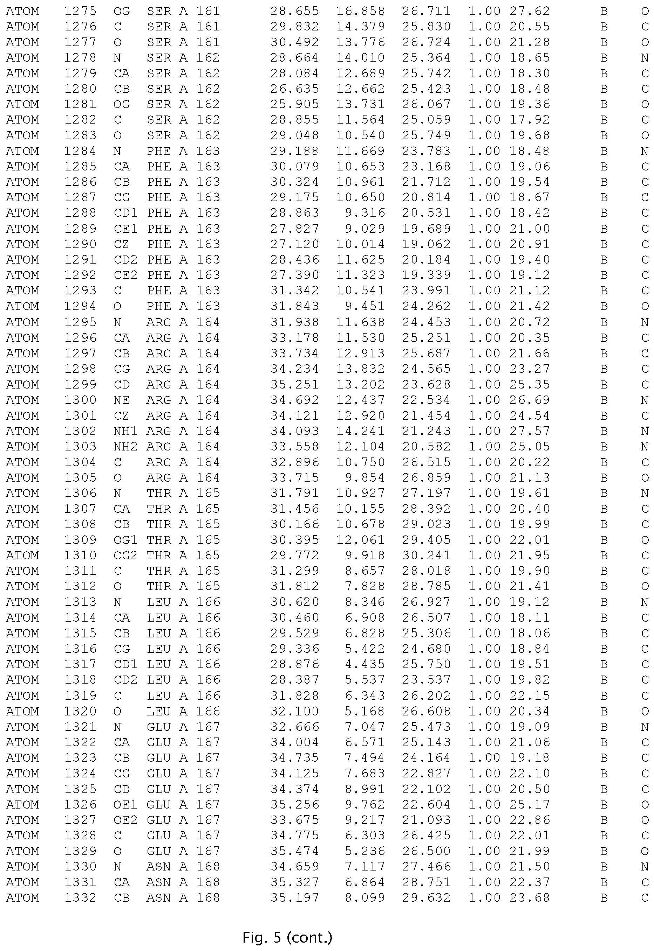

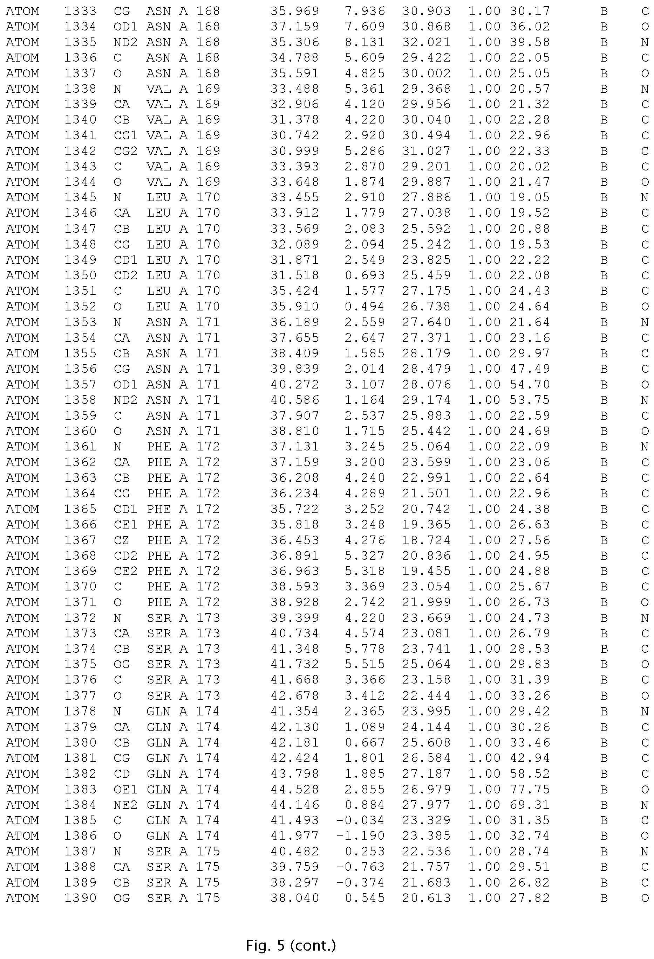

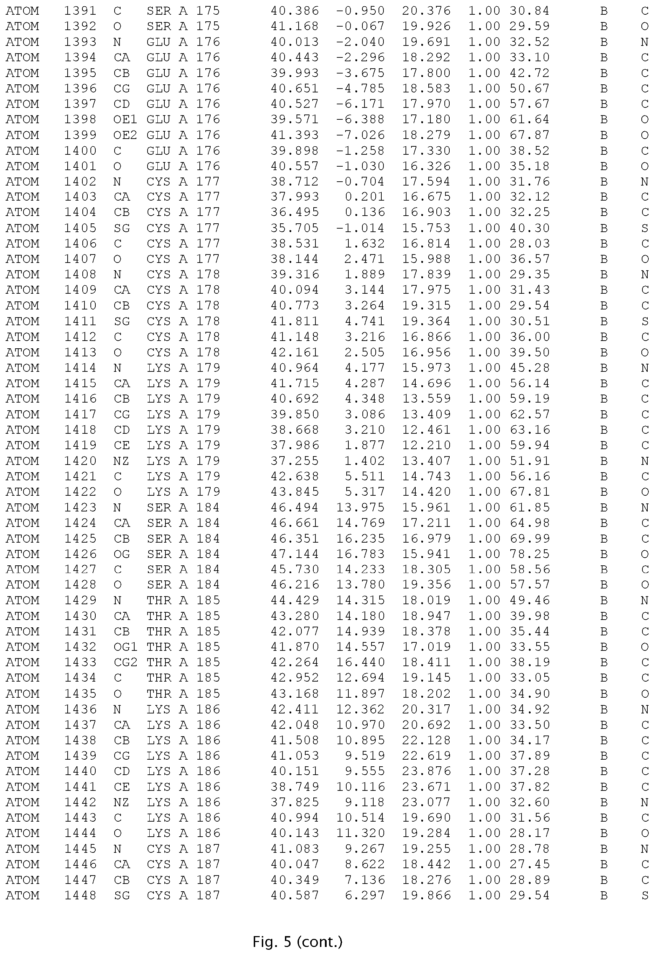

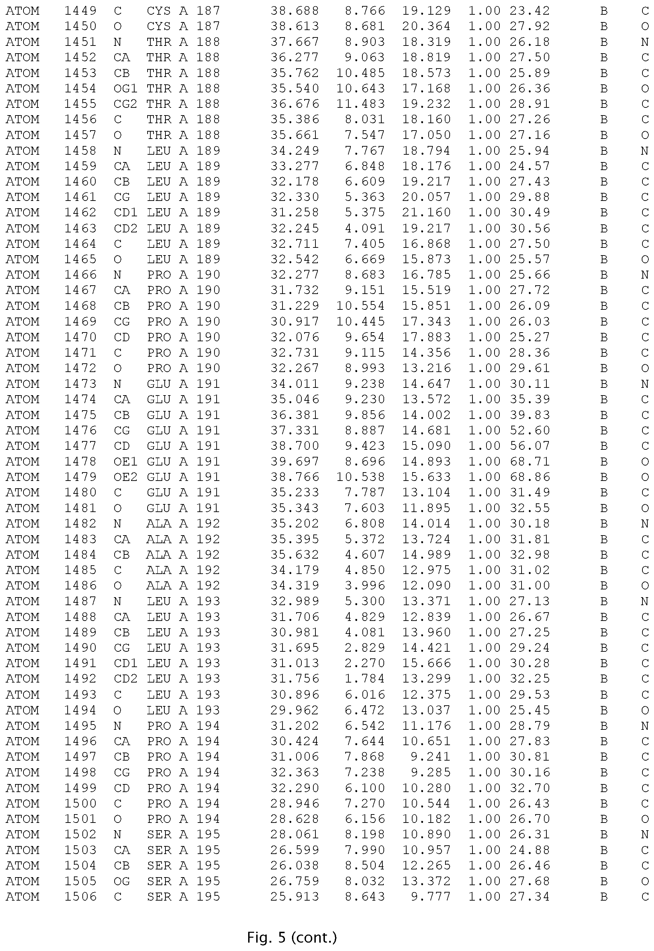

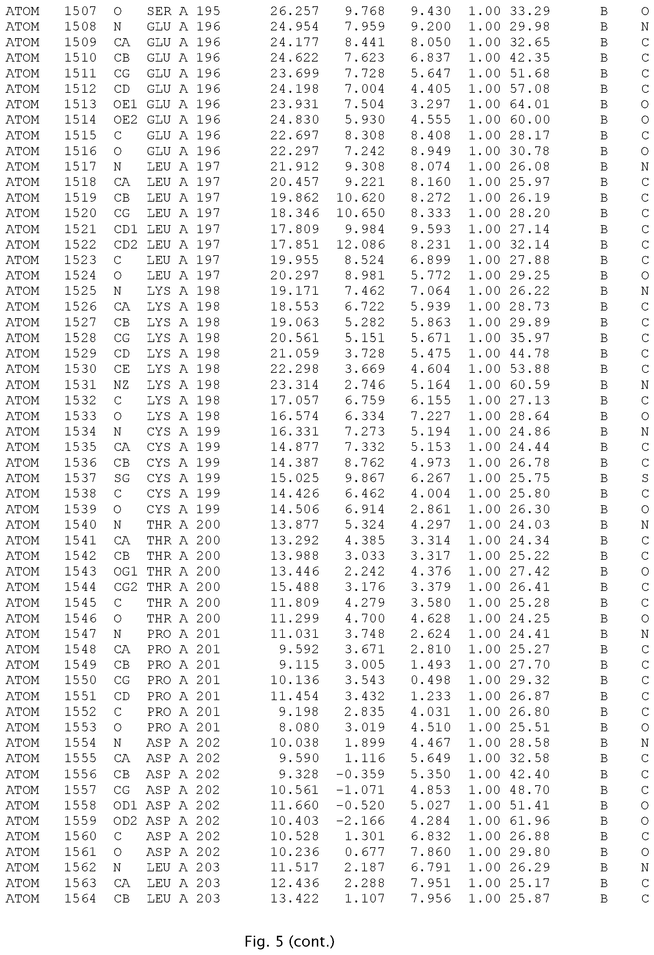

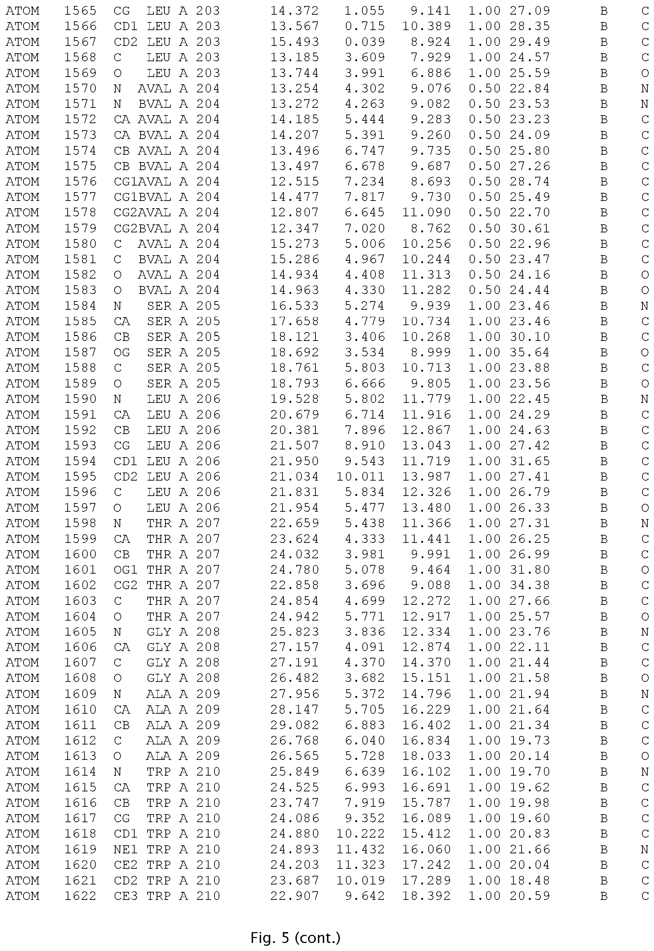

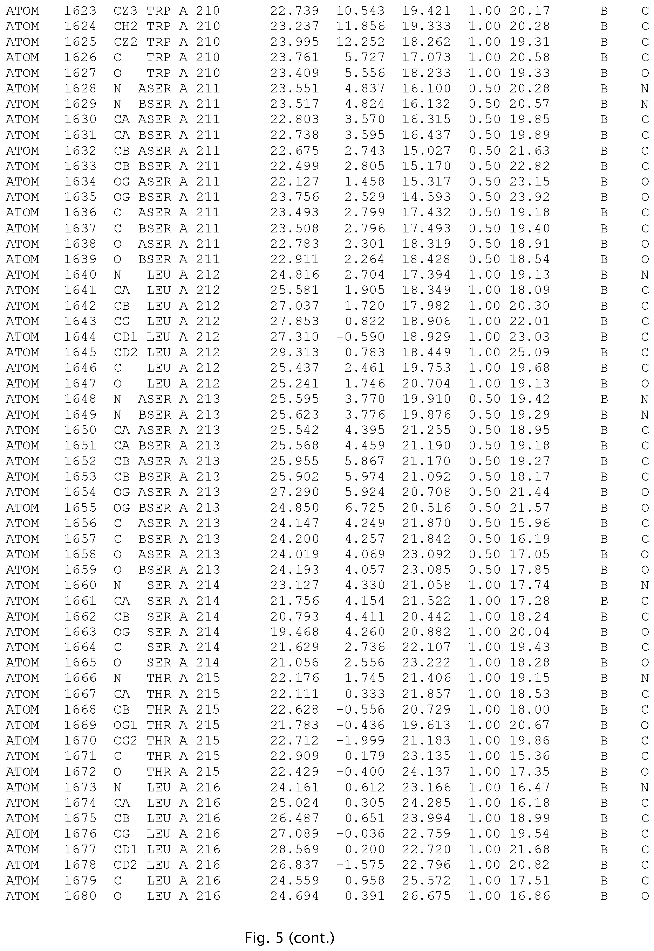

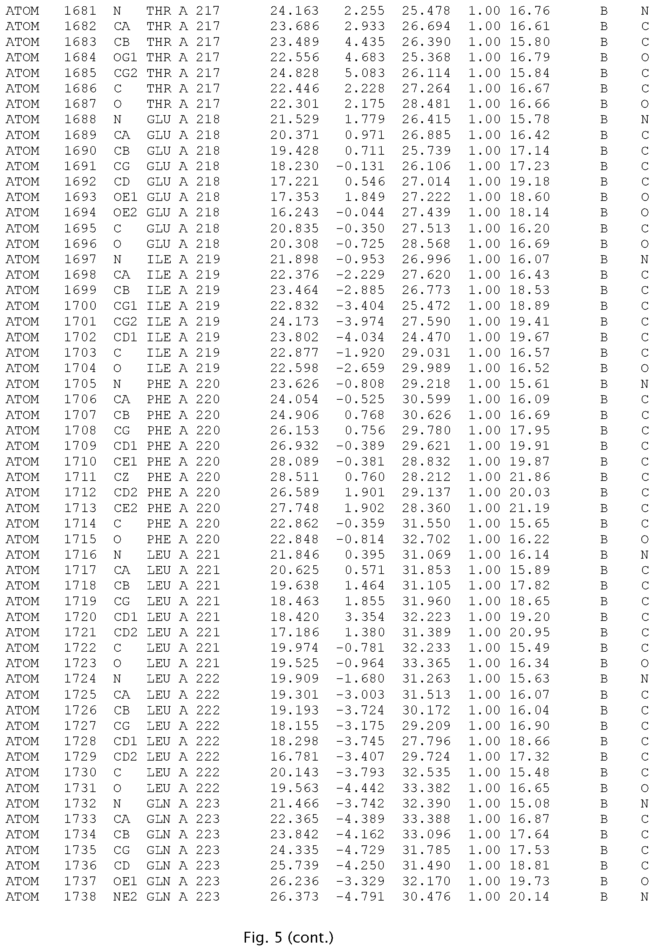

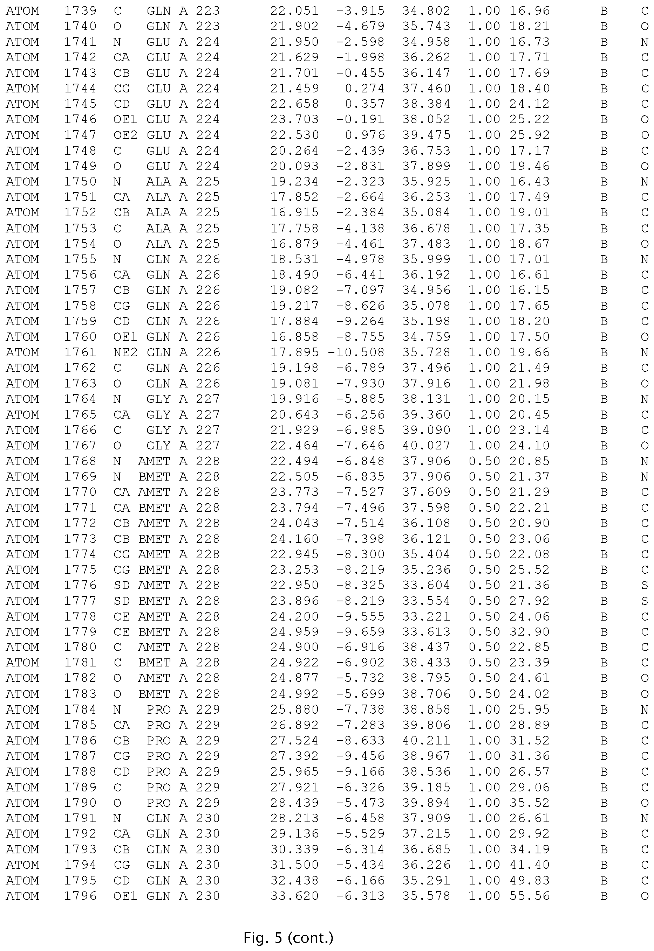

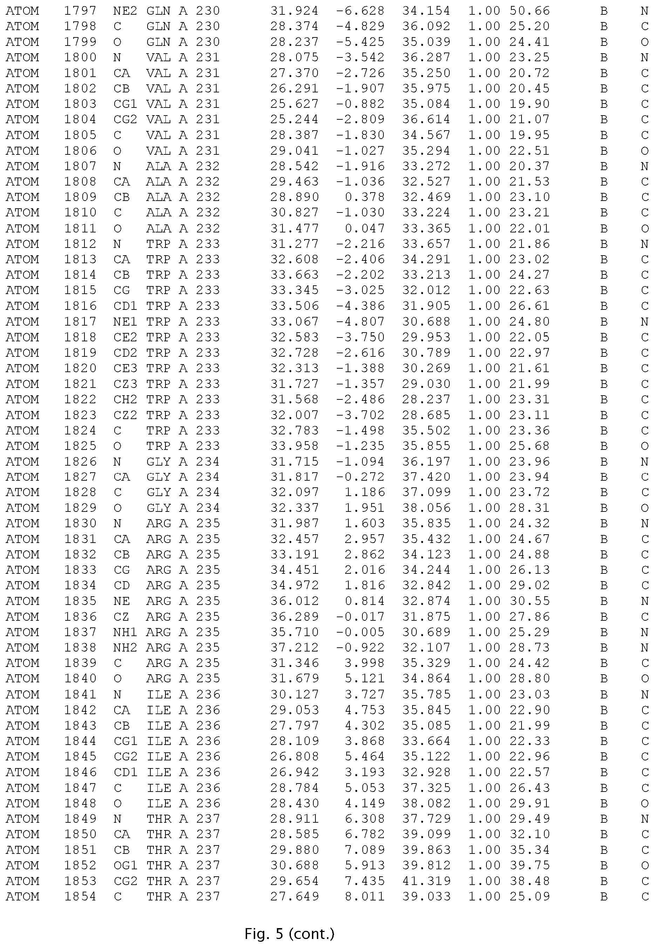

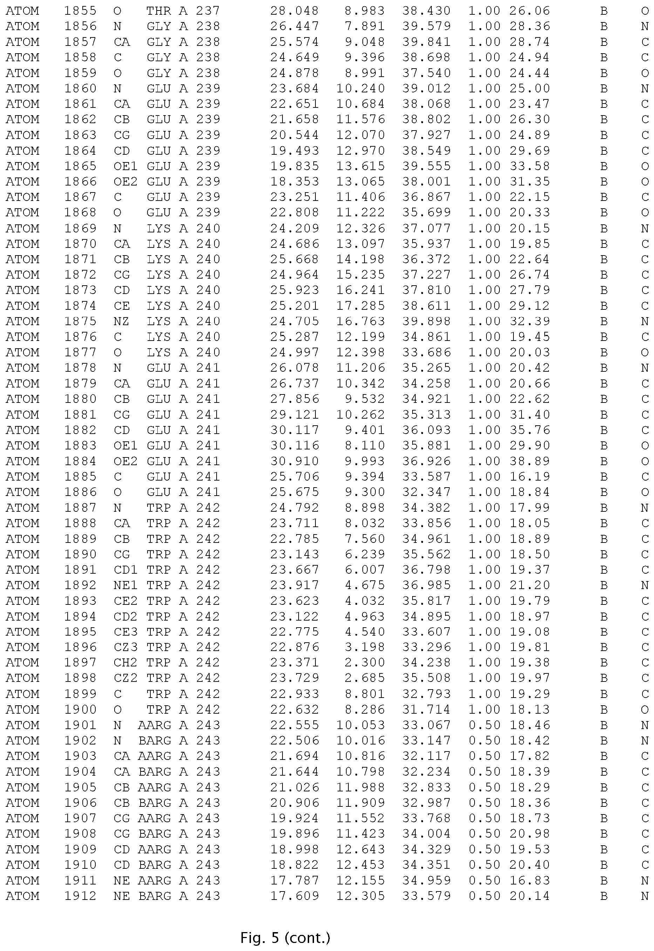

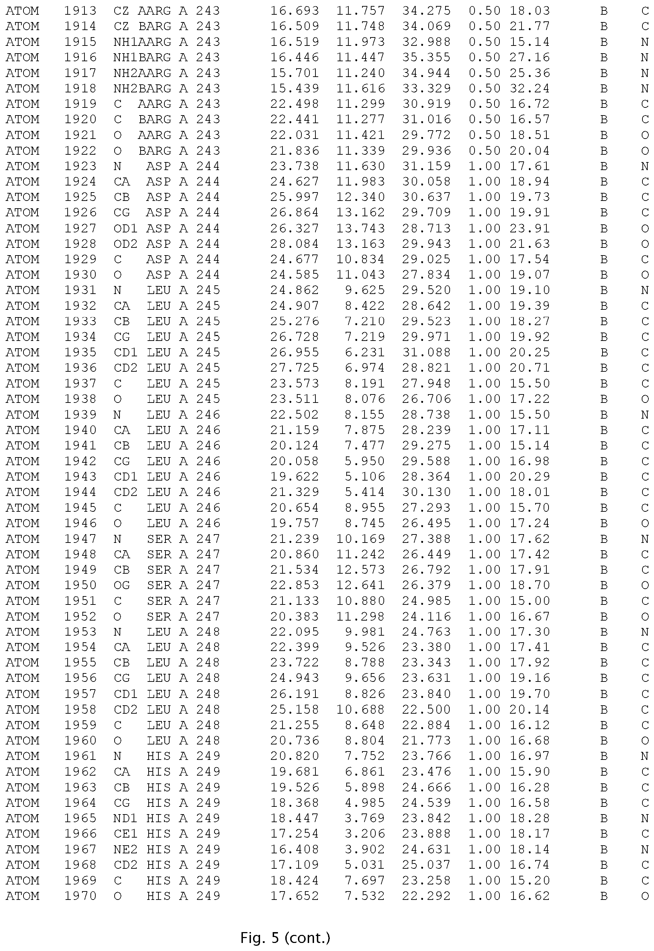

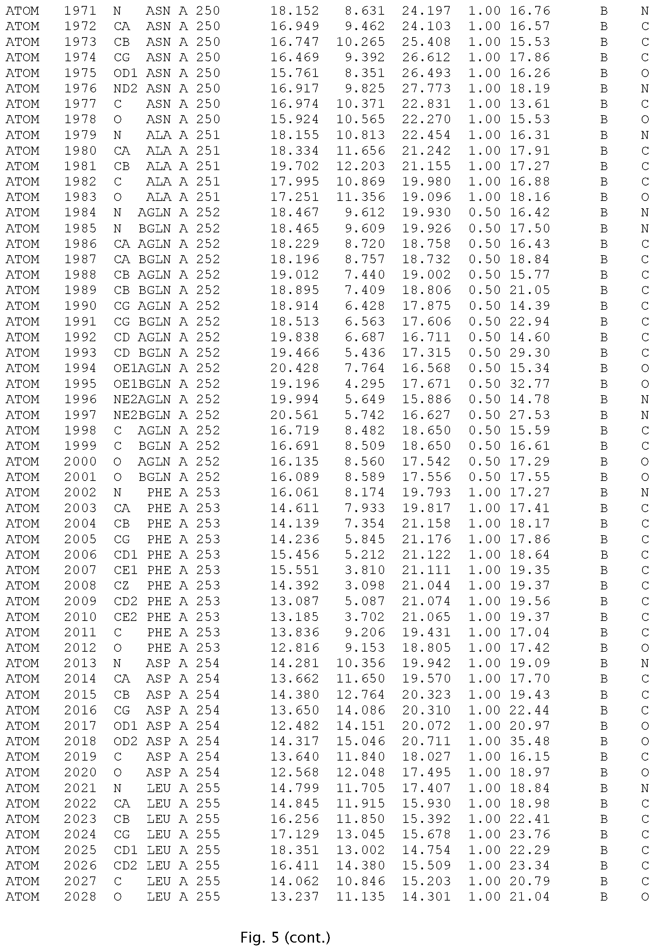

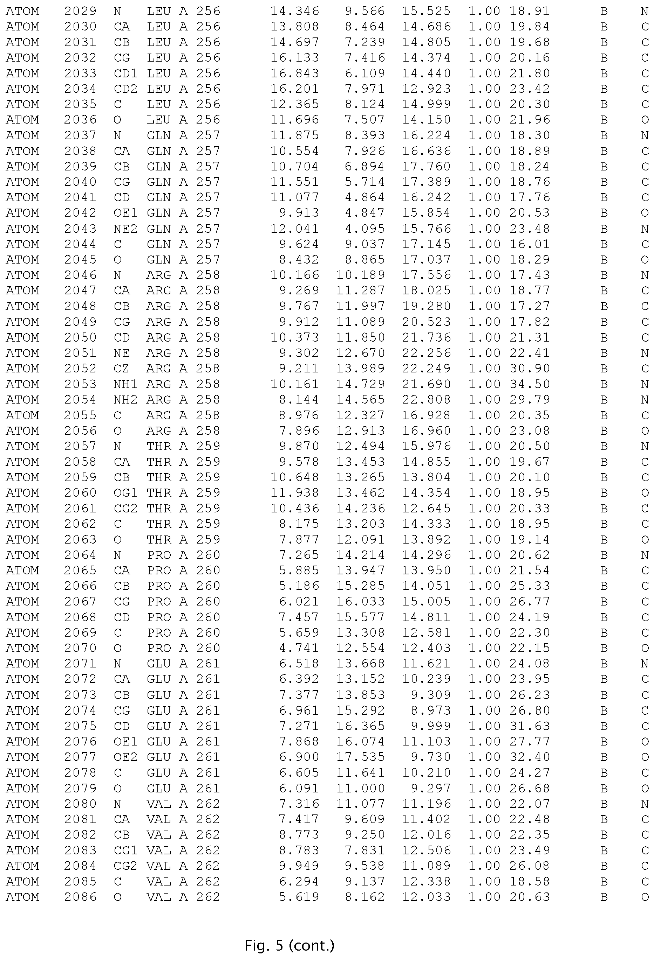

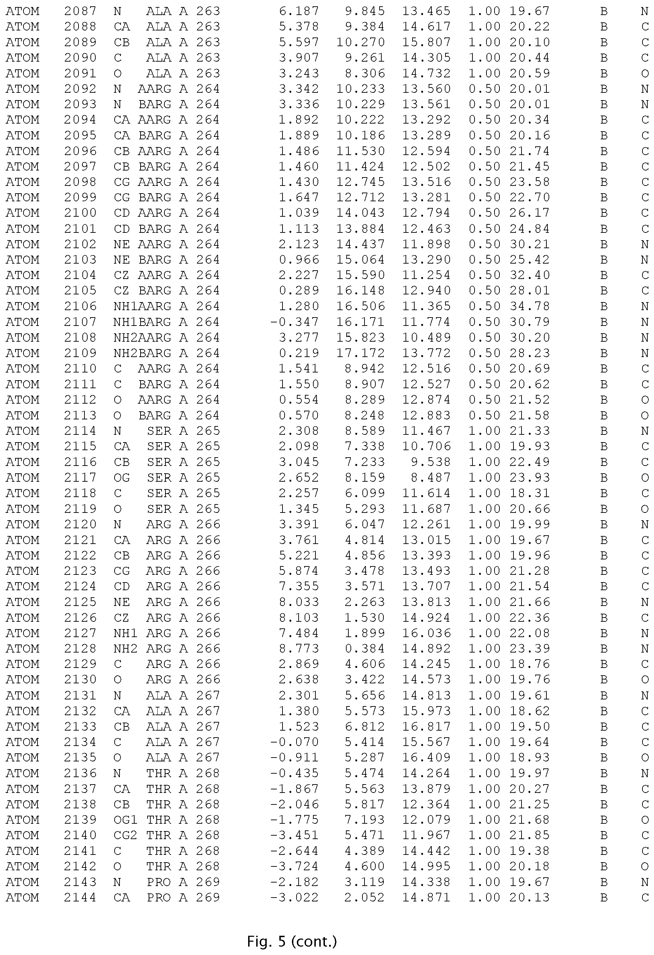

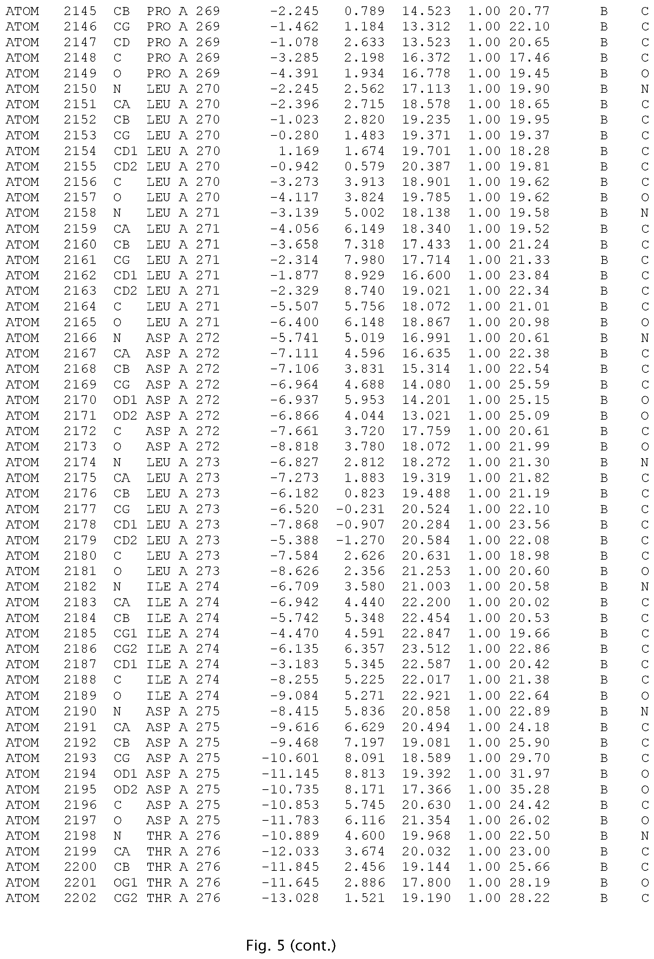

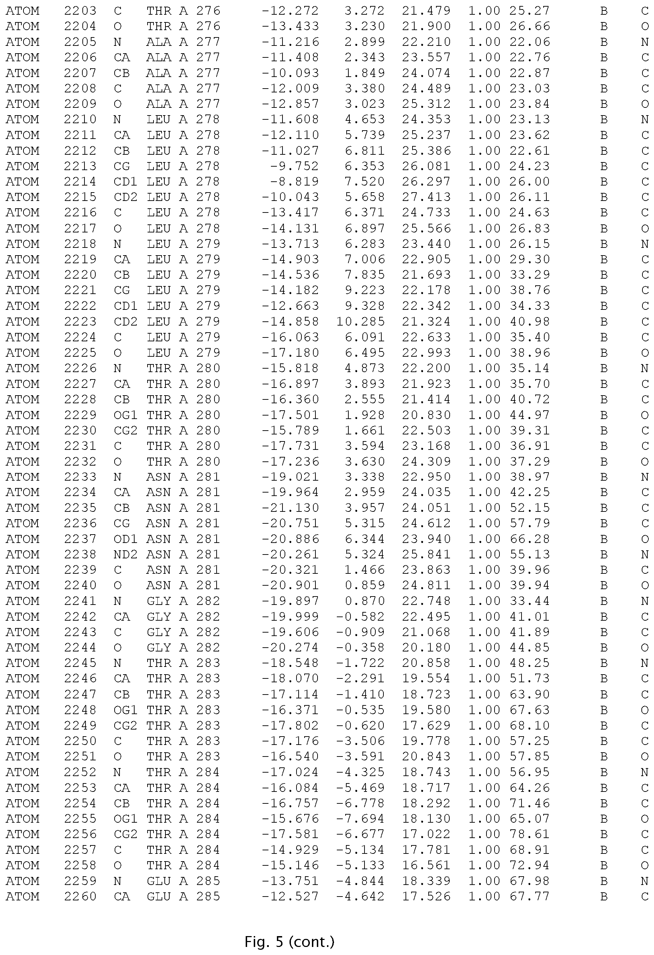

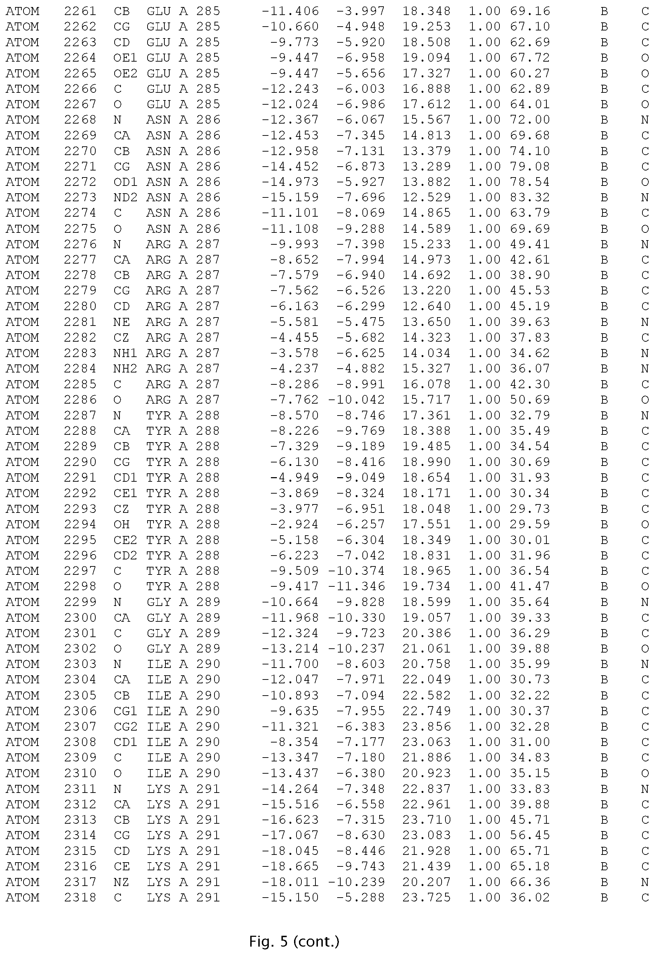

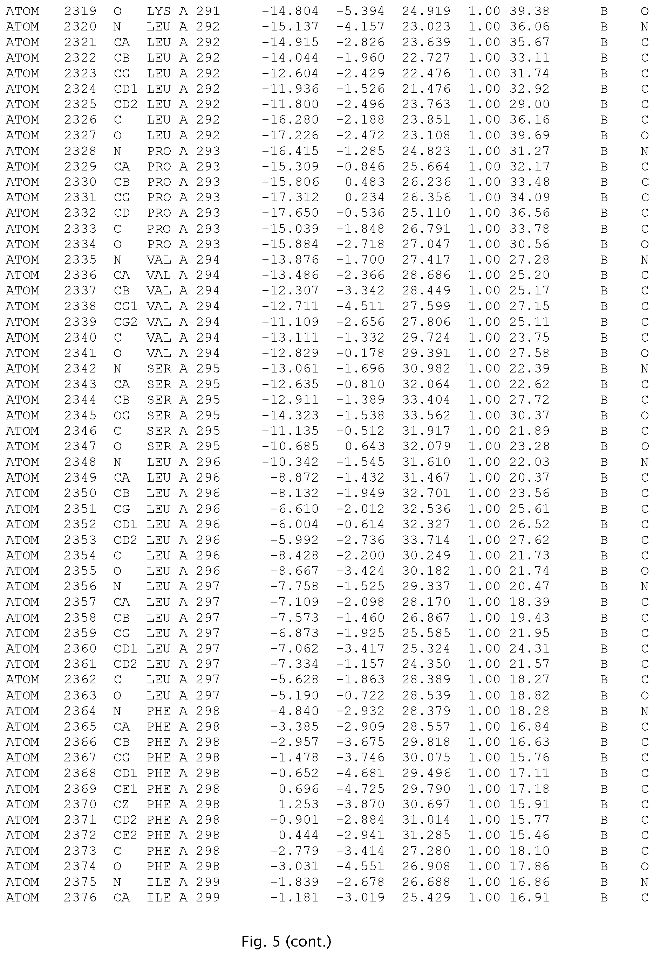

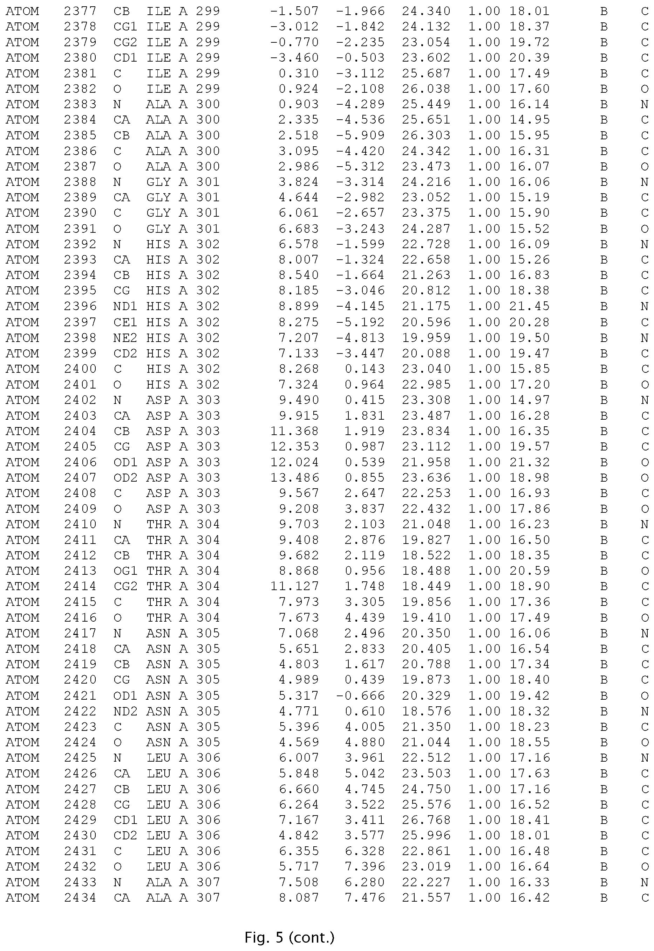

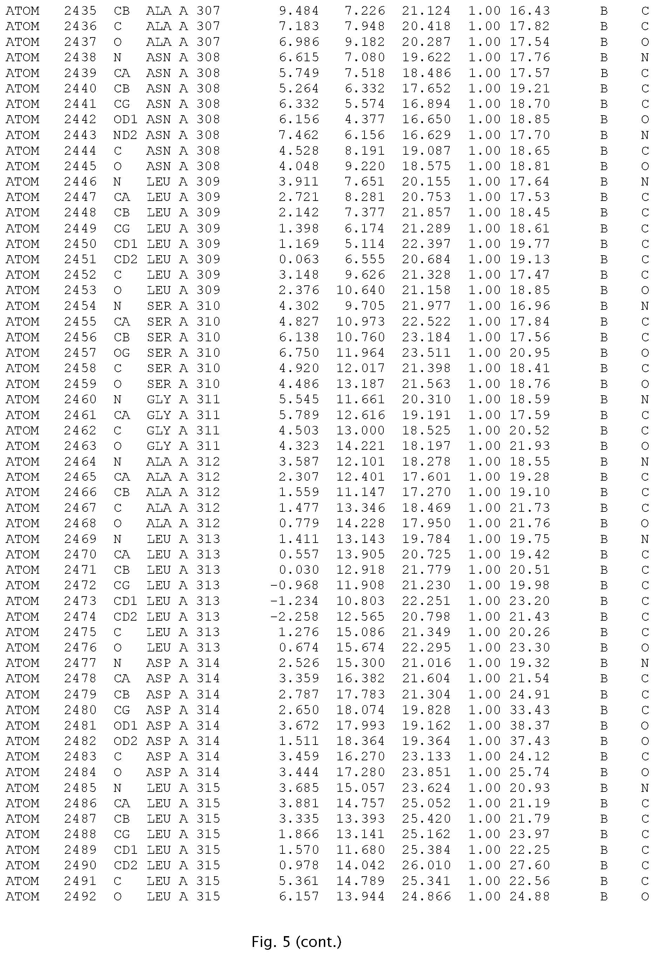

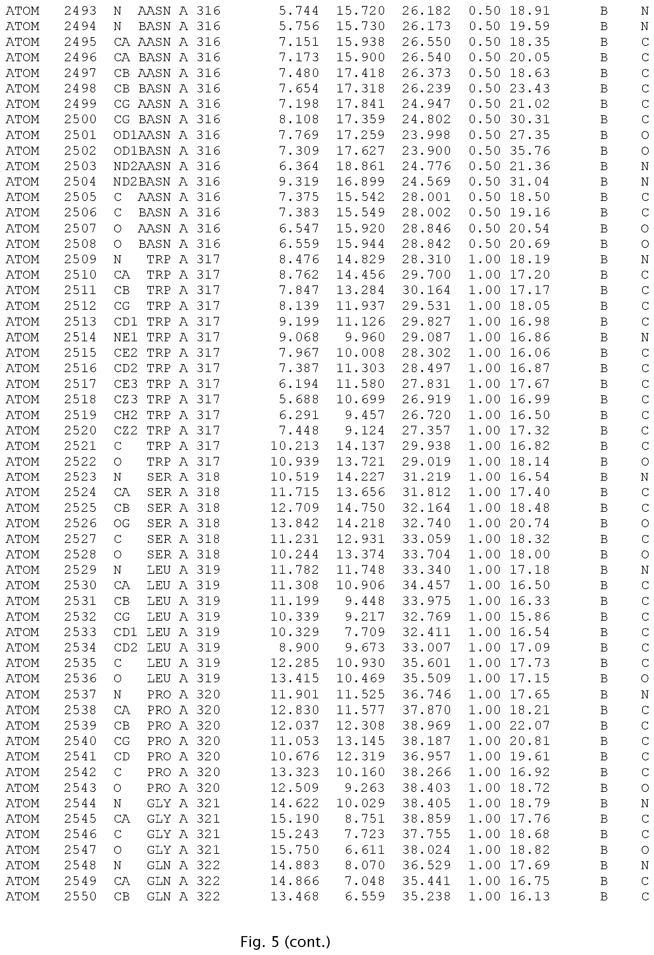

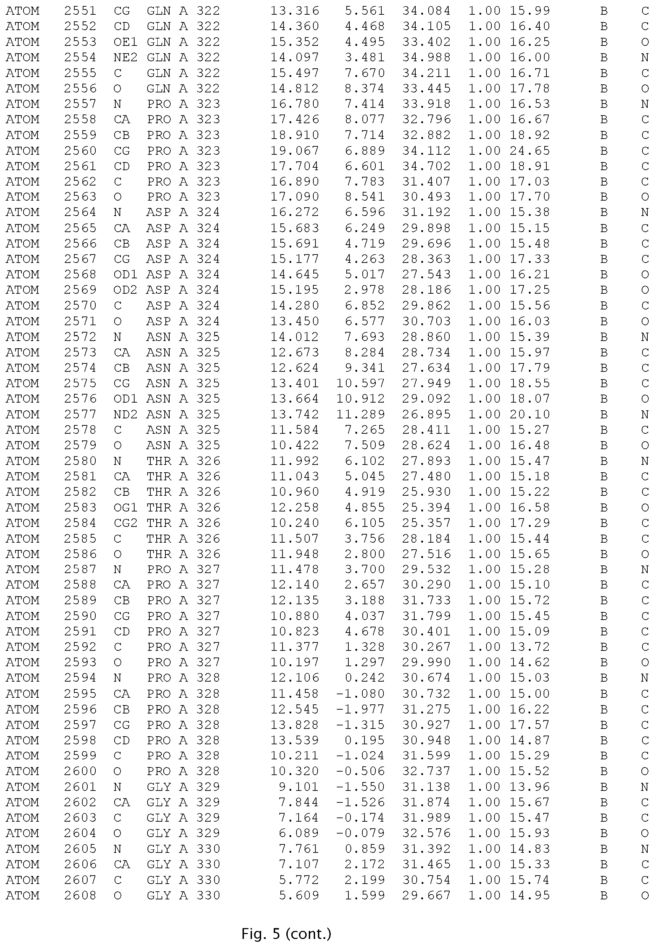

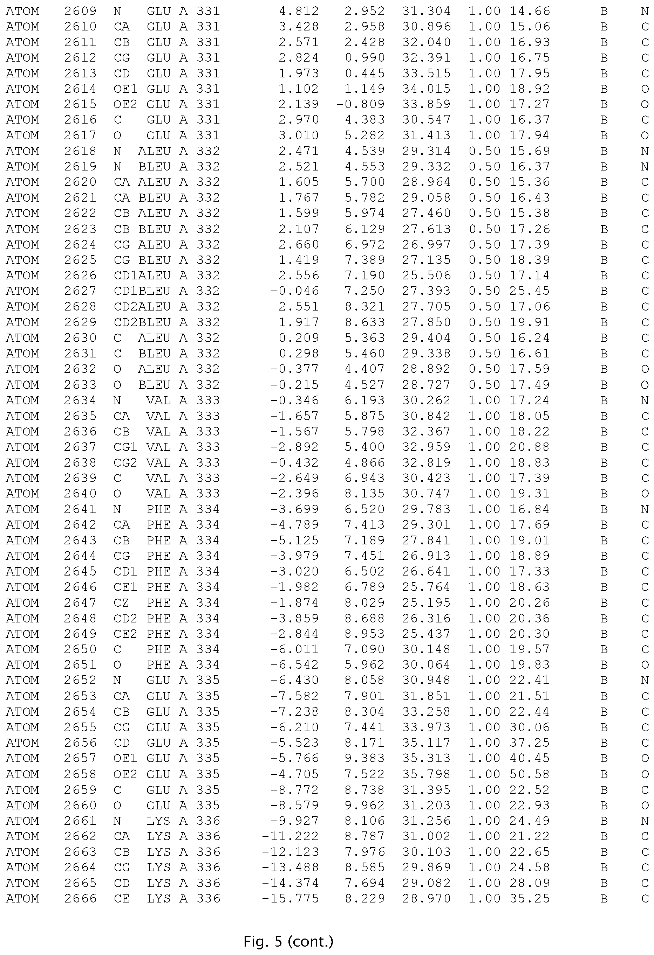

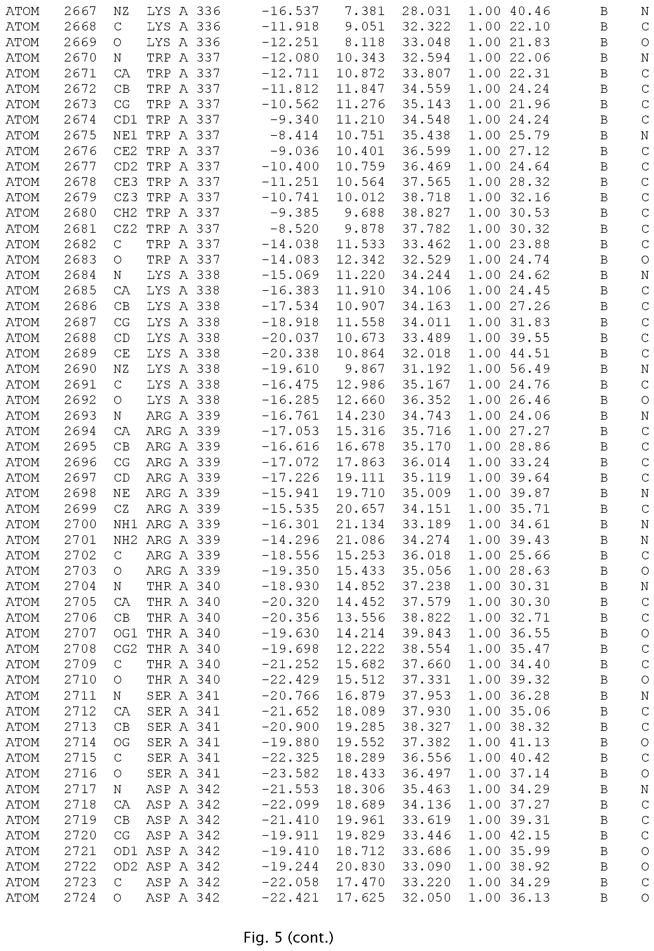

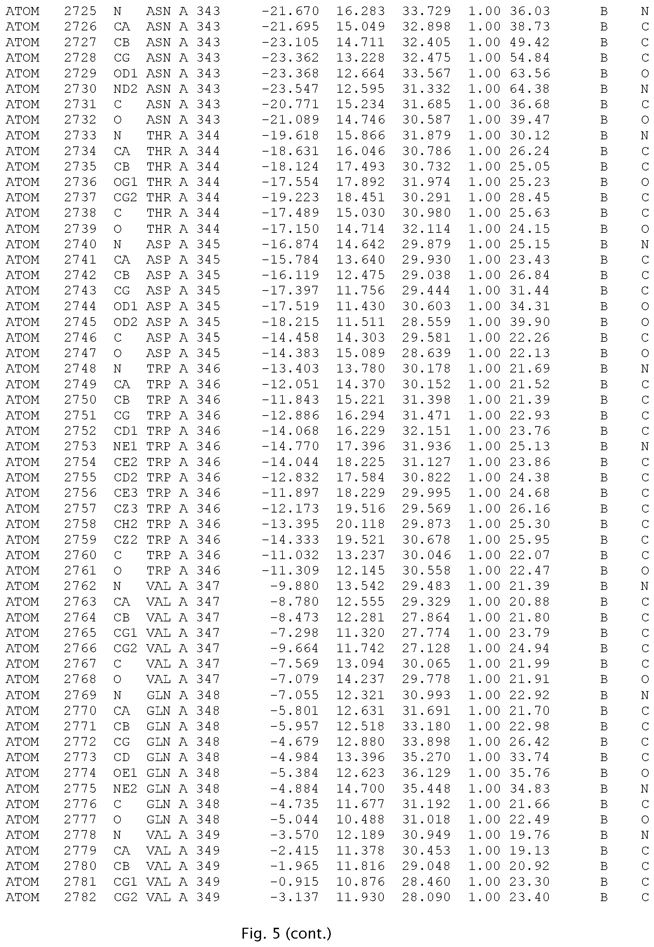

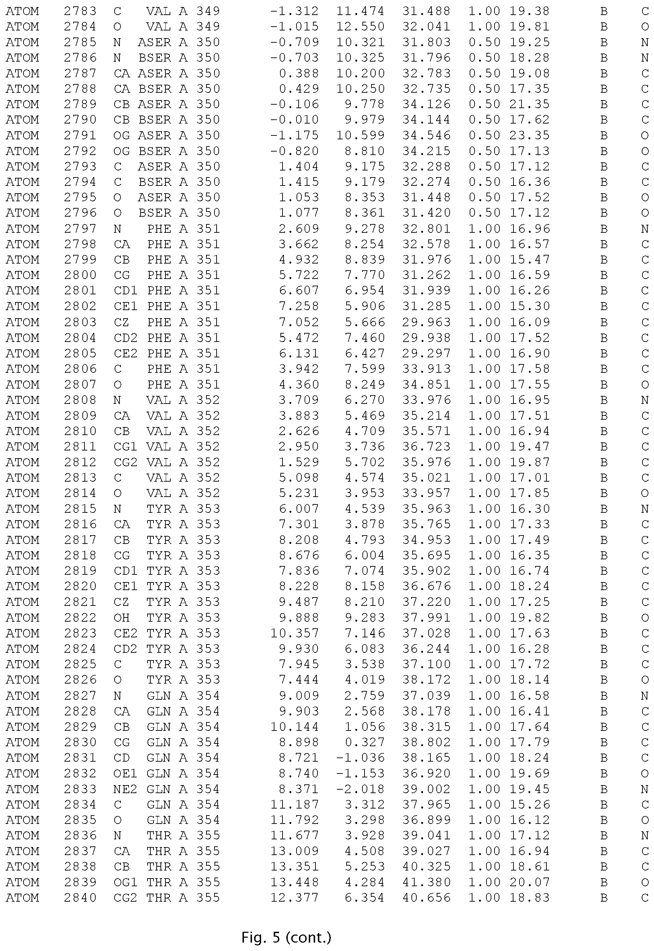

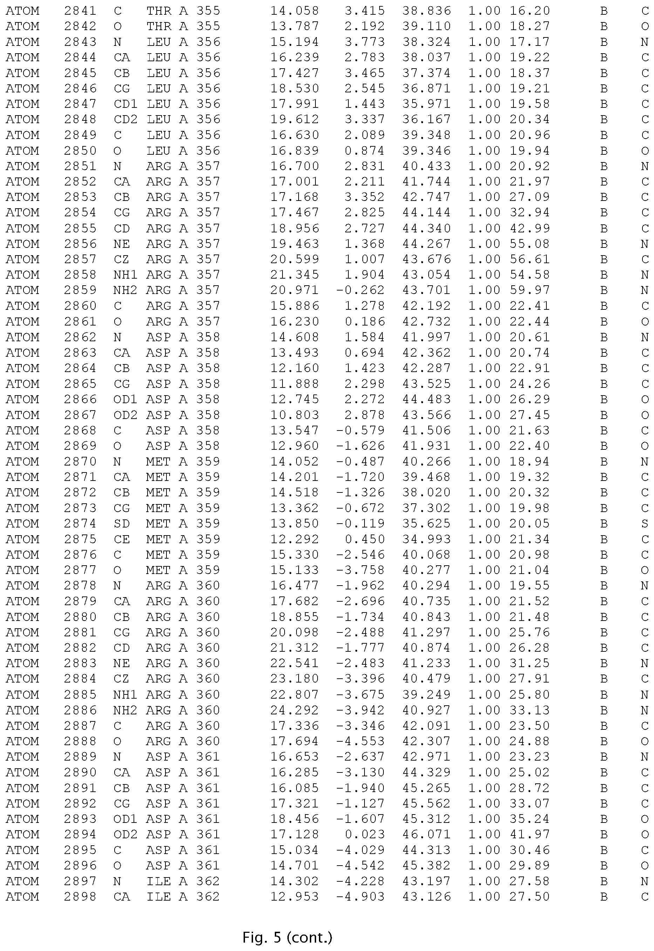

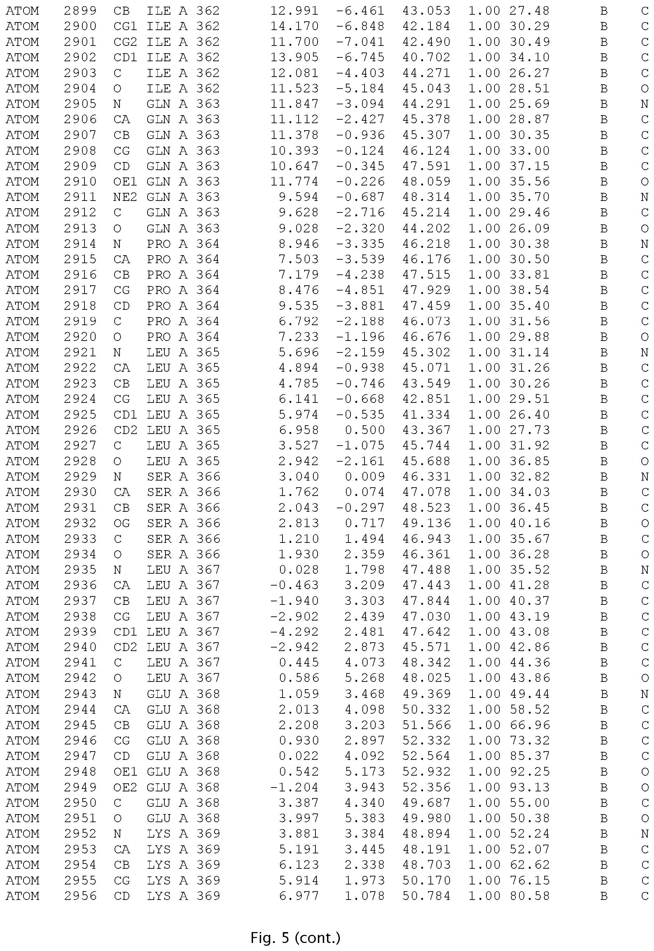

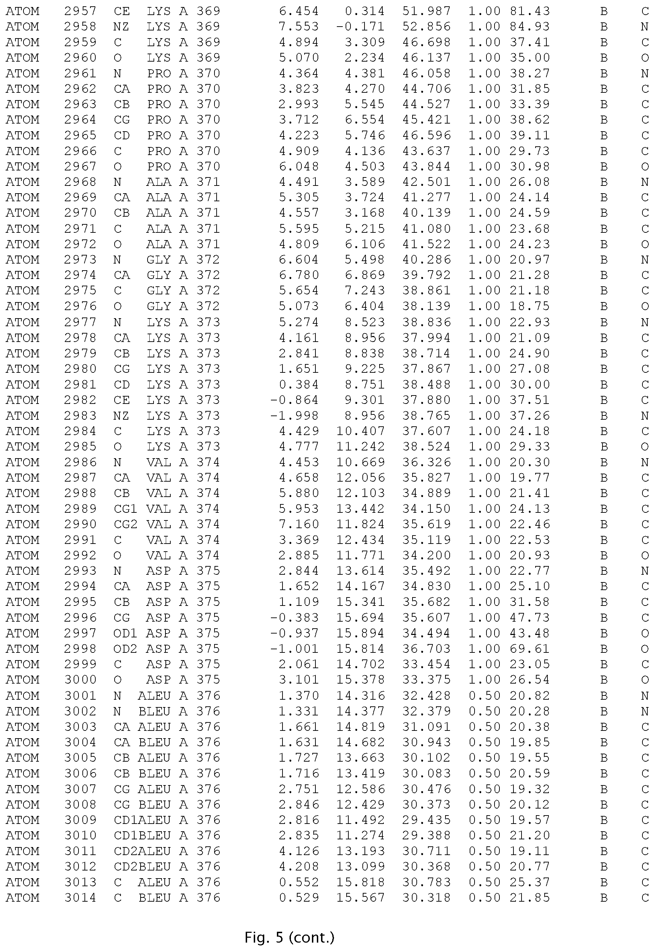

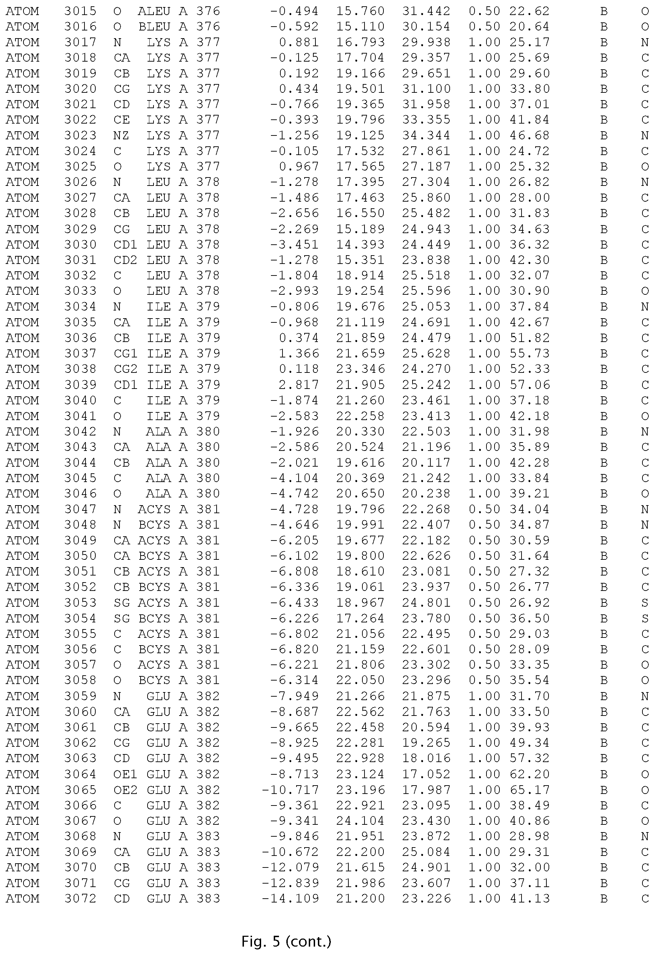

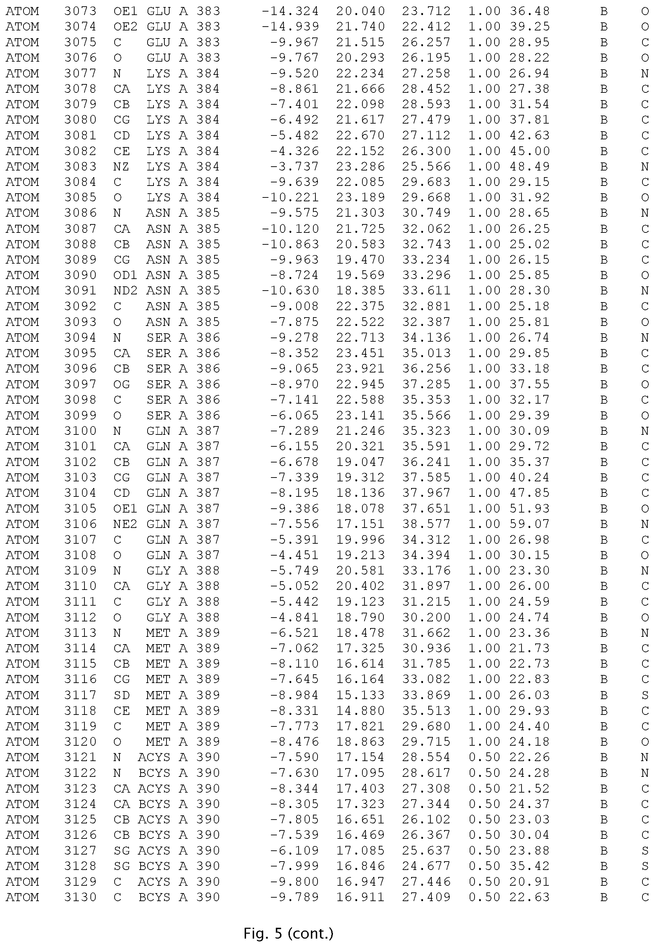

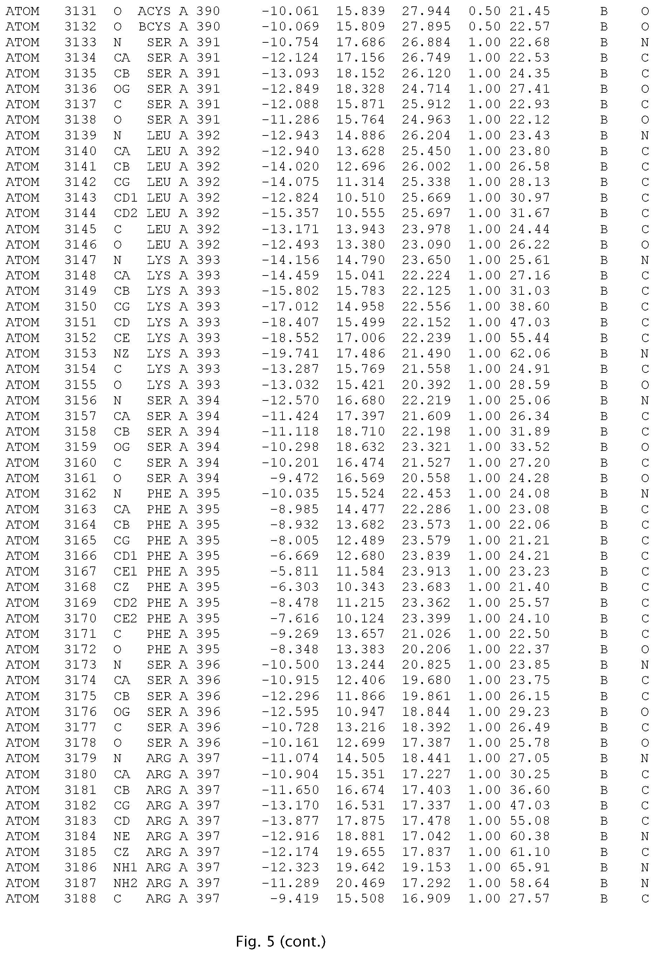

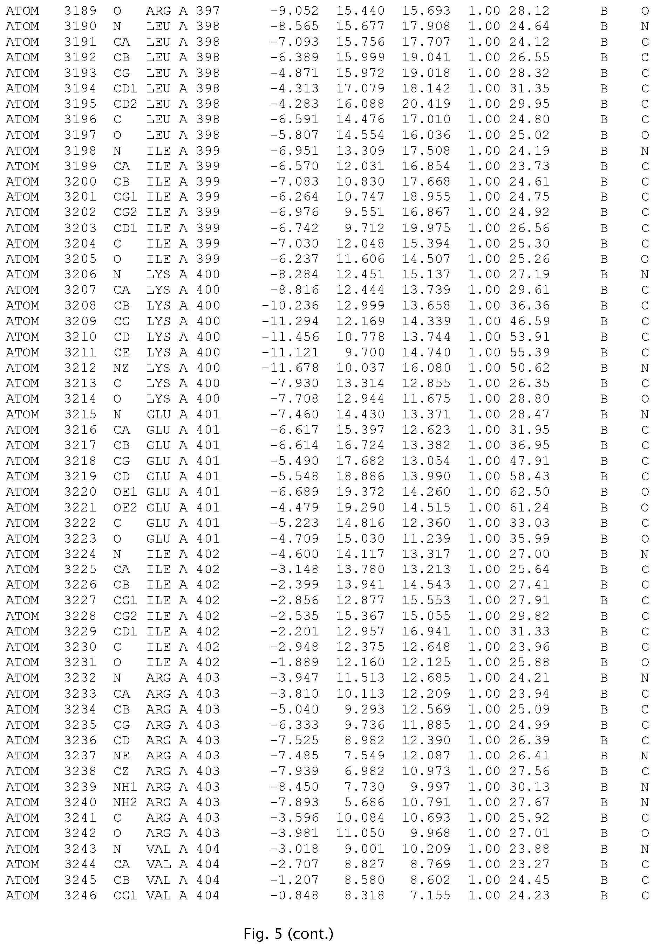

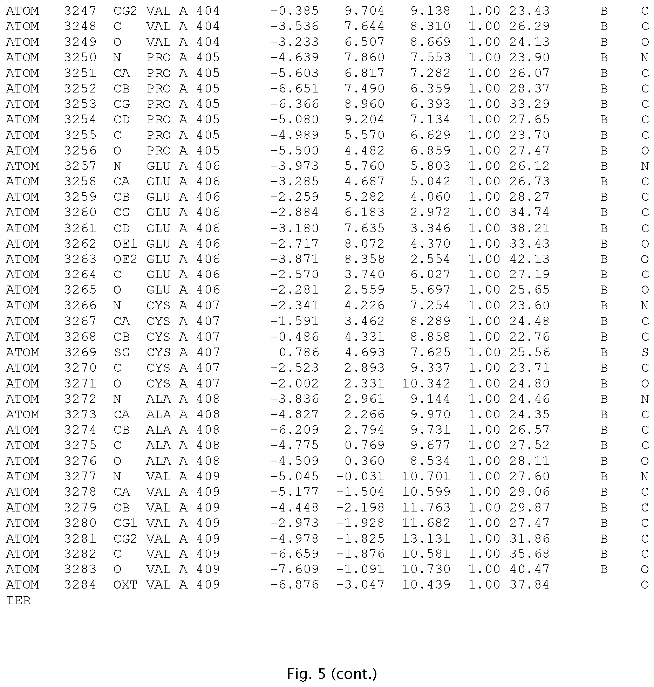

This application sets forth in Figure 5, the atomic coordinates of the three-dimensional structure of the phytase variant (var400).

### FIELD OF THE INVENTION

The present invention relates to phytase variants, polynucleotides encoding the variants, methods of producing the variants, and methods of using the variants.

### BACKGROUND OF THE INVENTION

Phytases are well-known enzymes, as are the advantages of adding them to foodstuffs for animals, including humans. Phytases have been isolated from various sources, including a number of fungal and bacterial strains.

It is an object of the present invention to provide alternative polypeptides having phytase activity (phytases) and polynucleotides encoding the polypeptides. The phytase variants of the invention exhibit modified or altered preferably improved properties as compared to the parent phytase. Non-limiting examples of such properties are: stability (such as acid-stability, heat-stability, steam stability, pelleting stability, and/or protease stability, in particular pepsin stability), temperature profile, pH profile, specific activity, substrate specificity, performance in animal feed (such as an improved release and/or degradation of phytate), susceptibility to glycation, and/or glycosylation pattern.

As described herein, mutagenesis of a parent polynucleotide encoding a phytase is employed to prepare variant (synthetic) DNAs encoding a phytase having improved properties relative to the phytase encoded by the parent polynucleotide.

### Citrobacter

The sequence of the phyA gene from a strain of *Citrobacter freundii* has been submitted by Zinin et al to the EMBL/GenBank/DDBJ databases with accession no. AY390262. The corresponding phytase amino acid sequence is found in the UniProt/TrEMBL databases with accession no. Q676V7. The expected mature part of Q676V7 is included in the present sequence listing as SEQ ID NO: 4.

WO-2004/085638 (Republic of National Fisheries Research and Development Institute of Korea) discloses, as SEQ ID NO: 7, the amino acid sequence of a phytase from *Citrobacter braakii* YH-15, deposited as KCCM 10427. The mature part of this amino acid sequence is included herein as SEQ ID NO: 3. This sequence is also found in the database Geneseqp with accession no. ADU50737.

WO 2006/037328 (Novozymes A/S) discloses the wildtype phytase of *Citrobacter braakii* ATCC 51113 (i.e., SEQ ID NO: 2 herein), as well as a variant thereof, which is also included in the present sequence listing, viz. as SEQ ID NO: 6.

WO 2006/038062 and WO 2006/038128 (Danisco A/S) both disclose the amino acid sequence of the phytase gene of *Citrobacter freundii* P3-42, deposited under accession number NCIMB 41247 and a number of variants thereof. This amino acid sequence is included herein as SEQ ID NO: 9. These applications disclose only one substitution in position 233 to a cysteine (S233C) according to the numbering used herein this would be S211C. The texts of WO 2006/038062 and WO 2006/038128 seem to be identical.

WO 2007/112739 (Novozymes A/S) discloses a large number of phytase variants with exemplification using *Citrobacter braakii* ATCC 51113 phytase as parent. WO 2007/112739 indicates inter alia the creation of disulfide bridges.

WO2011/117396 (Novozymes A/S) discloses additional phytase variants with exemplification using *Citrobacter braakii* ATCC 51113 phytase as variant by introducing two or more disulfide bridges in the molecule.

### SUMMARY OF THE INVENTION

The present application discloses phytase variants which has at least 70% identity to SEQ ID NO: 2 and which comprises the alterations N31C+G52C+A99C+K141C+T177C+V199C as compared to SEQ ID NO: 2, so as to form disulfide bridges between positions 52 and 99, 31 and 177, and 141 and 199 and further comprises a substitution in one or more position(s) selected from the following: 30, 36, 43, 46, 57, 60, 64, 73, 79, 119, 121, 123, 130, 134,138, 151, 155, 161, 162, 168, 176, 180, 184, 190, 207, 224, 230, 243, 273, 286, 336, 340, 358 and 375 using SEQ ID NO: 2 for numbering.

The present invention relates to phytase variants which has at least 70%, at least 75%, at least 80%, at least 85%, at least 90%, at least 95%, at least 98%, or at least 99% identity, but less than 100% identity, to SEQ ID NO: 2 and which comprises the alterations N31C+G52C+A99C+K141C+T177C+V199C+N203L as compared to SEQ ID NO: 2 and further comprises a substitution in one or more position(s) selected from the following: 30, 36, 43, 46, 57, 60, 64, 73, 79, 119, 121, 123, 130, 134,138, 151, 155, 161, 162, 168, 176, 180, 184, 190, 207, 224, 230, 243, 273, 286, 336, 340, 358 and 375 using SEQ ID NO: 2 for numbering, and which have phytase activity.

The present invention further relates to compositions, in particular animal feed compositions comprising the variants of the invention, and the use of such compositions for improving the nutritional value of an animal feed; reducing the phytate levels in animal manure; treating vegetable proteins; for liberating phosphorous from a phytate substrate; or for increasing weight gain, improving specific growth rate and/or improving Feed Conversion Ratio of an animal; or for improving nutrient retention, and/or nutrient digestibility in an animal.

### BRIEF DESCRIPTION OF THE SEQUENCE LISTING

In the Sequence listing the sequences apply as follows:
SEQ ID NO: 1 represents the polynucleotide sequence of the phytase from *Citrobacter braakii* ATCC 51113 (WO 2006/037328).
SEQ ID NO: 2 represents the polypeptide sequence of the phytase from *Citrobacter braakii* ATCC 51113 (WO 2006/037328).
SEQ ID NO: 3 represents the polypeptide sequence of the phytase from *Citrobacter braakii* YH-15 (WO-2004/085638).
SEQ ID NO: 4 represents the polypeptide sequence of the phytase from *Citrobacter freundii* (UniProt/TrEMBL accession no. Q676V7).
SEQ ID NO: 5 represents a variant of SEQ ID NO: 2 (18 is Xaa and 323 are Xaa).
SEQ ID NO: 6 represents a variant of SEQ ID NO: 2 (18 is Gly and 323 is Pro).
SEQ ID NO: 7 represents Citrobacter braakii ATCC 51113 signal peptide.
SEQ ID NO: 8 represents Citrobacter braakii ATCC 51113 pro-peptide.
SEQ ID NO: 9 represents the polypeptide sequence of the phytase from *Citrobacter freundii* NCIMB 41247 (WO 2006/038062 and WO 2006/038128).
SEQ ID NO: 10 represents the mature polypeptide sequence of a variant of SEQ ID NO: 2, entitled var300.
SEQ ID NO: 11 represents the polynucleotide sequence encoding for SEQ ID NO: 10 with coding sequence (CDS) from nucleotide 11 to nucleotide 1351.
SEQ ID NO: 12 represents the mature polypeptide sequence of a variant of SEQ ID NO: 2, entitled var400.
SEQ ID NO: 13 represents the polynucleotide sequence encoding for SEQ ID NO: 12 with CDS from nucleotide 11 to nucleotide 1351.
SEQ ID NO: 14 represents the mature polypeptide sequence of a variant of SEQ ID NO: 2, entitled var404.
SEQ ID NO: 15 represents the polynucleotide sequence encoding for SEQ ID NO: 14 with CDS from nucleotide 11 to nucleotide 1351.
SEQ ID NO: 16 represents the mature polypeptide sequence of a variant of SEQ ID NO: 2, entitled var405.
SEQ ID NO: 17 represents the polynucleotide sequence encoding for SEQ ID NO: 16 with CDS from nucleotide 11 to nucleotide 1351.
SEQ ID NO: 18 represents the mature polypeptide sequence of a variant of SEQ ID NO: 2, entitled var406.
SEQ ID NO: 19 represents the polynucleotide sequence encoding for SEQ ID NO: 18 with CDS from nucleotide 11 to nucleotide 1351.
SEQ ID NO: 20 represents the mature polypeptide sequence of a variant of SEQ ID NO: 2, entitled var411.
SEQ ID NO: 21 represents the polynucleotide sequence encoding for SEQ ID NO: 20 with CDS from nucleotide 1 to nucleotide 1341.
SEQ ID NO: 22 represents a polypeptide sequence of a protease.

### BRIEF DESCRIPTION OF THE DRAWINGS

Figure 1 shows an example of an alignment of the phytase of SEQ ID NO: 2 and the phytase of SEQ ID NO: 9.
Figure 2 shows feed consumption ratio (FR) and body weight (BW) evolution during the experimental trial.
Figure 3 shows schema for bone P retention sampling.

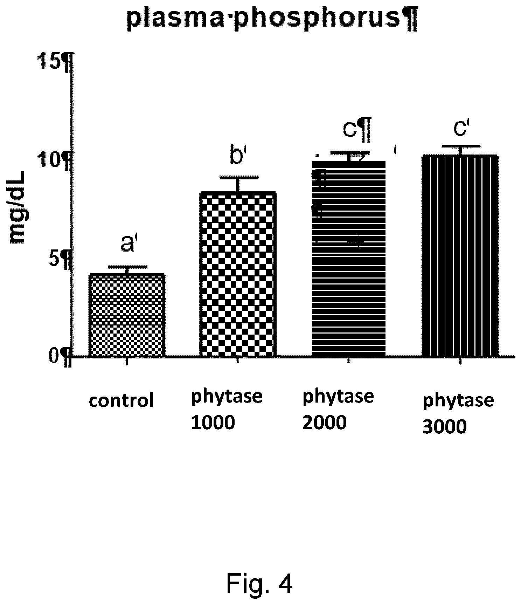

Figure 4 shows phosphorus in the plasma of fish from different experimental treatments after 91 days of feeding. Data are presented as mean ± SD (Standard Deviation). Significant treatments (p<0.05) are shown by different letters.
Figure 5 sets forth the atomic coordinates of the three-dimensional structure of the phytase variant (var400). These atomic coordinates can aid in generating a three-dimensional model depicting the structure of the phytase variant (var400) and a three-dimensional model of homologous structures, such as variants of the aforementioned phytase variant.

### DETAILED DESCRIPTION OF THE INVENTION

A phytase with improved properties, including improved instrinsic temperature and pH stability and improved *in vivo* efficacy per enzyme unit (FYT) is herein described. As known to the person skilled in the art, these types of improvements not only allow for flexibility in the formulation of the product and the consequent cost-savings with this flexibility, it provides for improved removal of phytate and anti-nutritional factors, improved release of phosphorous, calcium and myo-inositol and increased digestibility of phosphorous, improved muscle protein accretion by myo-inositol release and minimized P excretion for improved sustainability.

Disclosed herein but not claimed are phytase variants which has at least 70% identity to SEQ ID NO: 2 and which comprises the alterations N31C+G52C+A99C+K141C+T177C+V199C as compared to SEQ ID NO: 2, so as to form disulfide bridges between positions 52 and 99, 31 and 177, and 141 and 199.

Disclosed but not claimed are phytase variants which has at least 70%, at least 75%, at least 80%, at least 85%, at least 90% or at least 95% identity to SEQ ID NO: 2 and which comprises the alterations N31C/G52C/A99C/K141C/T177C/V199C as compared to SEQ ID NO: 2 and further comprises a substitution in one or more position(s) selected from the following: 30, 36, 43, 46, 57, 60, 64, 73, 79, 119, 121, 123, 130, 134,138, 151, 155, 161, 162, 168, 176, 180, 184, 190, 207, 224, 230, 243, 273, 286, 336, 340, 358 and 375 using SEQ ID NO: 2 for numbering, wherein the variants have phytase activity.

### Variants

The present invention provides phytase variants, comprising the substitutions N31C/G52C/A99C/K141C/T177C/V199C/N203L as compared to SEQ ID NO: 2 and further comprises an alteration in one or more position(s) selected from the following: 30, 36, 43, 46, 57, 60, 64, 73, 79, 119, 121, 123, 130, 134,138, 151, 155, 161, 162, 168, 176, 180, 184, 190, 207, 224, 230, 243, 273, 286, 336, 340, 358 and 375 using SEQ ID NO: 2 for numbering, wherein the variants have phytase activity.

In an embodiment, the alteration is a substitution.

In an embodiment, the variant has sequence identity of at least 70%, at least 75%, at least 80%, at least 85%, at least 90%, at least 91%, at least 92%, at least 93%, at least 94%, at least 95%, at least 96%, at least 97%, at least 98%, or at least 99%, but less than 100%, to the amino acid sequence of the parent phytase.

In another embodiment, the variant has at least 70%, at least 75%, at least 80%, at least 85%, at least 90%, at least 91%, at least 92%, at least 93%, at least 94%, at least 95%, such as at least 96%, at least 97%, at least 98%, or at least 99%, but less than 100%, sequence identity to the mature polypeptide of SEQ ID NO: 2.

In one aspect, the variants of the invention comprises the substitutions N31C/G52C/A99C/K141C/T177C/V199C/N203L as compared to SEQ ID NO: 2 and further alterations, where the number of further alterations in the variants of the present invention is 1-30, e.g., 1-20, 1-10 and 1-5, such as 1, 2, 3, 4, 5, 6, 7, 8, 9 or 10 alterations.

In another aspect, the variants of the invention comprises the substitutions N31C/G52C/A99C/K141C/T177C/N199C/N203L as compared to SEQ ID NO: 2 and further one or more substitution in positions corresponding to 57, 73, 121, 134, 155, 207 and 273 using SEQ ID NO: 2 for numbering. In a preferred embodiment, the variants comprises the substitutions N31C/G52C/A99C/K141C/T177C/N199C/N203L as compared to SEQ ID NO: 2 and further two or more substitution, e.g. 2, 3, 4, 5, 6 or 7 substitutions; in positions corresponding to 57, 73, 121, 134, 155, 207 and 273 using SEQ ID NO: 2 for numbering.

In another aspect, the variant comprises or consists of a substitution at a position corresponding to position 57. In another aspect, the amino acid at a position corresponding to position 57 is substituted with Ala, Arg, Asn, Asp, Cys, Gln, Gly, His, Ile, Leu, Lys, Met, Phe, Pro, Ser, Thr, Trp, Tyr, or Val, preferably with Tyr. In another aspect, the variant comprises or consists of the substitution E57Y of the mature polypeptide of SEQ ID NO: 2.

In another aspect, the variant comprises or consists of a substitution at a position corresponding to position 73. In another aspect, the amino acid at a position corresponding to position 73 is substituted with Ala, Arg, Asp, Cys, Gln, Glu, Gly, His, Ile, Leu, Lys, Met, Phe, Pro, Ser, Thr, Trp, Tyr, or Val, preferably with Pro. In another aspect, the variant comprises or consists of the substitution N73P of the mature polypeptide of SEQ ID NO: 2.

In another aspect, the variant comprises or consists of a substitution at a position corresponding to position 121. In another aspect, the amino acid at a position corresponding to position 121 is substituted with Ala, Arg, Asp, Cys, Gln, Glu, Gly, His, Ile, Leu, Lys, Met, Phe, Pro, Ser, Thr, Trp, Tyr, or Val, preferably with Pro. In another aspect, the variant comprises or consists of the substitution N121P of the mature polypeptide of SEQ ID NO: 2.

In another aspect, the variant comprises or consists of a substitution at a position corresponding to position 134. In another aspect, the amino acid at a position corresponding to position 134 is substituted with Ala, Arg, Asp, Asn, Cys, Gln, Glu, Gly, His, Ile, Leu, Lys, Met, Phe, Pro, Thr, Trp, Tyr, or Val, preferably with Gln. In another aspect, the variant comprises or consists of the substitution S134Q of the mature polypeptide of SEQ ID NO: 2.

In another aspect, the variant comprises or consists of a substitution at a position corresponding to position 155. In another aspect, the amino acid at a position corresponding to position 155 is substituted with Ala, Arg, Asp, Asn, Cys, Gln, Glu, Gly, His, Ile, Leu, Lys, Met, Phe, Pro, Ser, Thr, Trp, or Val, preferably with Phe. In another aspect, the variant comprises or consists of the substitution Y155F of the mature polypeptide of SEQ ID NO: 2.

In another aspect, the variant comprises or consists of a substitution at a position corresponding to position 207. In another aspect, the amino acid at a position corresponding to position 207 is substituted with Ala, Arg, Asp, Asn, Cys, Gln, Glu, Gly, His, Ile, Leu, Lys, Met, Phe, Ser, Thr, Trp, Tyr, or Val, preferably with Thr. In another aspect, the variant comprises or consists of the substitution P207T of the mature polypeptide of SEQ ID NO: 2.

In another aspect, the variant comprises or consists of a substitution at a position corresponding to position 273. In another aspect, the amino acid at a position corresponding to position 273 is substituted with Ala, Arg, Asp, Asn, Cys, Gln, Glu, Gly, His, Ile, Leu, Lys, Phe, Pro, Ser, Thr, Trp, Tyr, or Val, preferably with Leu. In another aspect, the variant comprises or consists of the substitution M273L of the mature polypeptide of SEQ ID NO: 2.

In another aspect, the variant comprises or consists of an alteration at positions corresponding to positions 57 and 73, such as those described above.

In another aspect, the variant comprises or consists of alterations at positions corresponding to positions 57 and 121, such as those described above.

In another aspect, the variant comprises or consists of alterations at positions corresponding to positions 57 and 134, such as those described above.

In another aspect, the variant comprises or consists of an alteration at positions corresponding to positions 57 and 155, such as those described above.

In another aspect, the variant comprises or consists of alterations at positions corresponding to positions 57 and 207, such as those described above.

In another aspect, the variant comprises or consists of alterations at positions corresponding to positions 57 and 273, such as those described above.

In another aspect, the variant comprises or consists of alterations at positions corresponding to positions 73, and 121, such as those described above.

In another aspect, the variant comprises or consists of alterations at positions corresponding to positions 73 and 134, such as those described above.

In another aspect, the variant comprises or consists of alterations at positions corresponding to positions 73, and 155, such as those described above.

In another aspect, the variant comprises or consists of alterations at positions corresponding to positions 73 and 207, such as those described above.

In another aspect, the variant comprises or consists of alterations at positions corresponding to positions 73 and 273, such as those described above.

In another aspect, the variant comprises or consists of alterations at positions corresponding to positions 121 and 134, such as those described above.

In another aspect, the variant comprises or consists of alterations at positions corresponding to positions 121 and 155, such as those described above.

In another aspect, the variant comprises or consists of alterations at positions corresponding to positions 121 and 207, such as those described above.

In another aspect, the variant comprises or consists of alterations at positions corresponding to positions 121 and 273, such as those described above.

In another aspect, the variant comprises or consists of alterations at positions corresponding to positions 134 and 155, such as those described above.

In another aspect, the variant comprises or consists of alterations at positions corresponding to positions 134 and 207, such as those described above.

In another aspect, the variant comprises or consists of alterations at positions corresponding to positions 134 and 273, such as those described above.

In another aspect, the variant comprises or consists of alterations at positions corresponding to positions 155 and 207, such as those described above.

In another aspect, the variant comprises or consists of alterations at positions corresponding to positions 155 and 273, such as those described above.

In another aspect, the variant comprises or consists of alterations at positions corresponding to positions 207 and 273, such as those described above.

In another aspect, the variant comprises or consists of alterations at positions corresponding to positions 57, 73, and 121, such as those described above.

In another aspect, the variant comprises or consists of alterations at positions corresponding to positions 57, 73, and 134, such as those described above.

In another aspect, the variant comprises or consists of alterations at positions corresponding to positions 57, 73, and 155, such as those described above.

In another aspect, the variant comprises or consists of alterations at positions corresponding to positions 57, 73, and 207, such as those described above.

In another aspect, the variant comprises or consists of alterations at positions corresponding to positions 57, 73, and 273, such as those described above.

In another aspect, the variant comprises or consists of alterations at positions corresponding to positions 57, 121, and 134, such as those described above.

In another aspect, the variant comprises or consists of alterations at positions corresponding to positions 57, 121, and 155, such as those described above.

In another aspect, the variant comprises or consists of alterations at positions corresponding to positions 57, 121, and 207, such as those described above.

In another aspect, the variant comprises or consists of alterations at positions corresponding to positions 57, 121, and 273, such as those described above.

In another aspect, the variant comprises or consists of alterations at positions corresponding to positions 57, 155, and 207, such as those described above.

In another aspect, the variant comprises or consists of alterations at positions corresponding to positions 57, 155, and 273, such as those described above.

In another aspect, the variant comprises or consists of alterations at positions corresponding to positions 57, 207, and 273, such as those described above.

In another aspect, the variant comprises or consists of alterations at positions corresponding to positions 73, 121, and 134, such as those described above.

In another aspect, the variant comprises or consists of alterations at positions corresponding to positions 73, 121, and 155, such as those described above.

In another aspect, the variant comprises or consists of alterations at positions corresponding to positions 73, 121, and 207, such as those described above.

In another aspect, the variant comprises or consists of alterations at positions corresponding to positions 73, 121, and 273, such as those described above.

In another aspect, the variant comprises or consists of alterations at positions corresponding to positions 73, 134, and 155, such as those described above.

In another aspect, the variant comprises or consists of alterations at positions corresponding to positions 73, 134, and 207, such as those described above.

In another aspect, the variant comprises or consists of alterations at positions corresponding to positions 73, 134, and 273, such as those described above.

In another aspect, the variant comprises or consists of alterations at positions corresponding to positions 73, 155, and 207, such as those described above.

In another aspect, the variant comprises or consists of alterations at positions corresponding to positions 73, 134, and 207, such as those described above.

In another aspect, the variant comprises or consists of alterations at positions corresponding to positions 73, 134, and 273, such as those described above.

In another aspect, the variant comprises or consists of alterations at positions corresponding to positions 121, 134, and 155, such as those described above.

In another aspect, the variant comprises or consists of alterations at positions corresponding to positions 121, 134, and 207, such as those described above.

In another aspect, the variant comprises or consists of alterations at positions corresponding to positions 121, 134, and 273, such as those described above.

In another aspect, the variant comprises or consists of alterations at positions corresponding to positions 134, 155, and 207, such as those described above.

In another aspect, the variant comprises or consists of alterations at positions corresponding to positions 134, 155, and 273, such as those described above.

In another aspect, the variant comprises or consists of alterations at positions corresponding to positions 155, 207, and 273, such as those described above.

In another aspect, the variant comprises or consists of alterations at positions corresponding to positions 57, 73, 121, and 134, such as those described above.

In another aspect, the variant comprises or consists of alterations at positions corresponding to positions 57, 73, 121, and 155, such as those described above.

In another aspect, the variant comprises or consists of alterations at positions corresponding to positions 57, 73, 121, and 207, such as those described above.

In another aspect, the variant comprises or consists of alterations at positions corresponding to positions 57, 73, 121, and 273, such as those described above.

In another aspect, the variant comprises or consists of alterations at positions corresponding to positions 57, 73, 134, and 155, such as those described above.

In another aspect, the variant comprises or consists of alterations at positions corresponding to positions 57, 73, 134, and 207, such as those described above.

In another aspect, the variant comprises or consists of alterations at positions corresponding to positions 57, 73, 134, and 273, such as those described above.

In another aspect, the variant comprises or consists of alterations at positions corresponding to positions 57, 73, 155, and 207, such as those described above.

In another aspect, the variant comprises or consists of alterations at positions corresponding to positions 57, 73, 155, and 273, such as those described above.

In another aspect, the variant comprises or consists of alterations at positions corresponding to positions 57, 73, 207, and 273, such as those described above.

In another aspect, the variant comprises or consists of alterations at positions corresponding to positions 57, 121, 134, and 155, such as those described above.

In another aspect, the variant comprises or consists of alterations at positions corresponding to positions 57, 121, 134, and 207, such as those described above.

In another aspect, the variant comprises or consists of alterations at positions corresponding to positions 57, 121, 134, and 273, such as those described above.

In another aspect, the variant comprises or consists of alterations at positions corresponding to positions 57, 121, 155, and 207, such as those described above.

In another aspect, the variant comprises or consists of alterations at positions corresponding to positions 57, 121, 207, and 273, such as those described above.

In another aspect, the variant comprises or consists of alterations at positions corresponding to positions 57, 134, 155, and 207, such as those described above.

In another aspect, the variant comprises or consists of alterations at positions corresponding to positions 57, 134, 155, and 273, such as those described above.

In another aspect, the variant comprises or consists of alterations at positions corresponding to positions 57, 155, 207, and 273, such as those described above.

In another aspect, the variant comprises or consists of alterations at positions corresponding to positions 73, 121, 134, and 155, such as those described above.

In another aspect, the variant comprises or consists of alterations at positions corresponding to positions 73, 121, 134, and 207, such as those described above.

In another aspect, the variant comprises or consists of alterations at positions corresponding to positions 73, 121, 134, and 273, such as those described above.

In another aspect, the variant comprises or consists of alterations at positions corresponding to positions 73, 134, 155, and 207, such as those described above.

In another aspect, the variant comprises or consists of alterations at positions corresponding to positions 73, 134, 155, and 273, such as those described above.

In another aspect, the variant comprises or consists of alterations at positions corresponding to positions 73, 134, 207, and 273, such as those described above.

In another aspect, the variant comprises or consists of alterations at positions corresponding to positions 73, 155, 207, and 273, such as those described above.

In another aspect, the variant comprises or consists of alterations at positions corresponding to positions 121, 134, 155, and 207, such as those described above.

In another aspect, the variant comprises or consists of alterations at positions corresponding to positions 121, 134, 155, and 273, such as those described above.

In another aspect, the variant comprises or consists of alterations at positions corresponding to positions 121, 134, 207, and 273, such as those described above.

In another aspect, the variant comprises or consists of alterations at positions corresponding to positions 134, 155, 207, and 273, such as those described above.

In another aspect, the variant comprises or consists of alterations at positions corresponding to positions 57, 73, 121, 134, and 155, such as those described above.

In another aspect, the variant comprises or consists of alterations at positions corresponding to positions 57, 73, 121, 134, and 207, such as those described above.

In another aspect, the variant comprises or consists of alterations at positions corresponding to positions 57, 73, 121, 134, and 273, such as those described above.

In another aspect, the variant comprises or consists of alterations at positions corresponding to positions 57, 73, 121, 155, and 207, such as those described above.

In another aspect, the variant comprises or consists of alterations at positions corresponding to positions 57, 73, 121, 155, and 273, such as those described above.

In another aspect, the variant comprises or consists of alterations at positions corresponding to positions 57, 73, 121, 207, and 273, such as those described above.

In another aspect, the variant comprises or consists of alterations at positions corresponding to positions 57, 73, 134, 155, and 207, such as those described above.

In another aspect, the variant comprises or consists of alterations at positions corresponding to positions 57, 73, 134, 155, and 273, such as those described above.

In another aspect, the variant comprises or consists of alterations at positions corresponding to positions 57, 73, 134, 207, and 273, such as those described above.

In another aspect, the variant comprises or consists of alterations at positions corresponding to positions 57, 73, 155, 207, and 273, such as those described above.

In another aspect, the variant comprises or consists of alterations at positions corresponding to positions 57, 121, 134, 155, and 207, such as those described above.

In another aspect, the variant comprises or consists of alterations at positions corresponding to positions 57, 121, 134, 155, and 273, such as those described above.

In another aspect, the variant comprises or consists of alterations at positions corresponding to positions 57, 121, 134, 207, and 273, such as those described above.

In another aspect, the variant comprises or consists of alterations at positions corresponding to positions 57, 121, 155, 207, and 273, such as those described above.

In another aspect, the variant comprises or consists of alterations at positions corresponding to positions 57, 134, 155, 207, and 273, such as those described above.

In another aspect, the variant comprises or consists of alterations at positions corresponding to positions 73, 121, 134, 155, and 207, such as those described above.

In another aspect, the variant comprises or consists of alterations at positions corresponding to positions 73, 121, 134, 155, and 273, such as those described above.

In another aspect, the variant comprises or consists of alterations at positions corresponding to positions 73, 121, 134, 207, and 273, such as those described above.

In another aspect, the variant comprises or consists of alterations at positions corresponding to positions 73, 121, 155, 207, and 273, such as those described above.

In another aspect, the variant comprises or consists of alterations at positions corresponding to positions 73, 134, 155, 207, and 273, such as those described above.

In another aspect, the variant comprises or consists of alterations at positions corresponding to positions 121, 134, 155, 207, and 273, such as those described above.

In another aspect, the variant comprises or consists of alterations at positions corresponding to positions 57, 73, 121, 134, 155 and 207, such as those described above.

In another aspect, the variant comprises or consists of alterations at positions corresponding to positions 57, 73, 121, 134, 155, and 273, such as those described above.

In another aspect, the variant comprises or consists of alterations at positions corresponding to positions 57, 73, 121, 134, 207, and 273, such as those described above.

In another aspect, the variant comprises or consists of alterations at positions corresponding to positions 57, 73, 121, 155, 207 and 273, such as those described above.

In another aspect, the variant comprises or consists of alterations at positions corresponding to positions 57, 73, 134, 155, 207, and 273, such as those described above.

In another aspect, the variant comprises or consists of alterations at positions corresponding to positions 57, 121, 134, 155, 207, and 273, such as those described above.

In another aspect, the variant comprises or consists of alterations at positions corresponding to positions 73, 121, 134, 155, 207, and 273, such as those described above.

In another aspect, the variant comprises or consists of alterations at positions corresponding to positions 57, 73, 121, 134, 155, 207, and 273, such as those described above.

In another aspect, the variant comprises or consists of one or more (e.g., several) substitutions selected from the group consisting of 30Q, 36A, 43C, 46C, 57Y, 60H, 64Q, 73P, 79Q, 119P, 121P, 123C, 130T,C, 134Q, 138A, 151S, 155F, 161T, 162A, 176P, 180N, 184Q, 190T, 207T, 224Q, 230E, 243N, 273L, 286S, 336R, 340L,P, 358Q and 375K.

In another aspect, the variant comprises or consists of one or more (e.g., several) substitutions selected from the group consisting of K30Q, Q36A, P43C, W46C, E57Y, Q60H, L64Q, N73P, S79Q, E119P, N121P, P123C, M130T,C, S134Q, L138A, N151S, Y155F, S161T, S162A, N168R, E176P, T180N, S184Q, P190T, P207T, E224Q, Q230E, R243N, M273L, N286S, K336R, T340L,P, D358Q and D375K.

In another aspect, the variant comprises or consists of the substitutions.

In a preferred embodiment, the variants comprises the substitutions N31C/G52C/A99C/K141C/T177C/V199C/N203L as compared to SEQ ID NO: 2, or of a polypeptide having at least 65%, at least 70%, at least 75%, at least 80%, at least 85%, at least 90%, at least 91%, at least 92%, at least 93%, at least 94%, at least 95%, at least 96%, at least 97%, at least 98%, at least 99% identity to the mature polypeptide of SEQ ID NO: 2 which has phytase activity, and further the variant comprises substitutions in the positions: 57, 73, 121, 134, 155, 207 and 273, preferably 57Y, 73P, 121P, 134Q, 155Y, 207T and 273L; and further comprises one or more substitution in one or more of the positions: 30, 36, 43, 46, 60, 64, 79, 119, 123, 130, 138, 151, 161, 162, 168, 176, 180, 184, 190, 224, 230, 243, 286, 336, 340, 358 and 375.

If a variant comprises the substitution P43C as compared to SEQ ID NO: 2 it is preferred that it also comprises the substitution W46C. If a variant comprises the substitution W46C as compared to SEQ ID NO: 2 it is preferred that it also comprises the substitution P43C. If a variant comprises the substitution P123C as compared to SEQ ID NO: 2 it is preferred that it also comprises the substitution M130C. If a variant comprises the substitution M130C as compared to SEQ ID NO: 2 it is preferred that it also comprises the substitution P123C.

The amino acid changes may be of a minor nature, that is conservative amino acid substitutions or insertions that do not significantly affect the folding and/or activity of the protein; small deletions, typically of 1-30 amino acids; small amino- or carboxyl-terminal extensions, such as an amino-terminal methionine residue; a small linker peptide of up to 20-25 residues; or a small extension that facilitates purification by changing net charge or another function, such as a polyhistidine tract, an antigenic epitope or a binding domain.

Examples of conservative substitutions are within the groups of basic amino acids (arginine, lysine and histidine), acidic amino acids (glutamic acid and aspartic acid), polar amino acids (glutamine and asparagine), hydrophobic amino acids (leucine, isoleucine and valine), aromatic amino acids (phenylalanine, tryptophan and tyrosine), and small amino acids (glycine, alanine, serine, threonine and methionine). Amino acid substitutions that do not generally alter specific activity are known in the art and are described, for example, by H. Neurath and R.L. Hill, 1979, In, The Proteins, Academic Press, New York. Common substitutions are Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/lle, Leu/Val, Ala/Glu, and Asp/Gly.

Alternatively, the amino acid changes are of such a nature that the physico-chemical properties of the polypeptides are altered. For example, amino acid changes may improve the thermal stability of the polypeptide, alter the substrate specificity, change the pH optimum, and the like.

Essential amino acids in a polypeptide can be identified according to procedures known in the art, such as site-directed mutagenesis or alanine-scanning mutagenesis (Cunningham and Wells, 1989, Science 244: 1081-1085). In the latter technique, single alanine mutations are introduced at every residue in the molecule, and the resultant mutant molecules are tested for phytase activity to identify amino acid residues that are critical to the activity of the molecule. See also, Hilton et al., 1996, J. Biol. Chem. 271: 4699-4708. The active site of the enzyme or other biological interaction can also be determined by physical analysis of structure, as determined by such techniques as nuclear magnetic resonance, crystallography, electron diffraction, or photoaffinity labeling, in conjunction with mutation of putative contact site amino acids. See, for example, de Vos et al., 1992, Science 255: 306-312; Smith et al., 1992, J. Mol. Biol. 224: 899-904; Wlodaver et al., 1992, FEBS Lett. 309: 59-64. The identity of essential amino acids can also be inferred from an alignment with a related polypeptide.

The crystal structure of the phytase variant (var400) was solved at a resolution of 1.33 Å. The atomic coordinates of this structure are shown in Figure 5. These atomic coordinates can be used to generate a three-dimensional model depicting the structure of the phytase variant (var400) or homologous structures (such as more variants of the present invention). The three-dimensional structure shows the molecular consequence of the introduced mutations, and it confirms that disulfide bridges between positions 52 and 99, 31 and 177, and 141 and 199 are formed.

In an embodiment, the variant has improved stability under storage conditions compared to the parent enzyme.

In an embodiment, the variant has improved thermostability compared to the parent enzyme.

Examples of variants according to the invention including variants having following substitution in comparison with SEQ ID NO: 2:

N31C/G52C/E57Y/N73P/A99C/N121P/S134Q/K141C/Y155F/T177C/V199C/N203L/
P207T/M273L;

N31C/Q36A/G52C/E57Y/Q60H/L64Q/N73P/A99C/E119S/N121P/M130T/S134Q/L138A/
K141C/Y155F/S161T/S162A/E176P/T177C/T180N/S184Q/P190T/V199C/N203L/P207T/E224Q
/Q230E/R243N/M273L/K336R/T340L;

N31C/Q36A/P43C/W46C/G52C/E57Y/Q60H/L64Q/N73P/A99C/E119S/N121P/M130T/
S134Q/L138A/K141C/Y155F/S161T/S162A/E176P/T177C/T180N/S184Q/P190T/V199C/N203L
/P207T/ E224Q/Q230E/R243N/M273L/N286S/K336R/T340L;

N31C/Q36A/G52C/E57Y/Q60H/L64Q/N73P/A99C/E119S/N121P/P123C/M130C/
S134Q/L138A/K141C/N151S/Y155F/S161T/S162A/E176P/T177C/T180N/S184Q/P190T/
V199C/N203L/P207T/E224Q/Q230E/R243N/M273L/K336R/T340L; and

N31C/Q36A/P43C+W46C+G52C+E57Y+Q60H+L64Q+N73P+A99C+E119S+N121P+P1
23C+M130C+S134Q+L138A/K141C/Y155F/S161T/S162A/E176P/T177C/T180N/S184Q/P190T
/V199C/N203L/ P207T/E224Q/Q230E/R243N/M273L/N286S/K336R/T340L.

### Phytase Polypeptides, Percentage of Identity

In the present context a phytase is a polypeptide having phytase activity, i.e. an enzyme which catalyzes the hydrolysis of phytate (myo-inositol hexakisphosphate) to (1) myo-inositol and/or (2) mono-, di-, tri-, tetra- and/or penta-phosphates thereof and (3) inorganic phosphate.

In the present context the term a phytate substrate encompasses, i.e., phytic acid and any phytate (salt of phytic acid), as well as the phosphates listed under (2) above.

The ENZYME site at the internet (http://www.expasy.ch/enzyme/) is a repository of information relative to the nomenclature of enzymes. It is primarily based on the recommendations of the Nomenclature Committee of the International Union of Biochemistry and Molecular Biology (IUB-MB) and it describes each type of characterized enzyme for which an EC (Enzyme Commission) number has been provided (Bairoch A. The ENZYME database, 2000, Nucleic Acids Res 28:304-305). See also the handbook Enzyme Nomenclature from NC-IUBMB, 1992).

According to the ENZYME site, three different types of phytases are known: a so-called 3-phytase (alternative name 1-phytase; a myo-inositol hexaphosphate 3-phosphohydrolase, EC 3.1.3.8), a so-called 4-phytase (alternative name 6-phytase, name based on 1L-numbering system and not 1D-numbering, EC 3.1.3.26), and a so-called 5-phytase (EC 3.1.3.72). For the purposes of the present invention, all three types are included in the definition of phytase.

In a particular embodiment, the phytases of the invention belong to the family of acid histidine phosphatases, which includes the *Escherichia coli* pH 2.5 acid phosphatase (gene appA) as well as fungal phytases such as *Aspergillus awamorii* phytases A and B (EC: 3.1.3.8) (gene phyA and phyB). The histidine acid phosphatases share two regions of sequence similarity, each centered around a conserved histidine residue. These two histidines seem to be involved in the enzymes' catalytic mechanism. The first histidine is located in the N-terminal section and forms a phosphor-histidine intermediate while the second is located in the C-terminal section and possibly acts as proton donor.

In a further particular embodiment, the phytases of the invention have a conserved active site motif, viz. R-H-G-X-R-X-P, wherein X designates any amino acid (see amino acids 16 to 22 of SEQ ID NOs: 2, 3, 4, 6 and amino acids 38-44 of SEQ ID NO: 9). In a preferred embodiment, the conserved active site motif is R-H-G-V-R-A-P, i.e. amino acids 16-22 (by reference to SEQ ID NO: 2) are RHGVRAP.

For the purposes of the present invention the phytase activity is determined in the unit of FYT, one FYT being the amount of enzyme that liberates 1 micro-mol inorganic ortho-phosphate per min. under the following conditions: pH 5.5; temperature 37°C; substrate: sodium phytate (C₆ H₆O₂₄P₆Na₁₂) in a concentration of 0.0050 mol/l. Suitable phytase assays are the FYT and FTU assays described in Example 1 of WO 00/20569. FTU is for determining phytase activity in feed and premix. Phytase activity may also be determined using the assays of Example 1 ("Determination of phosphatase activity" or "Determination of phytase activity").

In a particular embodiment the phytase of the invention is isolated. The term "isolated" as used herein refers to a polypeptide which is at least 20% pure, preferably at least 40% pure, more preferably at least 60% pure, even more preferably at least 80% pure, most preferably at least 90% pure, and even most preferably at least 95% pure, as determined by SDS-PAGE. In particular, it is preferred that the polypeptides are in "essentially pure form", i.e., that the polypeptide preparation is essentially free of other polypeptide material with which it is natively associated. This can be accomplished, for example, by preparing the polypeptide by means of well-known recombinant methods or by classical purification methods.

The relatedness between two amino acid sequences is described by the parameter "identity". For purposes of the present invention, the alignment of two amino acid sequences is determined by using the Needle program from the EMBOSS package (http://emboss.org) version 2.8.0. The Needle program implements the global alignment algorithm described in Needleman, S. B. and Wunsch, C. D. (1970) J. Mol. Biol. 48, 443-453. The substitution matrix used is BLOSUM62, gap opening penalty is 10, and gap extension penalty is 0.5.

The degree of identity between an amino acid sequence of the present invention ("invention sequence") and the amino acid sequence referred to in the claims (SEQ ID NO: 2) is calculated as the number of exact matches in an alignment of the two sequences, divided by the length of the "invention sequence," or the length of the SEQ ID NO: 2, whichever is the shortest. The result is expressed in percent identity.

An exact match occurs when the "invention sequence" and SEQ ID NO: 2 have identical amino acid residues in the same positions of the overlap (in the alignment example below this is represented by "|"). The length of a sequence is the number of amino acid residues in the sequence (e.g. the length of amino acids 1-411 of SEQ ID NO: 2 is 411).

Example 11 in WO2011/117396 is an example of an alignment of the phytase of SEQ ID NO: 2 and the phytase of SEQ ID NO: 9, and the example illustrates how to calculate the percentage of identity between these two backbones.

In another, purely hypothetical, alignment example below, the overlap is the amino acid sequence "HTWGER-NL" of Sequence 1; or the amino acid sequence "HGWGEDANL" of Sequence 2. In the example a gap is indicated by a "-".

Hypothetical alignment example:

Sequence 1:     ACMSHTWGER-NL
                    | |||  ||
Sequence 2:         HGWGEDANLAMNPS

In a particular embodiment, the percentage of identity of an amino acid sequence of a polypeptide with, or to, SEQ ID NO: 2 is determined by i) aligning the two amino acid sequences using the Needle program, with the BLOSUM62 substitution matrix, a gap opening penalty of 10, and a gap extension penalty of 0.5; ii) counting the number of exact matches in the alignment; iii) dividing the number of exact matches by the length of the shortest of the two amino acid sequences, and iv) converting the result of the division of iii) into percentage.

In the above hypothetical example, the number of exact matches is 6, the length of the shortest one of the two amino acid sequences is 12; accordingly the percentage of identity is 50%.

In particular embodiments of the phytase of the invention, the degree of identity to SEQ ID NO: 2 is at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or at least 99%. In still further particular embodiments, the degree of identity is at least 98.0%, 98.2%, 98.4%, 98.6%, 98.8%, 99.0%, 99.1%, 99.2%, 99.3%, 99.4%, 99.5%, 99.6%, 99.7%, 99.8%, or at least 99.9%. In alternative embodiments, the degree of identity is at least 70%, 71%, 72%, or at least 73%.

In still further particular embodiments, the phytase of the invention has no more than 1, 2, 3, 4, 5, 6, 7, 8, 9, or no more than 10 modifications as compared to SEQ ID NO: 2 or any other parent phytase; no more than 11, 12, 13, 14, 15, 16, 17, 18, 19, or no more than 20 modifications as compared to SEQ ID NO: 2 or any other parent phytase; no more than 21, 22, 23, 24, 25, 26, 27, 28, 29, or no more than 30 modifications as compared to SEQ ID NO: 2 or any other parent phytase; no more than 31, 32, 33, 34, 35, 36, 37, 38, 39, or not more than 40 modifications as compared to SEQ ID NO: 2 or any other parent phytase; no more than 41, 42, 43, 44, 45, 46, 47, 48, 49, or no more than 50 modifications as compared to SEQ ID NO: 2 or any other parent phytase; no more than 51, 52, 53, 54, 55, 56, 57, 58, 59, or no more than 60 modifications as compared to SEQ ID NO: 2 or any other parent phytase; no more than 61, 62, 63, 64, 65, 66, 67, 68, 69, or no more than 70 modifications as compared to SEQ ID NO: 2 or any other parent phytase; no more than 71, 72, 73, 74, 75, 76, 77, 78, 79, or no more than 80 modifications as compared to SEQ ID NO: 2 or any other parent phytase; no more than 81, 82, 83, 84, 85, 86, 87, 88, 89, or no more than 90 modifications as compared to SEQ ID NO: 2 or any other parent phytase; no more than 91, 92, 93, 94, 95, 96, 97, 98, 99, or no more than 100 modifications as compared to SEQ ID NO: 2 or any other parent phytase; no more than 101, 102, 103, 104, 105, 106, 107, 108, 109, or no more than 110 modifications as compared to SEQ ID NO: 2 or any other parent phytase; no more than 111, 112, 113, 114, 115, 116, 117, 118, 119, or no more than 120 modifications as compared to SEQ ID NO: 2 or any other parent phytase; or no more than 121, 122, 123, or 124 modifications as compared to SEQ ID NO: 2 or any other parent phytase.

### Position Numbering

The nomenclature used herein for defining amino acid positions is based on the amino acid sequence of the phytase derived from *Citrobacter braakii* ATCC 51113, the mature sequence of which is given in the sequence listing as SEQ ID NO: 2 (amino acids 1-411 of SEQ ID NO: 2). Accordingly, in the present context, the basis for numbering positions is SEQ ID NO: 2 starting with E1 and ending with E411.

When used herein the term "mature" part (or sequence) refers to that part of the polypeptide which is secreted by a cell which contains, as part of its genetic equipment, a polynucleotide encoding the polypeptide. In other words, the mature polypeptide part refers to that part of the polypeptide which remains after the signal peptide part, as well as a propeptide part, if any, has been cleaved off. The signal peptide part can be predicted by programs known in the art (e.g. SignalP). The expected signal peptide part of SEQ ID NO: 2 is included in the present sequence listing as SEQ ID NO: 8, which is encoded by SEQ ID NO: 7. SEQ ID NO: 2 is the expected mature part. Generally, the first amino acid of the mature part of an enzyme can be determined by N-terminal sequencing of the purified enzyme. Any difference between the signal peptide part and the mature part must then be due to the presence of a propeptide.

### Modifications, such as Substitutions, Deletions, Insertions

A phytase variant can comprise various types of modifications relative to a template (i.e. a reference or comparative amino acid sequence such as SEQ ID NO: 2): An amino acid can be substituted with another amino acid; an amino acid can be deleted; an amino acid can be inserted; as well as any combination of any number of such modifications. In the present context the term "insertion" is intended to cover also N- and/or C-terminal extensions.

The general nomenclature used herein for a single modification is the following: XDcY, where "X" and "Y" independently designate a one-letter amino acid code, or a "*" (deletion of an amino acid), "D" designates a number, and "c" designates an alphabetical counter (a, b, c, and so forth), which is only present in insertions. Reference is made to the below Table which describes purely hypothetical examples of applying this nomenclature to various types of modifications.

**Table**

| Type | Description | Example |
|---|---|---|
| Substitution | X=Amino acid in template | **G80A** |
| | D=Position in template | 80 AALNNSIGVLGVAPSAELYAVKVLGASGSG |
| | c empty | |
| | Y=Amino acid in variant | ;;;;;;;:;;;;;;;;;;;;;;;;;;;;;; AALNNSIAVLGVAPSAELYAVKVLGASGSG |
| Insertion | X="*" | ***80aT *80bY *85aS** |
| | D=Position in template before the insertion |         80   85 AALNNSIG..VLGVA.PSAELYAVKVLGASG ;;;;;;;;  ;;;;; ;;;;;;;;;;;;;;; AALNNSIGTYVLGVASPSAELYAVKVLGASG |
| | c="a" for first insertion at this position, "b" for next, etc. | |
| Deletion | X=Amino acid in template | **V81*** |
| | D=Position in template | 80 |
| | c empty | |
| | Y="*" | AALNNSIGVLGVAPSAELYAVKVLGASGSG ;;;;;;;; ;;;;;;;;;;;;;;;;;;;;; AALNNSIG.LGVAPSAELYAVKVLGASGSG |
| N-terminal extension | Insertions at position "0". | ***0aA *0bT *0cG** |
| | |         1 ...AQSVPWGISRVQ ;;;;;;;;;;;; ATGAQSVPWGISRVQ |
| C-terminal extension | Insertions after the N-terminal amino acid. | ***275aS *275bT** |
| | |                 270  275 ATSLGSTNLYGSGLVNAEAATR.. ;;;;;;;;;;;;;;;;;;;;;; ATSLGSTNLYGSGLVNAEAATRST |

As explained above, the position number ("D") is counted from the first amino acid residue of SEQ ID NO: 2.

Several modifications in the same sequence are separated by "/" (slash), e.g. the designation "1*/2*/3*" means that the amino acids in position number 1, 2, and 3 are all deleted, and the designation "104A/105F" means that the amino acid in position number 104 is substituted by A, and the amino acid in position number 105 is substituted by F.

Alternative modifications are separated by "," (comma), e.g., the designation "119R,K" means that the amino acid in position 119 is substituted with R or K.

The commas used herein in various other enumerations of possibilities mean what they usually do grammatically, viz. often and/or. E.g., the first comma in the listing "53V,Q, 121D, and/or 167Q" denotes an alternative (V or Q), whereas the two next commas should be interpreted as and/or options: 53 V or Q, and/or 121D, and/or 167Q.

In the present context, "at least one" (e.g. modification) means one or more, e.g. 1, 2, 3, 4, 5, 6, 7, 8, 9, or 10 modifications; or 12, 14, 15, 16, 18, 20, 22, 24, 25, 28, or 30 modifications; and so on, up to a maximum number of modifications of 125, 130, 140, 150, 160, 170, 180, 190, or of 200. The phytase variants of the invention, however, still have to be at least 74% identical to SEQ ID NO: 2, this percentage being determined as described above.

A substitution or extension without any indication of what to substitute or extend with refers to the insertion of any natural, or non-natural, amino acid, except the one that occupies this position in the template.

### Identifying Corresponding Position Numbers

As explained above, the mature phytase of *Citrobacter braakii* ATCC 51113 (SEQ ID NO: 2) is used as the standard for position numbering and, thereby, also for the nomenclature.

For another phytase, in particular a phytase variant of the invention, the position corresponding to position D in SEQ ID NO: 2 is found by aligning the two sequences as specified above in the section entitled "Phytase polypeptides, percentage of identity". From the alignment, the position in the sequence of the invention corresponding to position D of SEQ ID NO: 2 can be clearly and unambiguously identified (the two positions on top of each other in the alignment).

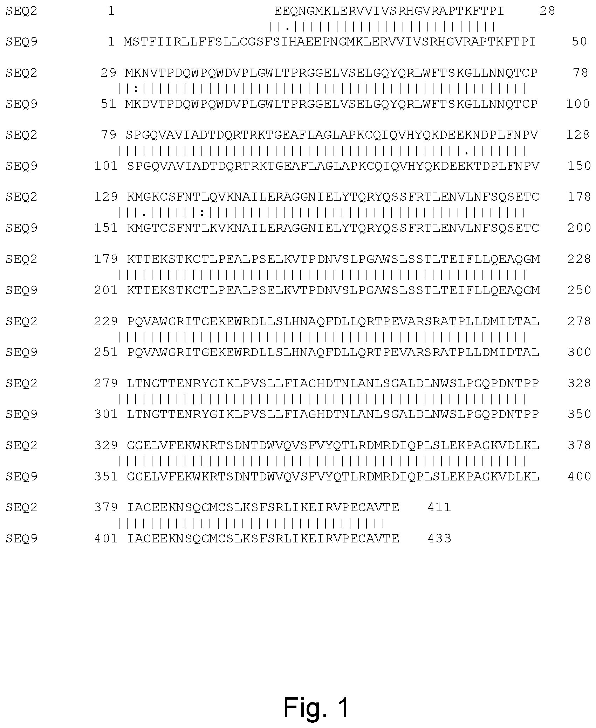

Figure 1 of the present invention is an example of an alignment of the phytase of SEQ ID NO: 2 and the phytase of SEQ ID NO: 9, and the example illustrates how corresponding positions in these two backbones are identified.

Below some additional, purely hypothetical, examples are included which are derived from the above Table which in the third column includes a number of alignments of two sequences.

Consider the third cell in the first row of the above Table: The upper sequence is the template, the lower the variant. Position number 80 refers to amino acid residue G in the template. Amino acid A occupies the corresponding position in the variant. Accordingly, this substitution is designated G80A.

Consider now the third cell in the second row of the above Table: The upper sequence is again the template and the lower the variant. Position number 80 again refers to amino acid residue G in the template. The variant has two insertions, viz. TY, after G80 and before V81 in the template. Whereas the T and Y of course would have their own "real" position number in the variant amino acid sequence, for the present purposes we always refer to the template position numbers, and accordingly the T and the Y are said to be in position number 80a and 80b, respectively.

Finally, consider the third cell in the last row of the above Table: Position number 275 refers to the last amino acid of the template. A C-terminal extension of ST are said to be in position number 275a and 275b, respectively, although, again, of course they have their own "real" position number in the variant amino acid sequence.

### Modified Properties, Reference Phytase

In a particular embodiment, the method of the invention for producing phytase variants provides variants having modified, preferably improved, properties.

The terms "modified" and "improved" imply a comparison with another phytase. Examples of such other, reference, or comparative, phytases are: SEQ ID NO: 2 and/or SEQ ID NO: 6. Still further examples of reference phytases may be SEQ ID NO: 3, and/or SEQ ID NO: 4. A still further example of a reference phytase may be SEQ ID NO: 9, and variants thereof.

Non-limiting examples of properties that are modified, preferably improved, are the following: Thermostability, pH profile, specific activity, performance in animal feed, pelleting stability, protease-sensibility, and/or glycosylation pattern. The phytase variants produced by the method of the invention exhibits improved thermostability and may also have a modified, preferably improved, temperature profile, and/or it may incorporate a change of a potential protease cleavage site.

### Thermal Performance

### Temperature-stability

Temperature stability may be determined as described in WO2011/117396, Example 3 by determining the activity during 30 minutes incubation at temperatures from 60°C or higher and comparing with a reference experiment performed at 37°C.

### Thermostability

Thermostability may be determined as described in WO2011/117396, Example 4, i.e., using DSC measurements to determine the denaturation temperature, Td, of the purified phytase protein. The Td is indicative of the thermostability of the protein: The higher the Td, the higher the thermostability. Accordingly, in a preferred embodiment, the phytase of the invention has a Td which is higher than the Td of a reference phytase, wherein Td is determined on purified phytase samples (preferably with a purity of at least 90% or 95%, determined by SDS-PAGE).

### Heat-stability

Heat stability may be determined as described in WO2011/117396, Example 5 by determining the temperature/activity profile of the variant phytases.

### Steam stability

Steam stability may be determined as described in WO 2011/117396, Example 7 by determining the residual activity of phytase molecules after steam treatment at 85°C or 90°C for a short time.

### Pelleting stability

Pelleting stability may be determined as described in WO 2001/117396, Example 8 by using enzyme granulate pre-mixed with feed. This premix is mixed with feed. From the mixer the feed is conditioned with steam to 95°C. After conditioning the feed is pressed to pellets and the residual activity determined.

In preferred embodiments, the thermal properties such as heat-stability, temperature stability, thermostability, steam stability, and/or pelleting stability as provided by the residual activity, Td or other parameter of the phytase of the invention is higher than the corresponding value, such as the residual activity or Td, of the phytase of SEQ ID NO: 2, more preferably at least 101% thereof, or at least 102%, 103%, 104%, 105%, 106%, 107%, 108%, 109%, or at least 110% thereof. Even more preferably, the value of the parameter, such as residual activity or Td, of the phytase of the invention is at least 120%, 130%, 140%, 150%, 160%, 170%, 180%, or at least 190% of the value for the phytase of SEQ ID NO: 2.

In still further particular embodiments, the thermostable phytase of the invention has a melting temperature, Tm (or a denaturation temperature, Td), as determined using Differential Scanning Calorimetry (DSC) as described in the Examples (i.e. in 20 mM sodium acetate, pH 4.0), of at least 50°C. In still further particular embodiments, the Tm is at least 51, 52, 53, 54, 55, 56, 57, 58, 59, 60, 61, 62, 62.5. 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 or at least 100°C. DSC measurements may also be performed as described in the Examples.

### Temperature profile/temperature stability

Whether or not a phytase of the invention has a modified temperature profile as compared to a reference phytase may be determined as described in WO 2011/117396, Example 5. Accordingly, in a particular embodiment the phytase of the invention has a modified temperature profile as compared to a reference phytase, wherein the temperature profile is determined as phytase activity as a function of temperature on sodium phytate at pH 5.5 in the temperature range of 20-90°C (in 10°C steps). A preferred buffer is in 0.25 M Na-acetate buffer pH 5.5. The activity at each temperature is preferably indicated as relative activity (in %) normalized to the value at optimum temperature. The optimum temperature is that temperature within the tested temperatures (i.e. those with 5-10°C jumps) where the activity is highest.

### Performance in animal feed

In a particular embodiment the phytase of the invention has an improved performance in animal feed as compared to a reference phytase. The performance in animal feed may be determined by the *in vitro* model. Accordingly, in a preferred embodiment the phytase of the invention has an improved performance in animal feed, wherein the performance is determined in an *in vitro* model, by preparing feed samples composed of 30% soybean meal and 70% maize meal with added CaCl₂ to a concentration of 5 g calcium per kg feed; pre-incubating them at 40°C and pH 3.0 for 30 minutes followed by addition of pepsin (3000 U/g feed) and phytase; incubating the samples at 40°C and pH 3.0 for 60 minutes followed by pH 4.0 for 30 minutes; stopping the reactions; extracting phytic acid and inositol-phosphates by addition of HCl to a final concentration of 0.5M and incubation at 40°C for 2 hours, followed by one freeze-thaw cycle and 1 hour incubation at 40°C; separating phytic acid and inositol-phosphates by high performance ion chromatography; determining the amount of residual phytate phosphorus (IP6-P); calculating the difference in residual IP6-P between the phytase-treated and a non-phytase-treated blank sample (this difference is degraded IP6-P); and expressing the degraded IP6-P of the phytase of the invention relative to degraded IP6-P of the reference phytase.

The phytase of the invention and the reference phytase are dosed in the same amount, preferably based on phytase activity units (FYT). A suitable dosage is 100-5000 FYT/kg feed, such as 125 to 4000 FTY/kg feed, such as 125 to 3000 FTY/kg. The phytases may be dosed in the form of purified phytases, or in the form of fermentation supernatants. Purified phytases preferably have a purity of at least 95%, as determined by SDS-PAGE.

In preferred embodiments, the degraded IP6-P value of the purified phytase of the invention, relative to the degraded IP6-P value of the reference phytase, is at least 101%, or at least 102%, 103%, 104%, 105%, 110%, 115%, or at least 120%. In still further preferred embodiments, the degraded IP6-P value of the purified phytase of the invention, relative to the degraded IP6-P value of the reference phytase, is at least 125%, 130%, 140%, 150%, 160%, 170%, 180%, 190%, or at least 200%. Preferably, the degraded IP6-P value of the phytase of the invention, relative to the degraded IP6-P value of the SEQ ID NO: 2 phytase, is at least 105%, 110%, 113%, 115%, 120%, 125%, or at least 130%.

The relative performance of a phytase of the invention may also be calculated as the percentage of the phosphorous released by the reference phytase.

In a still further particular embodiment, the relative performance of the phytase of the invention may be calculated as the percentage of the phosphorous released by the phytase of the invention, relative to the amount of phosphorous released by the reference phytase.

In still further particular embodiments, the relative performance of the phytase of the invention is at least 105%, preferably at least 110, 120, 130, 140, 150, 160, 170, 180, 190, or at least 200%.

### Steam stability

Thermostability is an important parameter, but associated with that also steam stability is important. In this respect reference is made to WO 2011/117396, Example 8.

### Low-allergenic variants

In a specific embodiment, the phytase variants produced by the method of the present invention are (also) low-allergenic variants, designed to invoke a reduced immunological response when exposed to animals, including man. The term immunological response is to be understood as any reaction by the immune system of an animal exposed to the phytase variant. One type of immunological response is an allergic response leading to increased levels of IgE in the exposed animal. Low-allergenic variants may be prepared using techniques known in the art. For example the phytase variant may be conjugated with polymer moieties shielding portions or epitopes of the phytase variant involved in an immunological response. Conjugation with polymers may involve *in vitro* chemical coupling of polymer to the phytase variant, e.g. as described in WO 96/17929, WO 98/30682, WO 98/35026, and/or WO 99/00489. Conjugation may in addition or alternatively thereto involve in vivo coupling of polymers to the phytase variant. Such conjugation may be achieved by genetic engineering of the nucleotide sequence encoding the phytase variant, inserting consensus sequences encoding additional glycosylation sites in the phytase variant and expressing the phytase variant in a host capable of glycosylating the phytase variant, see e.g. WO 00/26354. Another way of providing low-allergenic variants is genetic engineering of the nucleotide sequence encoding the phytase variant so as to cause the phytase variants to selfoligomerize, effecting that phytase variant monomers may shield the epitopes of other phytase variant monomers and thereby lowering the antigenicity of the oligomers. Such products and their preparation are described e.g. in WO 96/16177. Epitopes involved in an immunological response may be identified by various methods such as the phage display method described in WO 00/26230 and WO 01/83559, or the random approach described in EP 561907. Once an epitope has been identified, its amino acid sequence may be altered to produce altered immunological properties of the phytase variant by known gene manipulation techniques such as site directed mutagenesis (see e.g. WO 00/26230, WO 00/26354 and/or WO 00/22103) and/or conjugation of a polymer may be done in sufficient proximity to the epitope for the polymer to shield the epitope.

### Daily BW gain per bird (BW gain) and feed conversion ratio (FCR)

Daily Body weight gain per bird (BW gain) and feed conversion ratio (FCR) were calculated as follows:
Body weight gain per bird: difference between BW per bird at the end and at the beginning of a study
Daily BWgain: difference between BW per bird at the end and at the beginning of the study divided by the number of days
FCR: total feed consumption of a pen divided by total BW gain of that pen (total BW gain = total BW at the end + weight of removals and losses - total BW at the beginning).$FI = FCR * WG$

### Nucleic Acid Sequences and Constructs

The present invention also relates to nucleic acid sequences comprising a nucleic acid sequence which encodes a phytase variant of the invention.

The term "isolated nucleic acid sequence" refers to a nucleic acid sequence which is essentially free of other nucleic acid sequences, e.g., at least about 20% pure, preferably at least about 40% pure, more preferably at least about 60% pure, even more preferably at least about 80% pure, and most preferably at least about 90% pure as determined by agarose electrophoresis. For example, an isolated nucleic acid sequence can be obtained by standard cloning procedures used in genetic engineering to relocate the nucleic acid sequence from its natural location to a different site where it will be reproduced. The cloning procedures may involve excision and isolation of a desired nucleic acid fragment comprising the nucleic acid sequence encoding the polypeptide, insertion of the fragment into a vector molecule, and incorporation of the recombinant vector into a host cell where multiple copies or clones of the nucleic acid sequence will be replicated. The nucleic acid sequence may be of genomic, cDNA, RNA, semisynthetic, synthetic origin, or any combinations thereof.

The nucleic acid sequences of the invention can be prepared by introducing at least one mutation into a template phytase coding sequence or a subsequence thereof, wherein the mutant nucleic acid sequence encodes a variant phytase. The introduction of a mutation into the nucleic acid sequence to exchange one nucleotide for another nucleotide may be accomplished by any of the methods known in the art, e.g. by site-directed mutagenesis, by random mutagenesis, or by doped, spiked, or localized random mutagenesis.

Random mutagenesis is suitably performed either as localized or region-specific random mutagenesis in at least three parts of the gene translating to the amino acid sequence shown in question, or within the whole gene. When the mutagenesis is performed by the use of an oligonucleotide, the oligonucleotide may be doped or spiked with the three non-parent nucleotides during the synthesis of the oligonucleotide at the positions which are to be changed. The doping or spiking may be performed so that codons for unwanted amino acids are avoided. The doped or spiked oligonucleotide can be incorporated into the DNA encoding the phytase enzyme by any technique, using, e.g., PCR, LCR or any DNA polymerase and ligase as deemed appropriate.

Preferably, the doping is carried out using "constant random doping", in which the percentage of wild-type and mutation in each position is predefined. Furthermore, the doping may be directed toward a preference for the introduction of certain nucleotides, and thereby a preference for the introduction of one or more specific amino acid residues. The doping may be made, e.g., so as to allow for the introduction of 90% wild type and 10% mutations in each position. An additional consideration in the choice of a doping scheme is based on genetic as well as protein-structural constraints.

The random mutagenesis may be advantageously localized to a part of the parent phytase in question. This may, e.g., be advantageous when certain regions of the enzyme have been identified to be of particular importance for a given property of the enzyme.

Alternative methods for providing variants of the invention include gene shuffling e.g. as described in WO 95/22625 or in WO 96/00343, and the consensus derivation process as described in EP 897985.

### Nucleic Acid Constructs

A nucleic acid construct comprises a nucleic acid sequence of the present invention operably linked to one or more control sequences which direct the expression of the coding sequence in a suitable host cell under conditions compatible with the control sequences. Expression will be understood to include any step involved in the production of the polypeptide including, but not limited to, transcription, post-transcriptional modification, translation, posttranslational modification, and secretion.

The term "nucleic acid construct" as used herein refers to a nucleic acid molecule, either single- or double-stranded, which is isolated from a naturally occurring gene or which is modified to contain segments of nucleic acids in a manner that would not otherwise exist in nature. The term nucleic acid construct is synonymous with the term "expression cassette" when the nucleic acid construct contains the control sequences required for expression of a coding sequence of the present invention.

The term "control sequences" is defined herein to include all components, which are necessary or advantageous for the expression of a polynucleotide encoding a polypeptide of the present invention. Each control sequence may be native or foreign to the nucleotide sequence encoding the polypeptide. Such control sequences include, but are not limited to, a leader, polyadenylation sequence, propeptide sequence, promoter, signal peptide sequence, and transcription terminator. At a minimum, the control sequences include a promoter, and transcriptional and translational stop signals. The control sequences may be provided with linkers for the purpose of introducing specific restriction sites facilitating ligation of the control sequences with the coding region of the nucleotide sequence encoding a polypeptide.

The term "operably linked" denotes herein a configuration in which a control sequence is placed at an appropriate position relative to the coding sequence of the polynucleotide sequence such that the control sequence directs the expression of the coding sequence of a polypeptide.

When used herein the term "coding sequence" (CDS) means a nucleotide sequence, which directly specifies the amino acid sequence of its protein product. The boundaries of the coding sequence are generally determined by an open reading frame, which usually begins with the ATG start codon or alternative start codons such as GTG and TTG. The coding sequence may a DNA, cDNA, or recombinant nucleotide sequence.

### Expression Vector

The term "expression" includes any step involved in the production of the polypeptide including, but not limited to, transcription, post-transcriptional modification, translation, posttranslational modification, and secretion.

The term "expression vector" is defined herein as a linear or circular DNA molecule that comprises a polynucleotide encoding a polypeptide of the invention, and which is operably linked to additional nucleotides that provide for its expression.

A nucleic acid sequence encoding a phytase variant of the invention can be expressed using an expression vector which typically includes control sequences encoding a promoter, operator, ribosome binding site, translation initiation signal, and, optionally, a repressor gene or various activator genes.

The recombinant expression vector carrying the DNA sequence encoding a phytase variant of the invention may be any vector which may conveniently be subjected to recombinant DNA procedures, and the choice of vector will often depend on the host cell into which it is to be introduced. The vector may be one which, when introduced into a host cell, is integrated into the host cell genome and replicated together with the chromosome(s) into which it has been integrated.

The phytase variant may also be co-expressed together with at least one other enzyme of animal feed interest, such as a phytase, phosphatase, xylanase, galactanase, alpha-galactosidase, protease, phospholipase, amylase, and/or beta-glucanase. The enzymes may be co-expressed from different vectors, from one vector, or using a mixture of both techniques. When using different vectors, the vectors may have different selectable markers, and different origins of replication. When using only one vector, the genes can be expressed from one or more promoters. If cloned under the regulation of one promoter (di- or multi-cistronic), the order in which the genes are cloned may affect the expression levels of the proteins. The phytase variant may also be expressed as a fusion protein, i.e. that the gene encoding the phytase variant has been fused in frame to the gene encoding another protein. This protein may be another enzyme or a functional domain from another enzyme.

### Host Cells

The term "host cell", as used herein, includes any cell type which is susceptible to transformation, transfection, transduction, and the like with a nucleic acid construct comprising a polynucleotide of the present invention.

The present invention also relates to recombinant host cells, comprising a polynucleotide of the present invention, which are advantageously used in the recombinant production of the polypeptides. A vector comprising a polynucleotide of the present invention is introduced into a host cell so that the vector is maintained as a chromosomal integrant or as a self-replicating extrachromosomal vector as described earlier. The term "host cell" encompasses any progeny of a parent cell that is not identical to the parent cell due to mutations that occur during replication. The choice of a host cell will to a large extent depend upon the gene encoding the polypeptide and its source.

The host cell may be a unicellular microorganism, e.g., a prokaryote, or a non-unicellular microorganism, e.g., a eukaryote.

Useful unicellular microorganisms are bacterial cells such as gram positive bacteria including, but not limited to, a *Bacillus cell,* e.g., *Bacillus alkalophilus, Bacillus amyloliquefaciens, Bacillus brevis, Bacillus circulans, Bacillus clausii, Bacillus coagulans, Bacillus lautus, Bacillus lentus, Bacillus licheniformis, Bacillus megaterium, Bacillus stearothermophilus, Bacillus subtilis,* and *Bacillus thuringiensis;* or a *Streptomyces* cell, e.g., *Streptomyces lividans* and *Streptomyces murinus,* or gram negative bacteria such as *E. coli* and *Pseudomonas* sp. In a preferred aspect, the bacterial host cell is a *Bacillus lentus, Bacillus licheniformis, Bacillus stearothermophilus,* or *Bacillus subtilis* cell. In another preferred aspect, the *Bacillus* cell is an alkalophilic *Bacillus.*

The introduction of a vector into a bacterial host cell may, for instance, be effected by protoplast transformation (see, e.g., Chang and Cohen, 1979, Molecular General Genetics 168: 111-115), using competent cells (see, e.g., Young and Spizizin, 1961, Journal of Bacteriology 81: 823-829, or Dubnau and Davidoff-Abelson, 1971, Journal of Molecular Biology 56: 209-221), electroporation (see, e.g., Shigekawa and Dower, 1988, Biotechniques 6: 742-751), or conjugation (see, e.g., Koehler and Thorne, 1987, Journal of Bacteriology 169: 5771-5278).

The host cell may also be a eukaryote, such as a mammalian, insect, plant, or fungal cell.

In a preferred aspect, the host cell is a fungal cell. "Fungi" as used herein includes the phyla *Ascomycota, Basidiomycota, Chytridiomycota,* and *Zygomycota* (as defined by Hawksworth et al., In, Ainsworth and Bisby's Dictionary of The Fungi, 8th edition, 1995, CAB International, University Press, Cambridge, UK) as well as the Oomycota (as cited in Hawksworth et al., 1995, supra, page 171) and all mitosporic fungi (Hawksworth et al., 1995, supra).

In a more preferred aspect, the fungal host cell is a yeast cell. "Yeast" as used herein includes ascosporogenous yeast (Endomycetales), basidiosporogenous yeast, and yeast belonging to the Fungi Imperfecti (Blastomycetes). Since the classification of yeast may change in the future, for the purposes of this invention, yeast shall be defined as described in Biology and Activities of Yeast (Skinner, F.A., Passmore, S.M., and Davenport, R.R., eds, Soc. App. Bacteriol. Symposium Series No. 9, 1980).

In an even more preferred aspect, the yeast host cell is a *Candida, Hansenula, Kluyveromyces, Pichia, Saccharomyces, Schizosaccharomyces,* or *Yarrowia* cell.

In a most preferred aspect, the yeast host cell is a *Pichia pastoris, Pichia methanolica, Saccharomyces carlsbergensis, Saccharomyces cerevisiae, Saccharomyces diastaticus, Saccharomyces douglasii, Saccharomyces kluyveri, Saccharomyces norbensis* or *Saccharomyces oviformis* cell. In another most preferred aspect, the yeast host cell is a *Kluyveromyces lactis* cell. In another most preferred aspect, the yeast host cell is a *Yarrowia lipolytica* cell.

In another more preferred aspect, the fungal host cell is a filamentous fungal cell. "Filamentous fungi" include all filamentous forms of the subdivision Eumycota and Oomycota (as defined by Hawksworth et al., 1995, supra). The filamentous fungi are generally characterized by a mycelial wall composed of chitin, cellulose, glucan, chitosan, mannan, and other complex polysaccharides. Vegetative growth is by hyphal elongation and carbon catabolism is obligately aerobic. In contrast, vegetative growth by yeasts such as Saccharomyces cerevisiae is by budding of a unicellular thallus and carbon catabolism may be fermentative.

In an even more preferred aspect, the filamentous fungal host cell is an *Acremonium, Aspergillus, Aureobasidium, Bjerkandera, Ceriporiopsis, Coprinus, Coriolus, Cryptococcus, Filobasidium, Fusarium, Humicola, Magnaporthe, Mucor, Myceliophthora, Neocallimastix, Neurospora, Paecilomyces, Penicillium, Phanerochaete, Phlebia, Piromyces, Pleurotus, Schizophyllum, Talaromyces, Thermoascus, Thielavia, Tolypocladium, Trametes,* or *Trichoderma* cell.

In a most preferred aspect, the filamentous fungal host cell is an *Aspergillus awamori, Aspergillus fumigatus, Aspergillus foetidus, Aspergillus japonicus, Aspergillus nidulans, Aspergillus niger* or *Aspergillus oryzae* cell. In another most preferred aspect, the filamentous fungal host cell is a *Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides,* or *Fusarium venenatum* cell. In another most preferred aspect, the filamentous fungal host cell is a *Bjerkandera adusta, Ceriporiopsis aneirina, Ceriporiopsis aneirina, Ceriporiopsis caregiea, Ceriporiopsis gilvescens, Ceriporiopsis pannocinta, Ceriporiopsis rivulosa, Ceriporiopsis subrufa,* or *Ceriporiopsis subvermispora, Coprinus cinereus, Coriolus hirsutus, Humicola insolens, Humicola lanuginosa, Mucor miehei, Myceliophthora thermophila, Neurospora crassa, Penicillium purpurogenum, Phanerochaete chrysosporium, Phlebia radiata, Pleurotus eryngii, Thielavia terrestris, Trametes villosa, Trametes versicolor, Trichoderma harzianum, Trichoderma koningii, Trichoderma longibrachiatum, Trichoderma reesei,* or *Trichoderma viride* strain cell.

Fungal cells may be transformed by a process involving protoplast formation, transformation of the protoplasts, and regeneration of the cell wall in a manner known per se. Suitable procedures for transformation of *Aspergillus* and *Trichoderma* host cells are described in EP 238 023 and Yelton et al., 1984, Proceedings of the National Academy of Sciences USA 81: 1470-1474. Suitable methods for transforming Fusarium species are described by Malardier et al., 1989, Gene 78: 147-156, and WO 96/00787. Yeast may be transformed using the procedures described by Becker and Guarente, In Abelson, J.N. and Simon, M.I., editors, Guide to Yeast Genetics and Molecular Biology, Methods in Enzymology, Volume 194, pp 182-187, Academic Press, Inc., New York; Ito et al., 1983, Journal of Bacteriology 153: 163; and Hinnen et al., 1978, Proceedings of the National Academy of Sciences USA 75: 1920.

### Methods of Production

The present invention relates to methods for producing a phytase variant comprising (a) cultivating a host cell under conditions conducive for production of the phytase; and (b) recovering the phytase.

In the production methods of the present invention, the cells are cultivated in a nutrient medium suitable for production of the polypeptide using methods well known in the art. For example, the cell may be cultivated by shake flask cultivation, and small-scale or large-scale fermentation (including continuous, batch, fed-batch, or solid state fermentations) in laboratory or industrial fermentors performed in a suitable medium and under conditions allowing the polypeptide to be expressed and/or isolated. The cultivation takes place in a suitable nutrient medium comprising carbon and nitrogen sources and inorganic salts, using procedures known in the art. Suitable media are available from commercial suppliers or may be prepared according to published compositions (e.g., in catalogues of the American Type Culture Collection). If the polypeptide is secreted into the nutrient medium, the polypeptide can be recovered directly from the medium. If the polypeptide is not secreted, it can be recovered from cell lysates.

The resulting polypeptide may be recovered using methods known in the art. For example, the polypeptide may be recovered from the nutrient medium by conventional procedures including, but not limited to, centrifugation, filtration, extraction, spray-drying, evaporation, or precipitation.

The polypeptides of the present invention may be purified by a variety of procedures known in the art including, but not limited to, chromatography (e.g., ion exchange, affinity, hydrophobic, chromatofocusing, and size exclusion), electrophoretic procedures (e.g., preparative isoelectric focusing), differential solubility (e.g., ammonium sulfate precipitation), SDS-PAGE, or extraction (see, e.g., Protein Purification, J.-C. Janson and Lars Ryden, editors, VCH Publishers, New York, 1989).

### Compositions and Uses

In still further aspects, the present invention relates to compositions comprising a polypeptide of the present invention, as well as methods of using these.

The polypeptide compositions may be prepared in accordance with methods known in the art and may be in the form of a liquid or a dry composition. The polypeptide to be included in the composition may be stabilized in accordance with methods known in the art.

For a liquid formulation, the formulating agent may comprise a polyol (such as e.g. glycerol, ethylene glycol or propylene glycol), a salt (such as e.g. sodium chloride, sodium benzoate, potassium sorbate) or a sugar or sugar derivative (such as e.g. dextrin, glucose, sucrose, and sorbitol). Thus in one embodiment, the composition is a liquid composition comprising the polypeptide of the invention and one or more formulating agents selected from the list consisting of glycerol, ethylene glycol, 1,2-propylene glycol, 1,3-propylene glycol, sodium chloride, sodium benzoate, potassium sorbate, dextrin, glucose, sucrose, and sorbitol. The liquid formulation may be sprayed onto the feed after it has been pelleted or may be added to drinking water given to the animals.

For a solid formulation, the formulation may be for example as a granule, spray dried powder or agglomerate. The formulating agent may comprise a salt (organic or inorganic zinc, sodium, potassium or calcium salts such as e.g. such as calcium acetate, calcium benzoate, calcium carbonate, calcium chloride, calcium citrate, calcium sorbate, calcium sulfate, potassium acetate, potassium benzoate, potassium carbonate, potassium chloride, potassium citrate, potassium sorbate, potassium sulfate, sodium acetate, sodium benzoate, sodium carbonate, sodium chloride, sodium citrate, sodium sulfate, zinc acetate, zinc benzoate, zinc carbonate, zinc chloride, zinc citrate, zinc sorbate, zinc sulfate), starch or a sugar or sugar derivative (such as e.g. sucrose, dextrin, glucose, lactose, sorbitol).

In an embodiment, the solid composition is in the form of granulates or microgranulates. The granule may have a matrix structure where the components are mixed homogeneously. However, the granule typically comprises a core particle and one or more coatings, which typically are salt and/or wax coatings. Examples of waxes are polyethylene glycols; polypropylenes; Carnauba wax; Candelilla wax; bees wax; hydrogenated plant oil or animal tallow such as hydrogenated ox tallow, hydrogenated palm oil, hydrogenated cotton seeds and/or hydrogenated soy bean oil; fatty acid alcohols; mono-glycerides and/or di-glycerides, such as glyceryl stearate, wherein stearate is a mixture of stearic and palmitic acid; micro-crystalline wax; paraffin's; and fatty acids, such as hydrogenated linear long chained fatty acids and derivatives thereof. A preferred wax is palm oil or hydrogenated palm oil. The core particle can either be a homogeneous blend of phytase of the invention optionally combined with one or more additional enzymes and optionally together with one or more salts or an inert particle with the phytase of the invention optionally combined with one or more additional enzymes applied onto it.

In an embodiment, the material of the core particles are selected from the group consisting of inorganic salts (such as calcium acetate, calcium benzoate, calcium carbonate, calcium chloride, calcium citrate, calcium sorbate, calcium sulfate, potassium acetate, potassium benzoate, potassium carbonate, potassium chloride, potassium citrate, potassium sorbate, potassium sulfate, sodium acetate, sodium benzoate, sodium carbonate, sodium chloride, sodium citrate, sodium sulfate, zinc acetate, zinc benzoate, zinc carbonate, zinc chloride, zinc citrate, zinc sorbate, zinc sulfate), starch or a sugar or sugar derivative (such as e.g. sucrose, dextrin, glucose, lactose, sorbitol), sugar or sugar derivative (such as e.g. sucrose, dextrin, glucose, lactose, sorbitol), small organic molecules, starch, flour, cellulose and minerals and clay minerals (also known as hydrous aluminium phyllosilicates). In a preferred embodiment, the core comprises a clay mineral such as kaolinite or kaolin.

The salt coating is typically at least 1 µm thick and can either be one particular salt or a mixture of salts, such as Na₂SO₄, K₂SO₄, MgSO₄ and/or sodium citrate. Other examples are those described in e.g. WO 2008/017659, WO 2006/034710, WO 1997/05245, WO 1998/54980, WO 1998/55599, WO 2000/70034 or polymer coating such as described in WO 2001/00042.

In another embodiment, the composition is a solid composition comprising the phytase of the invention and one or more formulating agents selected from the list consisting of sodium chloride, sodium benzoate, potassium sorbate, sodium sulfate, potassium sulfate, magnesium sulfate, sodium thiosulfate, calcium carbonate, sodium citrate, dextrin, glucose, sucrose, sorbitol, lactose, starch and cellulose. In a preferred embodiment, the formulating agent is selected from one or more of the following compounds: sodium sulfate, dextrin, cellulose, sodium thiosulfate and calcium carbonate. In a preferred embodiment, the solid composition is in granulated form. In an embodiment, the solid composition is in granulated form and comprises a core particle, an enzyme layer comprising the phytase of the invention and a salt coating.

In a further embodiment, the formulating agent is selected from one or more of the following compounds: glycerol, ethylene glycol, 1, 2-propylene glycol or 1, 3-propylene glycol, sodium chloride, sodium benzoate, potassium sorbate, sodium sulfate, potassium sulfate, magnesium sulfate, sodium thiosulfate, calcium carbonate, sodium citrate, dextrin, glucose, sucrose, sorbitol, lactose, starch, kaolin and cellulose. In a preferred embodiment, the formulating agent is selected from one or more of the following compounds: 1, 2-propylene glycol, 1, 3-propylene glycol, sodium sulfate, dextrin, cellulose, sodium thiosulfate, kaolin and calcium carbonate.

The phytase of the invention can be used for degradation, in any industrial context, of, for example, phytate, phytic acid, and/or the mono-, di-, tri-, tetra- and/or penta-phosphates of myo-inositol. It is well known that the phosphate moieties of these compounds chelates divalent and trivalent cations such as metal ions, i.e. the nutritionally essential ions of calcium, iron, zinc and magnesium as well as the trace minerals manganese, copper and molybdenum. Besides, the phytic acid also to a certain extent binds proteins by electrostatic interaction.

Accordingly, preferred uses of the polypeptides or polynucleotides of the invention are in animal feed preparations (including human food) or in additives for such preparations.

In a particular embodiment, the polypeptide or polynucleotide of the invention can be used for improving the nutritional value of an animal feed. Non-limiting examples of improving the nutritional value of animal feed (including human food), are: improving feed digestibility; promoting growth of the animal; improving feed utilization; improving bio-availability of proteins; increasing the level of digestible phosphate; improving the release and/or degradation of phytate; improving bio-availability of trace minerals; improving bio-availability of macro minerals; eliminating the need for adding supplemental phosphate, trace minerals, and/or macro minerals; and/or improving egg shell quality. The nutritional value of the feed is therefore increased, and the growth rate and/or weight gain and/or feed conversion (i.e. the weight of ingested feed relative to weight gain) of the animal is/are improved. In another particular embodiment, the polypeptide or polynucleotide of the invention can be used for improving nutrient retention, and/or nutrient digestibility in an animal.

Furthermore, the polypeptide or polynucleotide of the invention can be used for reducing phytate level of manure.

### Animals, Animal Feed, and Animal Feed Additives

The term animal includes all animals, including human beings. Examples of animals are non-ruminants, and ruminants. Ruminant animals include, for example, animals such as sheep, goat, and cattle, e.g. cow such as beef cattle and dairy cows. In a particular embodiment, the animal is a non-ruminant animal. Non-ruminant animals include mono-gastric animals, e.g. pig or swine (including, but not limited to, piglets, growing pigs, and sows); poultry such as turkeys, ducks and chickens (including but not limited to broiler chicks, layers); fish (including but not limited to salmon, trout, tilapia, catfish and carp); and crustaceans (including but not limited to shrimp and prawn).

The term feed or feed composition means any compound, preparation, mixture, or composition suitable for, or intended for intake by an animal.

In the use according to the invention the polypeptide or polynucleotide can be fed to the animal before, after, or simultaneously with the diet. The latter is preferred.

The invention further relates to a method of enhancing one or more of the group selected from growth rate, phosphorus digestibility, whole-body phosphorus retention and/or reducing the FCR in an animal, said method comprising feeding the animal the phytase as defined herein. The method of the invention typically comprises phytase supplementation doses of 100-5000 FYT/kg feed, such as 125 to 4000 FTY/kg feed, such as 125 to 3000 FTY/kg feed.

The invention is further directed to a method of enhancing the growth rate or reducing the FCR in a mono-gastric animal, said method comprising feeding the animal the phytase as defined herein. The method of the invention typically comprises phytase supplementation doses of 100-5000 FYT/kg feed, such as 125 to 4000 FTY/kg feed, such as 125 to 3000 FTY/kg feed.

The invention is further directed to a method of enhancing the growth rate or reducing the FCR in an animal selected from the group consisting of poultry, swine, fish or crustacean, said method comprising feeding the animal the phytase as defined herein. The method of the invention typically comprises phytase supplementation doses of 100-5000 FYT/kg feed, such as 125 to 4000 FTY/kg feed, such as 125 to 3000 FTY/kg feed.

The invention is further directed to a method of enhancing the growth rate or reducing the FCR in poultry, said method comprising feeding the poultry the phytase as defined herein. The poultry may be typically selected from the group consisting of turkeys, ducks and chickens (including but not limited to broiler chicks, layers), typically chickens, particularly broiler chickens and layer chickens. The method of the invention typically comprises phytase supplementation doses of 100-5000 FYT/kg feed, such as 125 to 4000 FTY/kg feed, such as 125 to 3000 FTY/kg feed.

The invention is further directed to a method of enhancing the growth rate or reducing the FCR in swine, said method comprising feeding the swine the phytase as defined herein. The method of the invention typically comprises phytase supplementation doses of 100-5000 FYT/kg feed, such as 125 to 4000 FTY/kg feed, such as 125 to 3000 FTY/kg feed.

The invention is further directed to a method of enhancing the growth rate or reducing the FCR in fish or crustaceans said method comprising feeding the fish or crustaceans the phytase as defined herein. The fish may typically be selected from the group consisting of salmon, trout, tilapia, catfish, seabream such as gilthead seabream, bass, such as seabass, and carp. The crustaceans may typically be selected from the group consisting of lobster, crab, crayfish, krill, shrimp and prawn. The method of the invention typically comprises phytase supplementation doses of 100-5000 FYT/kg feed, such as 125 to 4000 FTY/kg feed, such as 125 to 3000 FTY/kg feed.

In a particular embodiment, the polypeptide, in the form in which it is added to the feed, or when being included in a feed additive, is substantially pure. In a particular embodiment it is well-defined. The term "well-defined" means that the phytase preparation is at least 50% pure as determined by Size-exclusion chromatography (see Example 12 of WO 01/58275). In other particular embodiments the phytase preparation is at least 60, 70, 80, 85, 88, 90, 92, 94, or at least 95% pure as determined by this method.

A substantially pure, and/or well-defined polypeptide preparation is advantageous. For instance, it is much easier to dose correctly to the feed a polypeptide that is essentially free from interfering or contaminating other polypeptides. The term dose correctly refers in particular to the objective of obtaining consistent and constant results, and the capability of optimising dosage based upon the desired effect.

For the use in animal feed, however, the phytase polypeptide of the invention need not be that pure; it may e.g. include other polypeptides, in which case it could be termed a phytase preparation.

The phytase preparation can be (a) added directly to the feed (or used directly in a treatment process of proteins), or (b) it can be used in the production of one or more intermediate compositions such as feed additives or premixes that is subsequently added to the feed (or used in a treatment process). The degree of purity described above refers to the purity of the original polypeptide preparation, whether used according to (a) or (b) above.

Polypeptide preparations with purities of this order of magnitude are in particular obtainable using recombinant methods of production, whereas they are not so easily obtained and also subject to a much higher batch-to-batch variation when the polypeptide is produced by traditional fermentation methods.

Such polypeptide preparation may of course be mixed with other polypeptides.

The polypeptide can be added to the feed in any form, be it as a relatively pure polypeptide, or in admixture with other components intended for addition to animal feed, i.e. in the form of animal feed additives, such as the so-called pre-mixes for animal feed.

In a further aspect the present invention relates to compositions for use in animal feed, such as animal feed, and animal feed additives, e.g. premixes.

A further aspect of the invention is directed to an animal feed additive comprising the phytase, as defined herein. The animal feed additive may be for use in a feed for a mono-gastric or ruminant, typically a monogastric animal. The animal feed additive is for use in a animal feed for an animal typically selected from the group consisting poultry, swine, fish or crustacean. The animal feed additive typically comprises the phytase in amount 100-5000 FYT/kg feed, such as 125 to 4000 FTY/kg feed, such as 125 to 3000 FTY/kg feed.

The invention is further directed to an animal feed additive comprising the phytase, as defined herein, for use in a feed for poultry. The feed additive for poultry is typically for feed for poultry selected from the group consisting of turkeys, ducks and chickens (including but not limited to broiler chicks, layers), typically chickens, particularly broiler chickens and layer chickens.

The invention is further directed to to an animal feed additive comprising the phytase, as defined herein, for use in a feed for swine.

The invention is further directed to an animal feed additive comprising the phytase, as defined herein, for use in a feed for fish or crustaceans. The fish is typically be selected from the group consisting of salmon, trout, tilapia, catfish, seabream such as gilthead seabream, bass, such as seabass, and carp. The crustaceans may typically be selected from the group consisting of lobster, crab, crayfish, krill, shrimp and prawn.

Apart from the polypeptide of the invention, the animal feed additives of the invention contain at least one fat-soluble vitamin, and/or at least one water soluble vitamin, and/or at least one trace mineral. The feed additive may also contain at least one macro mineral.

Further, optional, feed-additive ingredients are colouring agents, e.g. carotenoids such as beta-carotene, astaxanthin, and lutein; aroma compounds; stabilisers; antimicrobial peptides; polyunsaturated fatty acids; reactive oxygen generating species; and/or at least one other polypeptide selected from amongst phytase (EC 3.1.3.8 or 3.1.3.26); phosphatase (EC 3.1.3.1; EC 3.1.3.2; EC 3.1.3.39); xylanase (EC 3.2.1.8); galactanase (EC 3.2.1.89); alpha-galactosidase (EC 3.2.1.22); protease (EC 3.4.-.- ), phospholipase A1 (EC 3.1.1.32); phospholipase A2 (EC 3.1.1.4); lysophospholipase (EC 3.1.1.5); phospholipase C (3.1.4.3); phospholipase D (EC 3.1.4.4); amylase such as, for example, alpha-amylase (EC 3.2.1.1); and/or beta-glucanase (EC 3.2.1.4 or EC 3.2.1.6).

As demonstrated in Example 14 in feeding broilers, a combination of a protease and the phytase of the invention increased protein digestibility by 0.7 to 1.6%, protein retention by 0.9 to 4.4% and energy retention by 0.6 to 1.3%. This resulted in an improvement in body weight gain (BWG) by 2.3 or 3.7% and feed conversion ratio by 4.6 or 2.8%. Accordingly, an embodiment of the invention is directed to an animal feed or animal feed additive comprising the phytase of the invention and a protease. The protease may be selected from the proteases comprised in Ronozyme ProAct^{™}, Ronozyme ProAct360^{™}, Axtra^{®} PRO, Avizyme 1502, Cibenza, Enolzyme, Poultrygrow-250 or Aquagrow-175, preferably selected from Ronozyme ProAct^{™}, Ronozyme ProAct360^{™}, Axtra^{®} PRO and Cibenza, more preferably selected from Ronozyme ProAct^{™}, Ronozyme ProAct360^{™}, most preferably Ronozyme ProAct360^{™}. The invention is further directed to an animal feed or animal feed additive comprising the phytase of the invention and a protease selected from the group consisting of a protease having at least 70% sequence identity, to SEQ ID NO:1 or SEQ ID NO: 2 of WO0158276 and of a protease having at least 75% sequence identity to SEQ ID NO:3 of WO2019043191. In one embodiment, the protease is selected from a protease having at least 75% sequence identity, such as at least 80% sequence identity, such as at least 85% sequence identity, such as at least 90% sequence identity, such as at least 95% sequence identity, such as at least 98% sequence identity, such as at least 99% sequence identity, such as 100% sequence identity to a protease comprising the sequence of SEQ ID NO: 22 of the present invention.

Typically, the animal feed comprising the protease and the phytase comprises 100 to 5,000 FYT/kg of the phytase in the feed, such as 500 to 2000 FYT/kg. Typically the the animal feed comprising the protease and the phytase comprises 10,000 to 50,000 U/kg of the protease in the feed, such as 15,000 to 35,000 U/kg typically 20,000 to 40,000 U/kg.

In a particular embodiment, these other polypeptides are well-defined (as defined above for phytase preparations).

The phytase of the invention may also be combined with other phytases, for example ascomycete phytases such as *Aspergillus* phytases, for example derived from *Aspergillus ficuum, Aspergillus niger,* or *Aspergillus awamori;* or basidiomycete phytases, for example derived from *Peniophora lycii, Agrocybe pediades, Trametes pubescens,* or *Paxillus involutus;* or derivatives, fragments or variants thereof which have phytase activity.

Thus, in preferred embodiments of the use in animal feed of the invention, and in preferred embodiments of the animal feed additive and the animal feed of the invention, the phytase of the invention is combined with such phytases.

Examples of antimicrobial peptides (AMP's) are CAP18, Leucocin A, Tritrpticin, Protegrin-1, Thanatin, Defensin, Lactoferrin, Lactoferricin, and Ovispirin such as Novispirin (Robert Lehrer, 2000), Plectasins, and Statins, including the compounds and polypeptides disclosed in WO 03/044049 and WO 03/048148, as well as variants or fragments of the above that retain antimicrobial activity.

Examples of antifungal polypeptides (AFP's) are the *Aspergillus giganteus,* and *Aspergillus niger* peptides, as well as variants and fragments thereof which retain antifungal activity, as disclosed in WO 94/01459 and WO 02/090384.

Examples of polyunsaturated fatty acids are C18, C20 and C22 polyunsaturated fatty acids, such as arachidonic acid, docosohexaenoic acid, eicosapentaenoic acid and gammalinoleic acid.

Examples of reactive oxygen generating species are chemicals such as perborate, persulphate, or percarbonate; and polypeptides such as an oxidase, an oxygenase or a syntethase.

Usually fat- and water-soluble vitamins, as well as trace minerals form part of a so-called premix intended for addition to the feed, whereas macro minerals are usually separately added to the feed. Either of these composition types, when enriched with a polypeptide of the invention, is an animal feed additive of the invention.

In a particular embodiment, the animal feed additive of the invention is intended for being included (or prescribed as having to be included) in animal diets or feed at levels of 0.01 to 10.0%; more particularly 0.05 to 5.0%; or 0.2 to 1.0% (% meaning g additive per 100 g feed). This is so in particular for premixes.

The following are non-exclusive lists of examples of these components:
Examples of fat-soluble vitamins are vitamin A, vitamin D3, vitamin E, and vitamin K, e.g. vitamin K3.

Examples of water-soluble vitamins are vitamin B12, biotin and choline, vitamin B1, vitamin B2, vitamin B6, niacin, folic acid and panthothenate, e.g. Ca-D-panthothenate.

Examples of trace minerals are manganese, zinc, iron, copper, iodine, selenium, and cobalt.

Examples of macro minerals are calcium, phosphorus and sodium.

The nutritional requirements of these components (exemplified with poultry and piglets/pigs) are listed in Table A of WO 01/58275. Nutritional requirement means that these components should be provided in the diet in the concentrations indicated.

In the alternative, the animal feed additive of the invention comprises at least one of the individual components specified in Table A of WO 01/58275. At least one means either of, one or more of, one, or two, or three, or four and so forth up to all thirteen, or up to all fifteen individual components. More specifically, this at least one individual component is included in the additive of the invention in such an amount as to provide an in-feed-concentration within the range indicated in column four, or column five, or column six of Table A.

The present invention also relates to animal feed compositions. Animal feed compositions or diets have a relatively high content of protein. Poultry and pig diets can be characterised as indicated in Table B of WO 01/58275, columns 2-3. Fish diets can be characterised as indicated in column 4 of this Table B. Furthermore such fish diets usually have a crude fat content of 200-310 g/kg.

WO 01/58275 corresponds to US 09/779334

An animal feed composition according to the invention has a crude protein content of 50-800 g/kg, and furthermore comprises at least one polypeptide of the present invention.

Furthermore, or in the alternative (to the crude protein content indicated above), the animal feed composition of the invention has a content of metabolisable energy of 10-30 MJ/kg; and/or a content of calcium of 0.1-200 g/kg; and/or a content of available phosphorus of 0.1-200 g/kg; and/or a content of methionine of 0.1-100 g/kg; and/or a content of methionine plus cysteine of 0.1-150 g/kg; and/or a content of lysine of 0.5-50 g/kg.

In particular embodiments, the content of metabolisable energy, crude protein, calcium, phosphorus, methionine, methionine plus cysteine, and/or lysine is within any one of ranges 2, 3, 4 or 5 in Table B of WO 01/58275 (R. 2-5).

Crude protein is calculated as nitrogen (N) multiplied by a factor 6.25, i.e. Crude protein (g/kg) = N (g/kg) x 6.25. The nitrogen content is determined by the Kjeldahl method (A.O.A.C., 1984, Official Methods of Analysis 14th ed., Association of Official Analytical Chemists, Washington DC).

Metabolisable energy can be calculated on the basis of the NRC publication Nutrient requirements in swine, ninth revised edition 1988, subcommittee on swine nutrition, committee on animal nutrition, board of agriculture, national research council. National Academy Press, Washington, D.C., pp. 2-6, and the European Table of Energy Values for Poultry Feed-stuffs, Spelderholt centre for poultry research and extension, 7361 DA Beekbergen, The Netherlands. Grafisch bedrijf Ponsen & looijen bv, Wageningen. ISBN 90-71463-12-5.

The dietary content of calcium, available phosphorus and amino acids in complete animal diets is calculated on the basis of feed tables such as Veevoedertabel 1997, gegevens over chemische samenstelling, verteerbaarheid en voederwaarde van voedermiddelen, Central Veevoederbureau, Runderweg 6, 8219 pk Lelystad. ISBN 90-72839-13-7.

In a particular embodiment, the animal feed composition of the invention contains at least one protein. The protein may be an animal protein, such as meat and bone meal, and/or fish meal; or it may be a vegetable protein. The term vegetable proteins as used herein refers to any compound, composition, preparation or mixture that includes at least one protein derived from or originating from a vegetable, including modified proteins and protein-derivatives. In particular embodiments, the protein content of the vegetable proteins is at least 10, 20, 30, 40, 50, or 60% (w/w).

Vegetable proteins may be derived from vegetable protein sources, such as legumes and cereals, for example materials from plants of the families Fabaceae (Leguminosae), Cruciferaceae, Chenopodiaceae, and Poaceae, such as soybean meal, lupin meal and rapeseed meal.

In a particular embodiment, the vegetable protein source is material from one or more plants of the family Fabaceae, e.g. soybean, lupine, pea, or bean.

In another particular embodiment, the vegetable protein source is material from one or more plants of the family Chenopodiaceae, e.g. beet, sugar beet, spinach or quinoa.

Other examples of vegetable protein sources are rapeseed, sunflower seed, cotton seed, and cabbage.

Soybean is a preferred vegetable protein source.

Other examples of vegetable protein sources are cereals such as barley, wheat, rye, oat, maize (corn), rice, triticale, and sorghum.

In still further particular embodiments, the animal feed composition of the invention contains 0-80% maize; and/or 0-80% sorghum; and/or 0-70% wheat; and/or 0-70% Barley; and/or 0-30% oats; and/or 0-40% soybean meal; and/or 0-25% fish meal; and/or 0-25% meat and bone meal; and/or 0-20% whey.

Animal diets can e.g. be manufactured as mash feed (non pelleted) or pelleted feed. Typically, the milled feed-stuffs are mixed and sufficient amounts of essential vitamins and minerals are added according to the specifications for the species in question. Polypeptides can be added as solid or liquid polypeptide formulations. For example, a solid polypeptide formulation is typically added before or during the mixing step; and a liquid polypeptide preparation is typically added after the pelleting step. The polypeptide may also be incorporated in a feed additive or premix.

The final polypeptide concentration in the diet is within the range of 0.01-200 mg polypeptide protein per kg diet, for example in the range of 5-30 mg polypeptide protein per kg animal diet.

The phytase of the invention should of course be applied in an effective amount, i.e. in an amount adequate for improving solubilisation and/or improving nutritional value of feed. It is at present contemplated that the polypeptide is administered in one or more of the following amounts (dosage ranges): 0.01-200; 0.01-100; 0.5-100; 1-50; 5-100; 10-100; 0.05-50; or 0.10-10 - all these ranges being in mg phytase polypeptide protein per kg feed (ppm).

For determining mg phytase polypeptide protein per kg feed, the phytase is purified from the feed composition, and the specific activity of the purified phytase is determined using a relevant assay. The phytase activity of the feed composition as such is also determined using the same assay, and on the basis of these two determinations, the dosage in mg phytase protein per kg feed is calculated.

The same principles apply for determining mg phytase polypeptide protein in feed additives. Of course, if a sample is available of the phytase used for preparing the feed additive or the feed, the specific activity is determined from this sample (no need to purify the phytase from the feed composition or the additive).

### Methods for Producing Fermentation Products

Yet another aspect of the present invention relates to the methods for producing a fermentation product, such as, e.g., ethanol, beer, wine, distillers dried grains (DDG), wherein the fermentation is carried out in the presence of a phytase produced by the present invention. Examples of fermentation processes include, for example, the processes described in WO 01/62947. Fermentation is carried out using a fermenting microorganism, such as, yeast.

In a particular embodiment, the present invention provides methods for producing fermentation product, comprising (a) fermenting (using a fermenting microorganism, such as yeast) a carbohydrate containing material (e.g., starch) in the presence of a phytase of the present invention and (b) producing the fermentation product from the fermented carbohydrate containing material.

In a particular embodiment, the present invention provides methods for producing ethanol, comprising fermenting (using a fermenting microorganism, such as yeast) a carbohydrate containing material (e.g., starch) in the presence of a phytase of the present invention and producing or recovering ethanol from the fermented carbohydrate containing material.

In another embodiment, the present invention provides methods for producing ethanol comprising a) hydrolyzing starch, e.g., by a liquefaction and/or saccharification process, a raw starch hydrolysis process, b) fermenting the resulting starch in the presence of a phytase of the present invention, and c) producing ethanol.

The phytase may be added to the fermentation process at any suitable stage and in any suitable composition, including alone or in combination with other enzymes, such as, one or more alpha-amylases, glucoamylases, proteases, and/or cellulases.

In another embodiment, the present invention provides methods for producing ethanol comprising hydrolyzing biomass, and fermenting (using a fermenting microorganism, such as yeast) the resulting biomass in the presence of a phytase of the present invention.

### Plants

The present invention also relates to plants, e.g., a transgenic plant, plant part, or plant cell, comprising a polynucleotide of the present invention so as to express and produce the phytase in recoverable quantities. The phytase may be recovered from the plant or plant part. Alternatively, the plant or plant part containing the phytase may be used as such for improving the quality of a food or feed, e.g., improving nutritional value, palatability, and rheological properties, or to destroy an antinutritive factor.

The transgenic plant can be dicotyledonous (a dicot) or monocotyledonous (a monocot). Examples of monocot plants are grasses, such as meadow grass (blue grass, Poa), forage grass such as Festuca, Lolium, temperate grass, such as Agrostis, and cereals, e.g., wheat, oats, rye, barley, rice, sorghum, and maize (corn).

Examples of dicot plants are tobacco, legumes, such as lupins, potato, sugar beet, pea, bean and soybean, and cruciferous plants (family Brassicaceae), such as cauliflower, rape seed, and the closely related model organism *Arabidopsis thaliana.*

Examples of plant parts are stem, callus, leaves, root, fruits, seeds, and tubers as well as the individual tissues comprising these parts, e.g., epidermis, mesophyll, parenchyme, vascular tissues, meristems. Specific plant cell compartments, such as chloroplasts, apoplasts, mitochondria, vacuoles, peroxisomes and cytoplasm are also considered to be a plant part. Furthermore, any plant cell, whatever the tissue origin, is considered to be a plant part. Likewise, plant parts such as specific tissues and cells isolated to facilitate the utilization of the invention are also considered plant parts, e.g., embryos, endosperms, aleurone and seed coats.

Also included within the scope of the present invention are the progeny of such plants, plant parts, and plant cells.

The transgenic plant or plant cell expressing a phytase may be constructed in accordance with methods known in the art. In short, the plant or plant cell is constructed by incorporating one or more expression constructs encoding a phytase into the plant host genome or chloroplast genome and propagating the resulting modified plant or plant cell into a transgenic plant or plant cell.

The expression construct is conveniently a nucleic acid construct that comprises a polynucleotide encoding a phytase operably linked with appropriate regulatory sequences required for expression of the polynucleotide in the plant or plant part of choice. Furthermore, the expression construct may comprise a selectable marker useful for identifying plant cells into which the expression construct has been integrated and DNA sequences necessary for introduction of the construct into the plant in question (the latter depends on the DNA introduction method to be used).

The choice of regulatory sequences, such as promoter and terminator sequences and optionally signal or transit sequences, is determined, for example, on the basis of when, where, and how the phytase is desired to be expressed. For instance, the expression of the gene encoding a phytase may be constitutive or inducible, or may be developmental, stage or tissue specific, and the gene product may be targeted to a specific tissue or plant part such as seeds or leaves. Regulatory sequences are, for example, described by Tague et al., 1988, Plant Physiology 86: 506.

For constitutive expression, the 35S-CaMV, the maize ubiquitin 1, or the rice actin 1 promoter may be used (Franck et al., 1980, Cell 21: 285-294; Christensen et al., 1992, Plant Mol. Biol. 18: 675-689; Zhang et al., 1991, Plant Cell 3: 1155-1165). Organ-specific promoters may be, for example, a promoter from storage sink tissues such as seeds, potato tubers, and fruits (Edwards and Coruzzi, 1990, Ann. Rev. Genet. 24: 275-303), or from metabolic sink tissues such as meristems (Ito et al., 1994, Plant Mol. Biol. 24: 863-878), a seed specific promoter such as the glutelin, prolamin, globulin, or albumin promoter from rice (Wu et al., 1998, Plant Cell Physiol. 39: 885-889), a *Vicia faba* promoter from the legumin B4 and the unknown seed protein gene from *Vicia faba* (Conrad et al., 1998, J. Plant Physiol. 152: 708-711), a promoter from a seed oil body protein (Chen et al., 1998, Plant Cell Physiol. 39: 935-941), the storage protein *napA* promoter from *Brassica napus,* or any other seed specific promoter known in the art, *e.g.,* as described in WO 91/14772. Furthermore, the promoter may be a leaf specific promoter such as the *rbcs* promoter from rice or tomato (Kyozuka et al., 1993, Plant Physiol. 102: 991-1000), the chlorella virus adenine methyltransferase gene promoter (Mitra and Higgins, 1994, Plant Mol. Biol. 26: 85-93), the *aldP* gene promoter from rice (Kagaya et al., 1995, Mol. Gen. Genet. 248: 668-674), or a wound inducible promoter such as the potato *pin2* promoter (Xu et al., 1993, Plant Mol. Biol. 22: 573-588). Likewise, the promoter may be induced by abiotic treatments such as temperature, drought, or alterations in salinity or induced by exogenously applied substances that activate the promoter, e.g., ethanol, oestrogens, plant hormones such as ethylene, abscisic acid, and gibberellic acid, and heavy metals.

A promoter enhancer element may also be used to achieve higher expression of a phytase in the plant. For instance, the promoter enhancer element may be an intron that is placed between the promoter and the polynucleotide encoding a phytase. For instance, Xu *et al.,* 1993, *supra,* disclose the use of the first intron of the rice actin 1 gene to enhance expression.

The selectable marker gene and any other parts of the expression construct may be chosen from those available in the art.

The nucleic acid construct is incorporated into the plant genome according to conventional techniques known in the art, including Agrobacterium-mediated transformation, virus-mediated transformation, microinjection, particle bombardment, biolistic transformation, and electroporation (Gasser et al., 1990, Science 244: 1293; Potrykus, 1990, Bio/Technology 8: 535; Shimamoto et al., 1989, Nature 338: 274).

*Agrobacterium tumefaciens-mediated* gene transfer is a method for generating transgenic dicots (for a review, see Hooykas and Schilperoort, 1992, Plant Mol. Biol. 19: 15-38) and for transforming monocots, although other transformation methods may be used for these plants. A method for generating transgenic monocots is particle bombardment (microscopic gold or tungsten particles coated with the transforming DNA) of embryonic calli or developing embryos (Christou, 1992, Plant J. 2: 275-281; Shimamoto, 1994, Curr. Opin. Biotechnol. 5: 158-162; Vasil et al., 1992, Bio/Technology 10: 667-674). An alternative method for transformation of monocots is based on protoplast transformation as described by Omirulleh et al., 1993, Plant Mol. Biol. 21: 415-428. Additional transformation methods include those described in U.S. Patent Nos. 6,395,966 and 7,151,204

Following transformation, the transformants having incorporated the expression construct are selected and regenerated into whole plants according to methods well known in the art. Often the transformation procedure is designed for the selective elimination of selection genes either during regeneration or in the following generations by using, for example, co-transformation with two separate T-DNA constructs or site specific excision of the selection gene by a specific recombinase.

In addition to direct transformation of a particular plant genotype with a construct of the present invention, transgenic plants may be made by crossing a plant having the construct to a second plant lacking the construct. For example, a construct encoding a phytase can be introduced into a particular plant variety by crossing, without the need for ever directly transforming a plant of that given variety. Therefore, the present invention encompasses not only a plant directly regenerated from cells which have been transformed in accordance with the present invention, but also the progeny of such plants. As used herein, progeny may refer to the offspring of any generation of a parent plant prepared in accordance with the present invention. Such progeny may include a DNA construct prepared in accordance with the present invention. Crossing results in the introduction of a transgene into a plant line by cross pollinating a starting line with a donor plant line. Non-limiting examples of such steps are described in U.S. Patent No. 7,151,204.

Plants may be generated through a process of backcross conversion. For example, plants include plants referred to as a backcross converted genotype, line, inbred, or hybrid.

Genetic markers may be used to assist in the introgression of one or more transgenes of the invention from one genetic background into another. Marker assisted selection offers advantages relative to conventional breeding in that it can be used to avoid errors caused by phenotypic variations. Further, genetic markers may provide data regarding the relative degree of elite germplasm in the individual progeny of a particular cross. For example, when a plant with a desired trait which otherwise has a non-agronomically desirable genetic background is crossed to an elite parent, genetic markers may be used to select progeny which not only possess the trait of interest, but also have a relatively large proportion of the desired germplasm. In this way, the number of generations required to introgress one or more traits into a particular genetic background is minimized.

The present invention also relates to methods of producing a phytase of the present invention comprising: (a) cultivating a transgenic plant or a plant cell comprising a polynucleotide encoding the phytase under conditions conducive for production of the phytase; and (b) recovering the phytase.

The present invention is further described by the following examples that should not be construed as limiting the scope of the invention.

### EXAMPLES

Chemicals used were commercial products of at least reagent grade.

### Example 1: Preparation of variants, and determination of activity

### Expression of phytase variants in Aspergillus oryzae

The constructs comprising the C. braakii phytase variant genes in the examples were used to construct expression vectors for *Aspergillus.* The Aspergillus expression vectors consist of an expression cassette based on the *Aspergillus niger* neutral amylase II promoter fused to the *Aspergillus nidulans* triose phosphate isomerase non translated leader sequence (Pna2/tpi) and the *Aspergillus niger* amyloglycosidase terminator (Tamg). Also present on the plasmid was the *Aspergillus* selective marker *pyrG* from *Aspergillus nidulans* enabling growth on miminal media for an aspergillus which is pyrG minus. The expression plasmids for phytase variants were transformed into *Aspergillus* as described in Lassen et al. (2001), Applied and Environmental Micorbiology, 67, 4701-4707. For each of the constructs 4-6 strains were isolated, purified and cultivated in microtiterplates. Expression was determined using a p-nitrophenyl phosphate substrate. The best producing strain was fermented in Shake flasks.

### Purification of C. braakii phytase variants

The fermentation supernatant with the phytase variant was filtered through a Fast PES Bottle top filter with a 0.22 µm cut-off. The resulting solution was diluted with water to the double volume and pH was adjusted to 4.5 with acetic acid. Occasionally, the solution became a little cloudy and this removed by filtration through a Fast PES Bottle top filter with a 0.22 µm cut-off.

After pretreatment the phytase variant was purified by chromatography on S Sepharose, approximately 30 ml in a XK26 column, using as buffer A 50 mM sodium acetate pH 4.5, and as buffer B 50 mM sodium acetate + 1 M NaCl pH 4.5. The fractions from the column were analyzed for activity using the phosphatase assay (see below) and fractions with activity were pooled.

In some cases the solution containing the purified phytase variant was concentrated using an Amicon ultra-15 filtering device with a 30 kDa cut-off membrane.

The molecular weight, as estimated from SDS-PAGE, was approximately 45-50 kDa and the purity was > 95%.

### Determination of phosphatase activity

75 microliter phytase-containing enzyme solution is dispensed in a microtiter plate well, e. g. NUNC 269620 and 75 microliter substrate is added (for preparing the substrate, two 5 mg p-nitrophenyl phosphate tablets (Sigma, Cat.No. N-9389) are dissolved in 10 ml 0.1 M Na-acetate buffer, pH 5.5). The plate is sealed and incubated 15 min., shaken with 750 rpm at 37°C. After the incubation time 75 microliter stop reagent is added (the stop reagent is 0.1 M disodiumtetraborate in water) and the absorbance at 405 nm is measured in a microtiter plate spectrophotometer. One phosphatase unit is defined as the enzyme activity that releases 1 micromol phosphate/min under the given reaction conditions (buffer blind subtracted). The absorbance of 1 micromol p-nitrophenol is determined to be 56 AU (AU= absorbancy units) under assay conditions.

### Determination of phytase activity

75 microliter phytase-containing enzyme solution, appropriately diluted in 0.25M sodium acetate, 0.005% (w/v) Tween-20. pH5.5, is dispensed in a microtiter plate well, e. g. NUNC 269620, and 75 microliter substrate is added (prepared by dissolving 100mg sodium phytate from rice (Aldrich Cat.No. 274321) in 10ml 0.25M sodium acetate buffer, pH5.5). The plate is sealed and incubated 15min. shaken with 750rpm at 37°C. After incubation, 75 microliter stop reagent is added (the stop reagent being prepared by mixing 10 ml molybdate solution (10% (w/v) ammonium hepta-molybdate in 0.25% (w/v) ammonia solution), 10ml ammonium vanadate (0.24% commercial product from Bie&Berntsen, Cat.No. LAB17650), and 20ml 21.7% (w/v) nitric acid), and the absorbance at 405nm is measured in a microtiter plate spectrophotometer. The phytase activity is expressed in the unit of FYT, one FYT being the amount of enzyme that liberates 1 micromole inorganic ortho-phosphate per minute under the conditions above. An absolute value for the measured phytase activity may be obtained by reference to a standard curve prepared from appropriate dilutions of inorganic phosphate, or by reference to a standard curve made from dilutions of a phytase enzyme preparation with known activity (such standard enzyme preparation with a known activity is available on request from Novozymes A/S, Krogshoejvej 36, DK-2880 Bagsvaerd).

### Example 2: Single position variants

The parent phytase for this example was a variant having the sequence of SEQ ID NO: 2 with the substitutions: (var300) N31C/G52C/A99C/K141C/T177C/V199C/N203L.

96 single position variants of the parent phytase was prepared as described in Example 1. The substitutions were selected based on alignment of known phytases and identification of consensus sequences as known in the art. The thermostability of the variants were tested using Protein Thermal Shift^{™} (Applied Biosystems, Carlsbad, CA, US) according to the manufacturer's instructions. Variants having improved thermostability compared with the parent are shown in Table 1.

**Table 1.**

| **Variant** | **T (°C)** |
|---|---|
| K30Q | 70 |
| Q60H | 70 |
| L64Q | 70 |
| E119S | 70 |
| N121P | 71 |
| L138A | 70 |
| S162A | 70 |
| T180N | 70 |
| P190T | 70 |
| R243N | 70 |
| M273L | 71 |
| K336R | 70 |
| T340L | 70 |
| D358Q | 72 |
| Q36A | 70 |
| E57Y | 71 |
| N73P | 71 |
| S79Q | 70 |
| M130T | 70 |
| S134Q | 71 |
| Y155F | 71 |
| S161T | 70 |
| N168R | 70 |
| E176P | 70 |
| S184Q | 70 |
| P207T | 72 |
| E224Q | 70 |
| Q230E | 70 |
| T340P | 70 |
| D375K | 70 |

### Example 3: Combining beneficial positions

Based on the results disclosed in Example 2, a variant combining several of the beneficial substitutions was generated having the sequence of SEQ ID NO: 2 with the substitutions:
(var400):

N31C/G52C/E57Y/N73P/A99C/N121P/S134Q/K141C/Y155F/T177C/V199C/N203L/
P207T/M273L

A gene was designed and the variant produced as described in Example 1. Thermal shift assay was performed in triplicate on the variant showing 81°C.

### Example 4: Further Combinatorial variants

Based on the results disclosed in Example 2 a variant combining further beneficial substitutions using the variant generated in Example 3 as the parent phytase. Following variants having the sequence of SEQ ID NO: 2 with the substitutions were generated:
(var404) N31C/Q36A/G52C/E57Y/Q60H/N73P/A99C/E119S/N121P/M130T/S134Q/L138A/K141C/ 
Y155F/S161T/S162A/E176P/T177C/T180N/S184Q/P190T/V199C/N203L/P207T/E224Q/Q230E
/R243N/M273L/K336R/T340L
(var405) 
N31C/Q36A/P43C/W46C/G52C/E57Y/Q60H/L64Q/N73P/A99C/E119S/N121P/M130T/S134Q/
L138A/K141C/Y155F/S161T/S162A/E176P/T177C/T180N/S184Q/P190T/V199C/N203L/P207T/
E224Q/Q230E/ R243N/M273L/N286S/K336R/T340L
(var406) 
N31C/Q36A/G52C/E57Y/Q60H/L64Q/N73P/A99C/E119S/N121P/P123C/M130C/S134Q/L138A/
K141C/N151S/Y155F/S161T/S162A/E176P/T177C/T180N/S184Q/P190T/V199C/N203L/
P207T/E224Q/Q230E/R243N/M273L/K336R/T340L
(var411)

N31C/Q36A/P43C/W46C/G52C/E57Y/Q60H/L64Q/N73P/A99C/E119S/N121P/P123C/M130C/
S134Q/L138A/K141C/Y155F/S161T/S162A/E176P/T177C/T180N/S184Q/P190T/V199C/N203L
/P207T/E224Q/Q230E/R243N/M273L/N286S/K336R/T340L

### Example 5: Thermostability of generated variants

The following variants were constructed and tested for thermostability:

**Table 2.**

| Phytase | Substitutions |
|---|---|
| var400 | N31C/G52C/E57Y/N73P/A99C/N121P/S134Q/K141C/Y155F/T177C/V199C/ N203L/P207T/M273L |
| var404 | N31C/Q36A/G52C/E57Y/Q60H/N73P/A99C/E119S/N121P/M130T/S134Q/L138A/ K141C/Y155F/S161T/S162A/E176P/T177C/T180N/S184Q/P190T/V199C/ N203L/P207T/E224Q/Q230E/R243N/M273L/K336R/T340L |
| var405 | N31C/Q36A/P43C/W46C/G52C/E57Y/Q60H/L64Q/N73P/A99C/E119S/N121P/ M130T/S134Q/L138A/K141C/Y155F/S161T/S162A/E176P/T177C/T180N/S184Q/ P190T/V199C/N203L/P207T/E224Q/Q230E/R243N/M273L/N286S/K336R/T340L |
| var406 | N31C/Q36A/G52C/E57Y/Q60H/L64Q/N73P/A99C/E119S/N121P/P123C/M130C/ S134Q/L138A/K141C/N151S/Y155F/S161T/S162A/E176P/T177C/T180N/S184Q/ P190T/V199C/N203L/P207T/E224Q/Q230E/R243N/M273L/K336R/T340L |
| var411 | N31C/Q36A/P43C/W46C/G52C/E57Y/Q60H/L64Q/N73P/A99C/E119S/N121P/P1 23C/M130C/S134Q/L138A/K141C/Y155F/S161T/S162A/E176P/T177C/T180N/S1 84Q/P190T/V199C/N203L/P207T/E224Q/Q230E/R243N/M273L/N286S/K336R/T 340L |

The variants were tested for thermostability by nano Differential Scanning Fluorescence (nanoDSF).

Nano-DSF monitors intrinsic tryptophan (Trp) fluorescence of the protein as a function of temperature at 330 and 350 nm. The temperature stability of a protein can be expressed by Tm (the temperature at which there is an equal population of folded and unfolded molecules) found at the inflection point of the fluorescence signal.

NanoDSF was performed with a nanoDSF Prometheus NT.48 instrument (NanoTemper Technologies GmbH, München, Germany). Phytase variant samples (purified as described in Example 1, all of them in 50 mM Na-acetate, pH 4.5) were loaded into nanoDSF standard grade capillaries (NanoTemper Technologies GmbH; catalog number PR-C002) through capillary action. Three capillaries were filled for each sample. The capillaries were then placed into the instrument (up to 48 single capillaries can be loaded in a single run) and the laser intensity required for optimum signal generation was determined. The samples were run with the following experimental setting: temperature slope 2 °C/minute, start temperature 20 °C and end temperature 95 °C.

The data was analyzed using the software that is supplied with the instrument (PR. ThermControl v2.0.4, NanoTemper Technologies GmbH) and the Tm (for the ratio 350 nm/330 nm) was determined (results shown in Table 3 below).

Further the variants were tested using DSC using the assay disclosed in WO 2011/117396 Example 4. Results are shown in Table 3.

**Table 3.**

| Designation | nDSF (broth) (°C) | nDSF (purified) (°C) | DSC (°C) |
|---|---|---|---|
| var400 | 83.7 | 82.5 | 85.6 |
| var404 | 86.1 | 85.4 | 88.3 |
| var405 | 86.1 | 86.2 | 88.9 |
| var406 | 88.7 | 88.1 | 90.6 |
| var411 | 88.8 | 88.1 | |

The variants were also tested for thermostability in the pH range 1.0 to 8.5 using NanoDSF (described above). The variant samples were in 0.1M glycine, 0.1M acetic acid, 0.1M Bis-Tris, adjusted to the desired pH with either 0.5 M HCl or 0.1 M NaOH. The temperature slope was 3.33 °C/minute in this experiment. Results are shown in Tables 4a and 4b.

**Table 4a**

| | **pH** | | | | | | | |
|---|---|---|---|---|---|---|---|---|
| **Phytase** | **1.0** | **1.5** | **2.0** | **2.5** | **3.0** | **3.5** | **4.0** | **4.5** |
| C.b. wt SEQ ID2 | ns | 44.2 | 46.0 | 49.3 | 53.6 | 58.6 | 61.5 | 63.4 |
| var300 | 44.3 | 47.9 | 52.7 | 58.8 | 65.2 | 70.3 | 73.8 | 75.2 |
| var400 | 52.7 | 56.2 | 60.2 | 65.9 | 71.5 | 76.8 | 80.1 | 81.9 |
| var404 | 57.1 | 61.0 | 64.4 | 69.7 | 75.6 | 80.5 | 83.3 | 85.5 |
| var405 | 58.8 | 61.8 | 65.6 | 70.6 | 76.3 | 81.1 | 84.7 | 86.4 |
| var406 | 62.4 | 64.6 | 67.9 | 72.6 | 77.8 | 83.5 | 85.9 | 88.2 |
| var411 | 62.9 | 65.0 | 68.0 | 72.8 | 78.3 | 83.5 | 86.0 | 88.2 |

**Table 4b**

| | **pH** | | | | | | | |
|---|---|---|---|---|---|---|---|---|
| **Phytase** | **5.0** | **5.5** | **6.0** | **6.5** | **7.0** | **7.5** | **8.0** | **8.5** |
| C.b. wt SEQ ID 2 | 63.9 | 63.7 | 63.8 | 62.7 | 61.4 | 60.0 | 58.7 | 57.6 |
| var300 | 76.5 | 76.2 | 76.2 | 75.9 | 74.8 | 73.8 | 73.3 | 72.3 |
| var400 | 83.3 | 83.6 | 84.1 | 83.5 | 83.4 | 82.0 | 81.8 | 81.1 |
| var404 | 86.9 | 87.1 | 87.5 | 87.6 | 87.2 | 86.7 | 86.1 | 85.6 |
| var405 | 87.3 | 88.0 | 88.4 | 89.2 | 89.2 | 88.6 | 88.5 | 88.0 |
| var406 | 89.6 | 90.3 | 90.5 | 90.5 | 91.6 | 91.4 | 90.7 | 89.7 |
| var411 | 89.2 | 89.5 | 89.9 | 90.0 | 90.1 | 89.5 | 88.9 | 88.4 |

Conclusion: The variants have increased unfolding temperatures at all pH-values tested. The variants have improved thermostability compared to the wild type at all pHs.

### Example 6: pH-stability

The pH stability of the purified phytases of C.b. Wt (SEQ ID NO: 2) and var400 (SEQ ID NO: 12) at 37°C was determined by measuring residual phytase activity after incubation at 37°C and at various pH values for 1.0 and 24 hours. The phytases were incubated in 0.1M glycine, 0.1M acetic acid, 0.1M Bis-Tris, adjusted to the desired pH. Samples of the respective incubation mixtures were withdrawn after 0, 1.0 and 24 hours, the pH of the samples was adjusted to 5.5 by dilution in 0.25M sodium acetate, 0.005% (w/v) Tween20, pH5.5), and the residual activity at pH5.5 was determined using the method described in Example 1. The results, normalized to the activity found at 0 hours, are shown for 24 hours in Table 5 below.

**Table 5. pH stability at 37°C**

| **pH** | **C.b. Wt (SEQ ID NO: 2)** | **var400 (SEQ ID NO: 12)** |
|---|---|---|
| 24 hours | | |
| 1.0 | 1 | 64 |
| 2.0 | 12 | 96 |
| 3.0 | 61 | 101 |
| 4.0 | 99 | 102 |
| 5.0 | 97 | 105 |
| 6.0 | 101 | 94 |
| 7.0 | 105 | 97 |
| 8.0 | 90 | 103 |

Var400 has higher residual activity after incubation at very low pH (pH 1.0-3.0) after 24 hours incubation.

### Example 7: pH-stability in the presence of pepsin

The pH stability of the purified phytases of C.b. Wt (SEQ ID NO: 2) and variants (see tables below) at 37°C and in the presence of pepsin was determined by measuring residual phytase activity after incubation at 37°C and at various pH values for 30 and 60 minutes, respectively. The phytases were incubated in 0.1M glycine, 0.1M acetic acid, 0.1M Bis-Tris, adjusted to the desired pH and added 500 U/ml of pepsin (Sigma P7000). Samples of the respective incubation mixtures were withdrawn after 0, 30 minutes and 60 minutes, the pH of the samples was adjusted to 5.5 by dilution in 0.25M sodium acetate, 0.005% (w/v) Tween20, pH5.5), and the residual activity at pH5.5 was determined using the method described in Example 1. The results, normalized to the activity found at 0 hours, are shown in Tables 6 and 7 below.

**Table 6. Residual activity after 30 minutes incubation.**

| | **pH** | | | | | | | |
|---|---|---|---|---|---|---|---|---|
| **Phytase** | **1.0** | **1.5** | **2.0** | **2.5** | **3.0** | **3.5** | **4.0** | **4.5** |
| var300 | 3 | 3 | 24 | 62 | 78 | 86 | 88 | 93 |
| var400 | 28 | 48 | 81 | 93 | 89 | 93 | 96 | 97 |
| var404 | 77 | 87 | 95 | 97 | 98 | 93 | 100 | 95 |
| var405 | 84 | 91 | 94 | 96 | 96 | 100 | 98 | 102 |
| var406 | 89 | 90 | 95 | 100 | 101 | 97 | 96 | 96 |
| var411 | 94 | 93 | 91 | 92 | 91 | 97 | 96 | 93 |

**Table 7. Residual activity after 60 minutes incubation.**

| | **pH** | | | | | | | |
|---|---|---|---|---|---|---|---|---|
| **Phytase** | **1.0** | **1.5** | **2.0** | **2.5** | **3.0** | **3.5** | **4.0** | **4.5** |
| var300 | 5 | 4 | 13 | 59 | 77 | 88 | 92 | 97 |
| var400 | 14 | 36 | 73 | 88 | 81 | 86 | 92 | 99 |
| var404 | 67 | 81 | 90 | 90 | 93 | 87 | 98 | 95 |
| var405 | 78 | 83 | 88 | 90 | 93 | 99 | 95 | 101 |
| var406 | 88 | 86 | 90 | 98 | 92 | 95 | 94 | 94 |
| var411 | 92 | 90 | 86 | 88 | 89 | 92 | 94 | 93 |

The variants have increased pH-stability in the presence of pepsin at pH-values below 3.0 compared to the var300 reference.

### Example 8: Efficacy Study of a phytase in Broiler Chickens and Weaned Piglets

In the broiler study, birds (Cobb 500, male, commercially available from Yukou Poultry Husbandary Co., Ltd., Beijing, China) were housed in wire-floored battery cages in an environmentally controlled room. At the start of trial (day 8 of age), birds were sorted by weight and divided into replicate groups, each comprising 8 birds. The birds with similar cage weight were randomly allocated to one of the different treatments, and each treatment was replicated with 12 cages. There were 8 dietary treatments consisting of a negative control and the negative control supplemented with 7 levels of the phytase variant (var400): 187.5, 375, 750, 1125, 1500, 1875 or 2250 FYT/kg. A basal diet was prepared with corn and soybean meal as the main ingredients, and was formulated to be deficient only in total P (0.46%). The phytase variant was pre-mixed with a small amount of the basal diet before the complete mixing of the experimental diets to ensure uniformity of mixing. The feed was pelleted at 75°C. The analyzed phytase variant activities of the dietary treatments were 182, 328, 640, 1060, 1347, 1560 and 1931 FYT/kg.

The experimental diets were supplied to birds from day 8 to 18 of age, feed and water were supplied *ad libitum* through the whole trial. At day 8 and 17 of age, feed consumption and body weight (BW) by cage were recorded to calculate the body weight gain (WG), feed intake (FI) and the feed conversion ratio (FCR). Excreta were collected on day 14 through day 17. During this period, the excreta from 12 cages of each treatment were quantitatively collected once per day, and the excreta per cage from the 4 days were pooled together, frozen immediately at -20°C after collection. After thawing, the total excreta of each cage were homogenized, and the representative sub-samples were taken and freeze-dried for the determination of dry matter (DM), P, Ca and phytate-P. The total amount of feed consumption during the excreta collection period was recorded as well. At day 18 of age, the right tibia was taken from 2 birds randomly chosen from each of the 12 replicate cages. Tibias were defleshed, and cartilaginous caps were removed after collection. They were kept frozen in plastic bags at -20°C to maintain wetness until analysis of ash, Ca and total P content.

In the piglet study, 140 castrated male piglets (Redon x Large White) were used. The piglets were weaned at 28 days of age and had an average body weight of 7.5 ± 1.1 kg (mean ± standard deviation) at the start of trial. The piglets were housed in 35 flat-deck cages with 4 animals per cage in an environmentally controlled room. Each cage had a plastic-coated welded wire floor and was equipped with two water nipples and two stainless-steel feeders. The experimental diets were fed for 42 days which were divided into a starter phase of 14 days and a grower phase of 28 days. Water and feed were supplied *ad libitum.* The feed was offered in mash form.

There were 7 dietary treatments consisting of a positive control (PC), a negative control (NC) and the NC supplemented with 187.5, 375, 750, 1500 or 3000 FYT test phytase/kg feed (on analysis: 266, 383, 771, 1445, and 2914 FYT/kg in starter diets; 219, 395, 884, 1408, and 2730 FYT/kg in grower diets). The PC diets for the starter and grower phases met the pig's requirement for energy and nutrients prescribed by NRC (2012) for the body weight range of 7 to 11 kg and 11 to 25 kg, respectively, and were formulated with corn, soybean meal and rapeseed meal as the main ingredients. The NC diets were established by withdrawing the dicalcium phosphate from the PC diets resulting in P deficient diets (0.43% and 0.41% total P for starter and grower, respectively) with adequate Ca. The ingredient and nutrition compositions of diets are shown in Table 8.

**Table 8. ingredient and nutrition composition of experimental diets in broiler and piglet trials, %**

| | | **Broiler trial** | **Piglet trial** | | | |
|---|---|---|---|---|---|---|
| | | **8-18 day of age** | **Starter** | | **Grower** | |
| | **Ingredient** | **NC** | **NC** | **PC** | **NC** | **PC** |
| | Corn | 60.14 | 54.35 | 53.60 | 59.45 | 58.75 |
| | Soybean meal | 33.50 | 24.00 | 24.00 | 20.00 | 20.00 |
| | Rapeseed meal | -- | 12.00 | 12.00 | 12.00 | 12.00 |
| | Soybean oil | 2.50 | 4.50 | 4.70 | 3.70 | 3.90 |
| | NaCl | 0.41 | 0.20 | 0.20 | 0.05 | 0.05 |
| | NaHCO₃ | 0.14 | 0.35 | 0.35 | 0.35 | 0.35 |
| | DL-Met | 0.58 | 0.10 | 0.10 | 0.05 | 0.05 |
| | L-Lys HCl | 0.37 | 0.25 | 0.25 | 0.25 | 0.25 |
| | L-Thr | 0.15 | 0.10 | 0.10 | 0.05 | 0.05 |
| | L-Val | -- | 0.15 | 0.15 | 0.10 | 0.10 |
| | Limestone | 1.06 | 1.00 | 0.00 | 0.70 | 0.00 |
| | Dicalcium phosphate | 0.55 | 0.00 | 1.55 | 0.00 | 1.20 |
| | Vit-Min Premix⁽¹⁾ | 0.50 | 3.00 | 3.00 | 3.00 | 3.00 |
| | TiO₂ | 0.10 | 0.00 | 0.00 | 0.30 | 0.30 |
| Total | | 100.00 | 100.00 | 100.00 | 100.00 | 100.00 |
| | CP, % | 21.5 | 20.0 | 19.9 | 18.6 | 18.5 |
| | ME, kcal/kg | 3066 | 3404 | 3396 | 3374 | 3367 |
| | Dig Lys, % | 1.25 | 1.35 | 1.35 | 1.26 | 1.26 |
| | Diq Met, % | 0.85 | 0.43 | 0.43 | 0.37 | 0.37 |
| | Diq Thr, % | 0.83 | 0.83 | 0.83 | 0.74 | 0.74 |
| | Digestible P, % | -- | 0.17 | 0.40 | 0.16 | 0.34 |
| | Total P, % | 0.46 | 0.43 | 0.72 | 0.41 | 0.64 |
| | Phytate P, % | 0.24 | 0.28 | 0.28 | 0.27 | 0.27 |
| | Calcium, % | 0.70 | 0.83 | 0.84 | 0.71 | 0.74 |

| | | | | | | |
|---|---|---|---|---|---|---|
| ⁽¹⁾ Broiler premix supplied per kg of diet: vitamin A, 10,000 IU; vitamin D₃, 2,240 IU; 25-OH-D₃, 69 µg; vitamin E, 50 IU; vitamin K₃, 3 mg; vitamin B₁, 2 mg; vitamin B₂, 7 mg; vitamin B₆, 4 mg; vitamin B₁₂, 20 µg; biotin, 250 µg; folic acid, 2 mg; niacin, 60 mg; D-pantothenic acid, 12 mg; Fe, 40 mg; Cu, 15 mg; Mn, 110 mg; Zn, 90 mg; I, 0.5 mg; Se, 0.25 mg; and choline, 400 mg. | | | | | | |

Piglet premix supplied per kg of diet: vitamin A, 15,000 IU; vitamin D₃, 1,998 IU; vitamin E, 100 IU; vitamin K₃, 20 mg; vitamin B₁, 3.0 mg; vitamin B₂, 10 mg; vitamin B₆, 6 mg; vitamin B₁₂, 40 µg; biotin, 200 µg; D-pantothenic acid, 25 mg; folic acid, 1.5 mg; niacin, 35 mg; vitamin C, 100 mg; Cu, 160 mg; I, 2.0 mg; Fe, 200 mg; Mn, 60 mg; Zn, 100 mg; Se, 400 µg; choline, 375 mg; sodium, 1.5 g; chlorine, 3.2 g; Ca, 2.8 g; lysine, 2.9 g; methionine, 0.5 g; threonine, 1.4 g; tryptophan, 0.3 g; and valine, 0.2 g.

The pigs were weighed individually and feed consumption was recorded for each pen to calculate average daily gain (ADG), average daily feed intake (ADFI) and FCR. At the end of the trial, 2 pigs of each pen with body weight closest to the average body weight of their pen were slaughtered for collection of femurs. The right femurs were separated and removed of the soft tissue. A diaphysis section (~3.5 cm in length) of each femur was obtained by sawing and then subjected to compression to determine the force in Newton to break the bone. The broken bones were used for the determination of ash, Ca and P content.

The samples of diets and excreta collected from broiler and pig trials were ground to pass through a 0.5-mm screen before analysis. All samples were analyzed in duplicate. The samples were dried at 105 °C in an oven for 4 hours for dry matter determination (method 934.01; AOAC International, 2006). Ca and P were determined by Inductively Coupled Plasma-Optical Emission Spectrometry (ICP-OES; 5100 Dual View, Agilent, Santa Clara, CA, USA; method 985.01; AOAC International, 2006) after sulfuric acid mineralization. Dietary phytate P was calculated as the difference between total P and free P. Total P was determined after treating the dietary samples with megadose of phytase to release the P bound by phytate. The free P, not bound by phytate, was determined after overnight extraction in 0.66 M HCl. Phytase activity was measured by a colorimetric method. One phytase unit was defined as the amount of enzyme that releases 1 µmol of inorganic phosphate from 5.0 mM phytate per minute at 37°C and pH 5.5.

In both broiler and pig trials, the data were analyzed by one-way ANOVA using GLM procedure of SAS (SAS Inc., Gary, NC, USA, version 9.0). Orthogonal contrasts were constructed to test the linear and quadratic effects of phytase variant supplementation to the NC diet. Tukey's multiple comparison test was also applied in broiler trial. The least square means are presented.

### Result

In the broiler study, the body weight gain and feed consumption of birds in NC was 10~15% lower than Cobb 500 performance targets during day 8 to 17 of age, which was attributed to the P deficiency in NC diet. The increasing addition of the phytase variant to the NC diet improved the 8-17 d body weight gain and feed intake of birds both linearly and quadratically (p < 0.01). The achievement of performance targets was observed in treatments with high doses of phytase variant while no adverse effect was noted. Additionally, the data from excreta demonstrated that the P release from phytate-P degradation, and the corresponding retention of P and Ca were improved both linearly and quadratically (p < 0.01) with the increase of phytase variant supplementation. Consistent with the performance and excreta results, Ca and P were increasingly deposited in the bone ash in a dose-dependent manner with the increase of phytase variant addition.

In the piglet study, the growth performance of piglets was depressed by deficiency of P in feed as demonstrated by the significant reduction in ADG and ADFI of the NC piglets in comparison to PC during day 14 to 42 and the overall trial duration, which was gradually corrected by the increasing addition of the phytase variant. The test phytase improved ADG and ADFI during day 14 to 42 and the overall trial duration both linearly and quadratically (p < 0.01) with the increasing dose of phytase variant. This pattern was also observed with the average body weight of the piglets at the end of the trial. In keeping with the growth performance results, the significant improvement (p < 0.01) in bone strength and bone content of ash, Ca and P in association with added phytase variant also showed a dose-response relationship. Moreover, the growth performance and bone measurements achieved at 3,000 FYT/kg feed, the highest dose of phytase variant tested in the current study, exceeded the levels of the PC without causing any noticeable adverse effect during the trial. This showed that both the Ca and P supplied in the form of dicalcium phosphate in the PC diets could be completely replaced by phytase variant when included at 3,000 FYT/kg feed.

**Table 9. growth performance, retention of Ca (%) and P (%), phytate-P degradation (%) in excreta and bone parameters of broilers.**

| | **Phytase variant treatments, FYT/kg feed** | | | | | | | | **SEM** | ***P-*value** | **Linear** | **Quadratic** |
|---|---|---|---|---|---|---|---|---|---|---|---|---|
| **Items** | **NC** | **187.5** | **375** | **750** | **1125** | **1500** | **1875** | **2250** | | | | |
| **Body weight, g** | | | | | | | | | | | | |
| **D 8** | **193** | **192** | **192** | **192** | **192** | **192** | **192** | **193** | **0.3** | **0.08** | **0.68** | **0.11** |
| **D 17** | **652^{a}** | **687 ^{b}** | **691 ^{b}** | **710 ^{bc}** | **716 ^{bc}** | **710^{bc}** | **724 ^{c}** | **716 ^{bc}** | **7.3** | **< 0.01** | **< 0.01** | **< 0.01** |

| **D8 to 17** | | | | | | | | | | | | |
|---|---|---|---|---|---|---|---|---|---|---|---|---|
| **WG, g** | **460 ^{a}** | **495 ^{b}** | **499 ^{b}** | **518 ^{bc}** | **524 ^{bc}** | **518 ^{bc}** | **532 ^{c}** | **523 ^{bc}** | **7.3** | **< 0.01** | **< 0.01** | **< 0.01** |
| **FI, g** | **572 ^{a}** | **600 ^{ab}** | **609 ^{ab}** | **618 ^{b}** | **621 ^{b}** | **608 ^{ab}** | **640 ^{b}** | **622 ^{b}** | **10.0** | **< 0.01** | **< 0.01** | **0.05** |
| **FCR, g/g** | **1.244** | **1.215** | **1.220** | **1.195** | **1.187** | **1.174** | **1.205** | **1.190** | **0.017** | **0.10** | **0.01** | **0.06** |

| **Excreta** | | | | | | | | | | | | |
|---|---|---|---|---|---|---|---|---|---|---|---|---|
| **Ca retention** | **55.08 ^{a}** | **63.00 ^{b}** | **66.03 ^{b}** | **73.14 ^{c}** | **74.64 ^{c}** | **75.70 ^{c}** | **73.43 ^{c}** | **73.19 ^{c}** | **0.84** | **< 0.01** | **< 0.01** | **< 0.01** |
| **P retention** | **64.41 ^{a}** | **71.46 ^{b}** | **75.63 ^{c}** | **80.52 ^{d}** | **82.48 ^{d}** | **83.31 ^{d}** | **81.95 ^{d}** | **81.87 ^{d}** | **0.65** | **< 0.01** | **< 0.01** | **< 0.01** |
| **Phytate-P degradation** | **64.01 ^{a}** | **77.03 ^{b}** | **82.99 ^{c}** | **89.17 ^{d}** | **92.88 ^{de}** | **94.38 ^{e}** | **91.86 ^{de}** | **94.56 ^{e}** | **0.97** | **< 0.01** | **< 0.01** | **< 0.01** |

| **Bone** | | | | | | | | | | | | |
|---|---|---|---|---|---|---|---|---|---|---|---|---|
| **Ash, %** | **46.80 ^{a}** | **50.14 ^{b}** | **51.22 ^{bc}** | **52.36 ^{cd}** | **52.93 ^{cde}** | **53.56 ^{de}** | **54.67 ^{e}** | **53.74 ^{de}** | **0.46** | **< 0.01** | **< 0.01** | **< 0.01** |
| **Ca, %** | **17.10 ^{a}** | **18.26^{b}** | **18.57 ^{bc}** | **19.07 ^{bcd}** | **19.22 ^{cd}** | **19.38 ^{cd}** | **19.96 ^{d}** | **19.38 ^{cd}** | **0.20** | **< 0.01** | **< 0.01** | **< 0.01** |
| **P, %** | **8.05^{a}** | **8.61^{b}** | **8.91 ^{bc}** | **9.22 ^{cd}** | **9.38 ^{de}** | **9.44 ^{de}** | **9.67^{e}** | **9.39 ^{de}** | **0.09** | **< 0.01** | **< 0.01** | **< 0.01** |

Means with different superscript within the same row differ significantly (P < 0.05).

**Table 10. growth performance and bone parameters of the piglets**

| | | | | **Phytase variant treatments, FYT/kg feed** | | | | | **SEM** | | ***P* - value** | |
|---|---|---|---|---|---|---|---|---|---|---|---|---|
| **Items** | | **PC** | **NC** | **187.5** | **375** | **750** | **1500** | **3000** | | **PC vs. NC** | **Linear** | **Quadratic** |
| **Body weight, kg** | | | | | | | | | | | | |
| | **D 0** | **7.4** | **7.5** | **7.5** | **7.4** | **7.4** | **7.4** | **7.5** | **0.05** | **0.23** | **0.81** | **0.34** |
| | **D 14** | **10.9** | **10.1** | **10.6** | **10.0** | **10.8** | **10.5** | **11.1** | **0.33** | **0.13** | **0.05** | **0.94** |
| | **D 42** | **25.9** | **19.6** | **22.5** | **22.6** | **25.3** | **25.7** | **27.0** | **0.70** | **< 0.01** | **< 0.01** | **< 0.01** |
| **D 0 to 14** | | | | | | | | | | | | |
| | **ADG, g/d/pig** | **247** | **181** | **221** | **184** | **238** | **221** | **258** | **24** | **0.06** | **0.04** | **0.74** |
| | **ADFI, g/d/pig** | **369** | **324** | **351** | **294** | **384** | **337** | **373** | **26** | **0.24** | **0.19** | **0.86** |
| | **FCR, g/g** | **1.52** | **1.82** | **1.61** | **1.64** | **1.65** | **1.53** | **1.44** | **0.08** | **0.02** | **0.01** | **0.44** |
| **D 14 to 42** | | | | | | | | | | | | |
| | **ADG, g/d/pig** | **537** | **338** | **425** | **448** | **521** | **541** | **570** | **19** | **< 0.01** | **< 0.01** | **< 0.01** |
| | **ADFI, g/d/pig** | **721** | **432** | **564** | **578** | **707** | **627** | **717** | **25** | **< 0.01** | **< 0.01** | **< 0.01** |
| | **FCR, g/g** | **1.34** | **1.27** | **1.32** | **1.28** | **1.36** | **1.16** | **1.27** | **0.06** | **0.41** | **0.41** | **0.43** |
| **D 0 to 42** | | | | | | | | | | | | |
| | **ADG, g/d/pig** | **440** | **286** | **357** | **360** | **426** | **434** | **466** | **17** | **< 0.01** | **< 0.01** | **< 0.01** |
| | **ADFI, g/d/pig** | **603** | **396** | **493** | **483** | **599** | **530** | **602** | **21** | **< 0.01** | **< 0.01** | **< 0.01** |
| | **FCR, g/g** | **1.40** | **1.45** | **1.42** | **1.40** | **1.46** | **1.28** | **1.33** | **0.05** | **0.48** | **0.05** | **0.33** |
| **Bone, %** | | | | | | | | | | | | |
| **Strength, N** | | **366** | **95** | **171** | **229** | **275** | **344** | **417** | **28** | **< 0.01** | **< 0.01** | **< 0.01** |
| | **Ash** | **63.1** | **56.8** | **59.9** | **60.9** | **60.4** | **61.8** | **63.6** | **0.77** | **< 0.01** | **< 0.01** | **0.09** |
| | **Ca** | **23.6** | **21.4** | **22.7** | **23.0** | **22.6** | **23.3** | **24.0** | **0.31** | **< 0.01** | **< 0.01** | **0.12** |
| | **P** | **11.0** | **9.6** | **10.2** | **10.4** | **10.4** | **10.9** | **11.3** | **0.15** | **< 0.01** | **< 0.01** | **0.02** |

| | | | | | | | | | | | | |
|---|---|---|---|---|---|---|---|---|---|---|---|---|
| 8 replicates for each treatment. | | | | | | | | | | | | |

### Example 9: Apparent total tract digestibility of calcium and phosphorus in late-gestation and lactating sows supplemented with a phytase variant

The objective of this study was to evaluate a phytase variant (var400) in late-gestation and lactating sows with a genetic background of Large white, Landrace and Duroc and fed diets formulated using the National Research Council (NRC) (2012) suggested digestible P for feed ingredients. Forty-five late-gestation sows and 45 lactating sows were used in Examples 1 and 2, respectively, in a completely randomized design. The sows were provided with a control diet (Table 11) and the control diet added with 187.5 or 375 FYT phytase /kg feed. The diets were devoid of any inorganic P supplement and included 3 g/kg TiO₂ as an indigestible marker. Each dietary treatment was replicated with 15 sows individually-housed in farrowing stalls. The sows were allowed to adapt to the experimental diets for 5 days before a 5-d fecal collection by grab sampling. Digestibility of dry matter, Ca and P were calculated using the following equations: ${D}_{d} = \left[1-\left({Ti}_{i}/{Ti}_{o}\right)\right] \times 100 ;$ ${D}_{m} = \left[1-\left({T}_{i}/{T}_{o}\right)\times \left({M}_{o}/{M}_{i}\right)\right] \times 100 ;$ where D_{d} and Dₘ are the digestibility of DM and minerals (%), respectively; Tᵢ and Tₒ are the titanium concentration in diet and feces, respectively (% of DM); Mᵢ and Mₒ are the concentrations of minerals in diet and feces (% of DM), respectively. The digested Ca and P were calculated by multiplying the concentration of Ca and P in feed (%) by their corresponding Dₘ.

The digestibility and performance data were analyzed by a GLM procedure of SAS (SAS Inst. Inc., Cary, NC) with the model including the dietary treatment as the only fixed effect and error term. Orthogonal contrasts were constructed to test the linear and quadratic effects of supplementation of phytase and to compare control with the treatments with added phytase.

The results are shown in Table 12. The phytase released 0.07-0.12% digestible P for sows depending on the phytase dose and physiological stage of sows (more P release for lactating sows).

**Table 11. Ingredient and nutrient composition of the basal diets (g/kg of feed, as-is basis)**

| Items | Gestation | Lactation |
|---|---|---|
| Ingredients | | |
| Corn | 680.0 | 620.0 |
| Soybean meal | 80.0 | 140.0 |
| Wheat bran^{a} | 200.0 | 120.0 |
| Full-fat soybean | 0.0 | 50.0 |
| Soybean oil | 0.0 | 32.0 |
| NaCl | 2.0 | 3.0 |
| NaHCO₃ | 3.5 | 3.5 |
| L-Lys·HCl | 4.0 | 3.5 |
| DL-Met | 1.0 | 0.5 |
| L-Thr | 1.5 | 1.5 |
| L-Val | 0.5 | 1.5 |
| L-Trp | 0.5 | 0.5 |
| Limestone | 19.0 | 15.5 |
| Vitamin-mineral premix^{b} | 5.0 | 5.0 |
| Stay C, 35% vitamin C | 0.0 | 0.5 |
| TiO₂ | 3.0 | 3.0 |
| Total | 1000.0 | 1000.0 |

| Calculated nutrients and energy | | |
|---|---|---|
| ME, MJ/kg | 12.7 | 13.9 |
| Crude protein | 12.8 | 15.6 |
| Total Ca | 8.4 | 7.3 |
| Total P | 4.3 | 4.0 |
| Phytate P | 3.5 | 3.0 |
| Apparent total tract digestible P | 1.6 | 1.4 |
| Standardized total tract digestible P | 2.0 | 1.8 |

| Standardized ileal digestible | | |
|---|---|---|
| Lysine | 7.1 | 8.6 |
| Methionine | 2.8 | 2.6 |
| Threonine | 4.9 | 5.9 |
| Tryptophan | 1.6 | 1.9 |
| Valine | 4.9 | 7.0 |

| | | |
|---|---|---|
| ^{a} Wheat bran was pelleted at 95°C before the mixing of diet. ^{b} Premix supplied per kilogram of diet: vitamin A, 12,000 IU; vitamin D₃, 2,000 IU; vitamin E, 115 mg; vitamin K₃, 5.0 mg; vitamin B₁, 2.0 mg; vitamin B₂, 6 mg; vitamin B₆, 3.0 mg; vitamin B₁₂, 28 µg; D-biotin, 700 µg; pantothenic acid, 20 mg; folic acid, 5 mg; niacin, 30 mg; Cu (tribasic copper chloride), 20 mg; I (potassium iodate), 0.45 mg; Fe (ferrous sulfate), 120 mg; Mn (manganese sulfate), 60 mg; Zn (zinc sulfate), 100 mg; Se (sodium selenite), 400 µg; choline (choline chloride), 600 mg; and Ca (calcium carbonate) 0.5 g. | | |

**Table 12. Apparent total-tract digestibility of calcium (Ca) and phosphorus (P) and digested Ca and P in feed for gestating and lactating sows, %^{a}**

| Items | | Treatments, FYT/kg feed | | | SEM^{b} | Significance level | | |
|---|---|---|---|---|---|---|---|---|
| | | Control | 187.5 | 375.0 | | Phytase | Linear | Quadratic |
| Gestation | | | | | | | | |
| | Dry matter | 82.7 | 81.9 | 82.1 | 0.336 | 0.121 | 0.254 | 0.253 |
| | Calcium | 28.7 | 32.2 | 33.9 | 1.255 | 0.007 | 0.006 | 0.548 |
| | Phosphorus | 15.3 | 28.1 | 33.8 | 1.071 | < 0.001 | < 0.001 | 0.011 |
| | Digested Ca | 0.23 | 0.28 | 0.29 | 0.011 | < 0.001 | < 0.001 | 0.293 |
| | Digested P | 0.07 | 0.14 | 0.17 | 0.005 | < 0.001 | < 0.001 | 0.006 |
| | Digested Ca/digested P | 3.22 | 2.02 | 1.78 | 0.112 | < 0.001 | < 0.001 | 0.001 |
| Lactation | | | | | | | | |
| | Dry matter | 86.7 | 86.4 | 86.3 | 0.294 | 0.404 | 0.384 | 0.870 |
| | Calcium | 33.9 | 36.4 | 40.7 | 1.486 | 0.016 | 0.003 | 0.603 |
| | Phosphorus | 23.6 | 43.9 | 50.2 | 0.903 | < 0.001 | < 0.001 | < 0.001 |
| | Digested Ca | 0.24 | 0.25 | 0.29 | 0.010 | 0.046 | 0.007 | 0.414 |
| | Digested P | 0.10 | 0.19 | 0.22 | 0.004 | < 0.001 | < 0.001 | < 0.001 |
| | Digested Ca/P | 2.43 | 1.32 | 1.28 | 0.045 | < 0.001 | < 0.001 | < 0.001 |
| | Reproductive stage | | Treatment | Interaction | | | | |
| | Dry matter | < 0.001 | 0.222 | 0.716 | | | | |
| | Calcium | < 0.001 | < 0.001 | 0.622 | | | | |
| | Phosphorus | < 0.001 | < 0.001 | < 0.001 | | | | |
| | Digested Ca/digested P | 0.514 | < 0.001 | 0.313 | | | | |
| | Digested P | < 0.001 | < 0.001 | < 0.001 | | | | |
| | Digested Ca/P | < 0.001 | < 0.001 | 0.222 | | | | |

| | | | | | | | | |
|---|---|---|---|---|---|---|---|---|
| ^{a} Each least square mean represents 15 observations. ^{b} SEM: standard error of mean. | | | | | | | | |

### Example 10: Phytase efficacy in rainbow trout (Oncorhynchus mykiss)

### Experimental Diets

Experimental diets were prepared in the experimental feed mill of the Research Centre for Animal Nutrition & Health, DSM Nutritional Products in Village-Neuf (France) according to the formulation detailed in Table 13. Diets were prepared in mash and produced as extruded pellets using a Bühler twin screw extruder. After extrusion, pellets were coated with a mixture of oils heated at 40°C and phytase (var400). Experimental diets were kept at 4°C during the feeding trial.

Theoretical calculations were 0.821 % for total phosphorus, 0.326 % for phytate phosphorus and 11.5 g/Kg phytic acid (according to Allix 3 software, A-systems, France).

### Fish and rearing facilities

Two hundred and forty monosex (all female) Rainbow trout (IBW= 46.3 ± 1.2g) were randomly distributed in twelve tanks (250L; twenty fish per tank).

**Table 13. Detailed composition (%) of the basal diet and calculated crude protein/lipid in the diet**

| Ingredient | Experimental diet |
|---|---|
| Fish meal | 13.20 |
| Soybean meal | 8.00 |
| SPC | 17.00 |
| Rapeseed meal | 7.00 |
| Sunflower meal | 8.00 |
| Wheat | 11.00 |
| Wheat gluten | 12.00 |
| Fish oil | 11.00 |
| Rapeseed oil | 9.90 |
| Choline chloride 60% | 0.4 |
| DSM OVM VMF | 0.5 |
| L-Lysine | 0.5 |
| L-Methionine | 0.2 |
| Yttrium oxide | 200 ppm |
| Guar gum | 0.3 |
| Calc. crude protein | 40.0 |
| Calc. crude lipid | 24.0 |

### Feeding

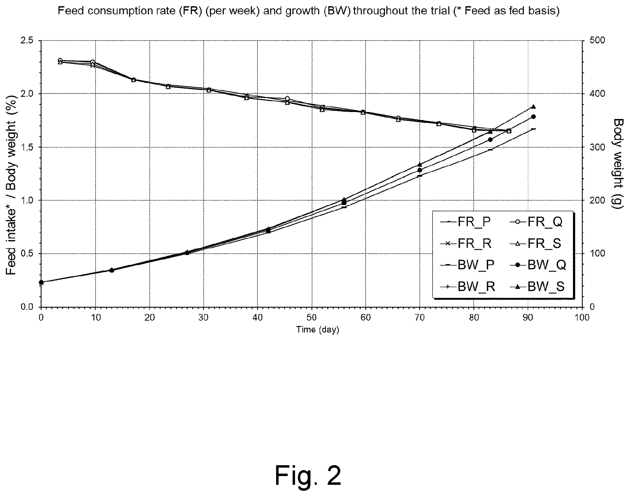

Animals were fed for 91 days. Trout were fed by hand twice a day (morning and afternoon) during the week and by automatic feeders during weekends. All fish were fed according to feeding ration table for a similar commercial diet fed to fish maintained at the same water temperature. Feed consumption and body weight thorough the experiment are represented in Figure 2. Feed consumption is calculated as percentage, based on the actual intake per week and the growth of the fish (specific growth rate) during the 2-week period. Feed consumption ranged from 2.31 % to 1.65 % throughout the experimental feeding (Figure 2).

### Zootechnical parameters

Fish were individually weighed at the beginning of the trial and the mean body weight of the fish per triplicate tanks was determined after bulk weighing of the fish at each time point considered. Bulk weight was recorded every two weeks. Before handling, fish were anesthetized (0.08 g/L tricaine methane sulfonate; MS222; PharmaQ Ltd., Overhalla, Norway).

The following zootechnical parameters were measured/ calculated:
- Survival (%)
- Performance
- Initial body weight, IBW (g)
- Final body weight, FBW (g)
- Weight gain, WG as FBW - IBW (g)
- Specific growth rate, SGR as {100 * (In (FBW/IBW)]) * d⁻¹ (%BW d⁻¹)
- Feed utilization
- Feed conversion ratio, FCR as Feed Intake * biomass gain⁻¹ (as-fed basis)

### Phosphorus whole body retention

At the beginning and at the end of the trial, whole fish were sampled and frozen at - 80°C to analyze phosphorus whole body retention.

### Plasma

At the end of the experimental feeding trial, five fish per tank (15 fish per treatment) were individually anaesthetized using 80 mg MS222·L⁻¹.

Four mL of blood were sampled using a lithium-heparin 4.5 mL syringe and kept in ice until further processing. Blood samples were centrifuged (10 min, 2000g, 4°C) and 1.5 mL plasma aliquots were frozen (-20°C) until analysis. Samples were analyzed individually.

Samples were analyzed individually but each tank was considered as a replicate for statistical purposes.

### Apparent digestibility coefficient (ADC)

Two different faeces collection were done 77 and 91 days after the start of the experimental feeding for the determination of phosphorus ADC. Animals were slightly anaesthetized (80 mg MS222·L⁻¹) and fecal material from all fish in each tank was collected by manually stripping faeces from the distal portion of the intestine by applying pressure to the abdominal cavity using three passes for each fish. Samples from both collection days were pooled and one sample per tank was lyophilized prior to storage and analysis.

The apparent digestibility coefficient (ADC) was calculated as outlined by the NRC (2001) on a dry matter basis: $ADC \left(%\right) = 100 - \left[\left(CMf/CMe\right)\times \left(CNe/CNf\right)\right] \times 100$
CMf =concentration of marker in feed; CMe =concentration of marker in faeces;
CNf = concentration of nutrient in feed; CNe =concentration of nutrient in faeces Phosphorus release represents the % of phosphorus released due to phytase supplementation. It is calculated as follows: 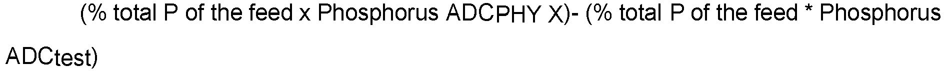 (% total P of the feed x Phosphorus ADCPHY X)- (% total P of the feed * Phosphorus ADCtest)

Where % total P is the total percentage of phosphorus in the diet; ADCPHY X is the digestibility of the phosphorus at a given phytase dose; and Phosphorus ADCₜₑₛₜ is the phosphorus digestibility of the test diet without phytase supplementation.

### Bone phosphorus retention

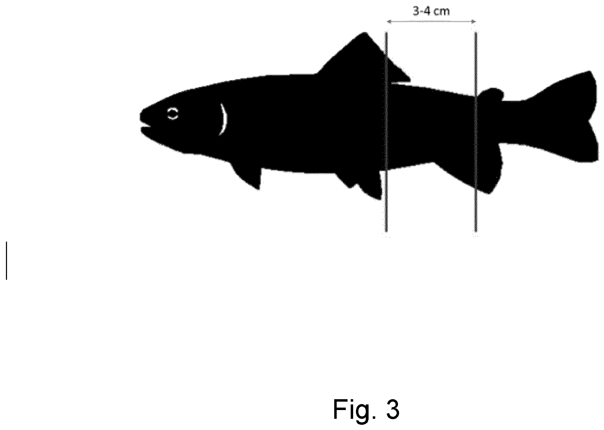

At the beginning and at the end of the trial, a 3-4 cm slide (see figure 3) was sampled in five fish per tank(15 fish per treatment) and frozen at -80°C. Skin and flesh will be removed to further process only the bones for analysis.

### Analyses and calculations

The analyses of the nutrient content in the feed, excreta, plasma, whole fish and vertebra samples were performed according to standard methods (VDLUFA 1976; AOAC, 2006).

Phytate-P in feed were measured by enzyme laboratory DNP R&D Solution Center (Kaiseraugst) with an enzymatic method using ammonium molybdate by calculation of the difference between the total P and free P, according to Zhai et al., 2001.

The crude protein was determined by a nitrogen analyzer (FP 528, LECO, St. Joseph, USA) using the Dumasmethod (CP=N*6.25).

Gross energy measurements were performed using an adiabatic bomb calorimeter (C 2000 basic, IKA, Staufen, Germany).

Calcium, Phosphorus, Zinc and Yttrium oxide concentrations in feed, whole fish, vertebra and excreta were determined by Inductively Coupled Plasma-Optical Emission Spectrometry (ICP-OES, 5100 Dual View, Agilent) according to DIN EN ISO 11885:1997 (DIN EN ISO 1998; AOAC, 2006) after sulfuric acid mineralization. Water analysis of phosphorus were performed on the same instrument on frozen water samples by direct injection.

The plasma concentrations of P concentration were determined by the mean of a Biomedical automate COBAS 6000 (Roche Diagnostics, CH-4202 Basel) using the respective Roche Diagnostic kits.

The lipid analyses of feed were performed at the external company Larebron by hot acidic etching with hydrochloric acid, followed by continuous extraction with petroleum ether and determination with a gravimetric method.

Phytase analysis in the diets was done at BioPract GmbH, Germany, according to ISO 30024:2009 (Animal feeding stuffs - Determination of phytase activity, https://www.iso.org/standard/45787.html).

### Result

Table 14 shows the analytical results obtained at BioPract GmbH.

**Table 14. Phytase content in the eight experimental treatments. LOD = below level of detection.**

| Treatment ID | Dose U/Kg (theoretical) | Pellet Ø (mm) | Dose U/Kg (analytical) |
|---|---|---|---|
| P | Control -0 | 3 | LOD |
| Q | phytase 1000 FYT/kg | 3 | 955 |
| R | phytase 2000 FYT/kg | 3 | 1740 |
| S | phytase 3000 FYT/kg | 3 | 2477 |
| P | Control -0 | 4 | LOD |
| Q | phytase 1000 FYT/kg | 4 | 977 |
| R | phytase 2000 FYT/kg | 4 | 1719 |
| S | phytase 3000 FYT/kg | 4 | 2895 |

### Fish behavior and water quality

No mortality was recorded through the whole experiment and fish accepted well all the experimental diets.

### Performance

Table 15 shows the zootechnical parameters at the end of the 91-day experimental feeding period. Control diet showed the poorest growth rate. Supplementation with phytase had a positive impact in growth. This trend was dose-related and supplementation in 1000 FYT/Kg group growth was significantly higher than the control group; similarly, animals with 2000 and 3000 FYT/Kg enzyme supplementation perform significantly better than the 1000 FYT/Kg.

Specific growth rate and feed conversion ratio were also significantly improved with any inclusion level of phytase but independently from the inclusion level.

**Table 15. Survival and growth performance of fish (day 0 to day 91).**

| ID | Dose | Survival | IBW | FBW | SGR | FCR |
|---|---|---|---|---|---|---|
| P | Control -0 | 100 ± 0 | 46.3 ± 0.0 | 334.7 ± 7.0 c | 2.17 ± 0.02 b | 0.87 ± 0.0 a |
| Q | phytase 1000 FYT/kg | 100 ± 0 | 46.3 ± 0.0 | 357.2 ± 10.1 b | 2.25 ± 0.03 a | 0.83 ± 0.01 b |
| R | phytase 2000 FYT/kg | 100 ± 0 | 46.3 ± 0.0 | 374.4 ± 3.0 a | 2.30 ± 0.01 a | 0.82 ± 0.01 b |
| S | phytase 3000 FYT/kg | 98.3 ± 2.9 | 46.3 ± 0.0 | 376.0 ± 9.7 a | 2.30 ± 0.03 a | 0.81 ± 0.01 b |
| | sign | ns | ns | p = 0.0006 | p = 0.0006 | P=0.0004 |

| | | | | | | |
|---|---|---|---|---|---|---|
| *Survival percentages referred to the whole duration of the trial; IBW: Initial Body Weight (g); FBW: Final Body Weigh (g)t; SGR: specific growth rate* (%BW/day); *FCR: feed conversion ratio* (as-fed basis). *Data arepresented* as *mean* ± *SD (Standard Deviation). Sign. = significance; Ns: no significant differences were observed between treatments.* *Whole body phosphorus retention* | | | | | | |

Table 16 shows the whole body retention (% of intake) for minerals and protein. Significant improvement in whole body zinc and protein retention at any supplementation dose. Phosphorus was significantly improved when phytase was supplemented at 1000 and 3000 FYT/Kg. Surprisingly, phytase inclusion at 2000 FYT/Kg showed an intermediate response.

**Table 16. Phosphorus, zinc, calcium and protein whole body retention (% of intake; day 0 to day 91).**

| ID | Dose | Phosphorus | Zinc | Calcium | Protein |
|---|---|---|---|---|---|
| P | Control -0 | 1.64 ± 0.29 b | 0.60 ± 0.04 b | 0.52 ± 0.12 | 2.22 ± 0.08 b |
| Q | phytase 1000 FYT/kg | 2.63 ± 0.56 a | 1.25 ± 0.25 a | 1.68 ± 0.78 | 2.65 ± 0.32 a |
| R | phytase 2000 FYT/kg | 2.27 ± 0.37 ab | 1.15 ± 0.24 a | 1.34 ± 0.38 | 2.48 ± 0.02 ab |
| S | phytase 3000 FYT/kg | 2.60 ± 0.17 a | 1.28 ± 0.10 a | 1.48 ± 0.30 | 2.57 ± 0.03 ab |
| | Sign | p=0.0398 | p=0.0055 | Ns p = 0.0640 | Ns p = 0.0526 |

| | | | | | |
|---|---|---|---|---|---|
| *Data are presented* as *mean ± SD (Standard Deviation; n=3). Sign. = significance; Ns: no significant differences were observed between treatments.* *Phosphorus content in plasma* | | | | | |

Plasma phosphorus concentration was significantly increased with the supplementation of phytase. The supplementation of the exogenous enzyme increased the circulating phosphorus 1.99, 2.3 and 2.44 fold when compared to control diet.

### Apparent digestibility coefficient (ADC)

The faecal digestibility for dry matter, protein, energy and minerals (phosphorus, calcium and zinc) is summarized in the Table 17.

Data show that the supplementation of phytase from 1000 FYT/kg significantly improved the faecal dry matter, energy digestibility as well as the faecal calcium, phosphorus and zinc digestibility. Phosphorus digestibility was increased by 60.1, 82.9 and 89.7% for phytase 1000 FYT/kg, phytase 2000 FYT/kg and phytase 3000 FYT/kg, respectively, when compared to the non-supplemented control group. Accordingly, based on the above results, an aspect of the invention is directed to method of feeding fish comprising adding the phytase, as defined herein, such as in an amount of 500 to 5000 FYT/kg. In typical embodiments, the amount of phytase is from 500 to 3000 FYT/kg, such as 750 to 3000 FYT/kg, such as 1000 to 3000 FYT/kg.

**Table 17. Apparent digestibility coefficient (%) and phosphorus release (Phos. Rel.; % phosphorus / kg feed).**

| ID | Dose | Dry matter | Protein | Energy | Phosphorus | Calcium | Zinc | Phos. Rel. |
|---|---|---|---|---|---|---|---|---|
| P | Control -0 | 69.8 ± 0.18 c | 89.6 ± 0.08 b | 78.9 ± 0.13c | 32.1 ± 1.06d | -0.6 ± 2.03 c | 20.0 ± 0.97c | |
| Q | phytase 1000 FYT/kg | 71.3 ±0.55 b | 90.0 ± 0.19 b | 79.6 ± 0.31 b | 51.4 ± 0.62c | 5.2 ± 1.46 b | 28.6 ± 0.65 b | 0.14 |
| R | phytase | 71.4 | 90.0 | 79.5 | 58.7 | 7.3 | 32.0 | 0.20 |
| | 2000 FYT/kg | ± 0.16b | ± 0.21b | ± 0.07b | ± 0.31b | ± 1.26ba | ± 1.90a | |
| S | phytase 3000 FYT/kg | 72.6 ±0.30a | 90.6 ± 0.20 a | 80.2 ± 0.30 a | 60.9 ± 1.89 a | 10.2 ± 1.89a | 34.0 ± 2.09 a | 0.21 |
| | Sign | p=0.0001 | p=0.0013 | p = 0.0006 | p < 0.0001 | p = 0.0003 | p < 0.0001 | |

| | | | | | | | | |
|---|---|---|---|---|---|---|---|---|
| *Newman-Keuls test: Means within* a *row, not sharing a common superscript, are significantly different (p<0.05). Digestibility were calculated with n=3 tanks per treatment.* | | | | | | | | |

### Bone phosphorus retention

Retention of phosphorus is summarized in Table 18. In the vertebrae, percentage of ash and the concentration of phosphorus increased significantly with phytase incorporation when compared to the negative control.

**Table 18. Retention of ash and phosphorus in vertebrae of fish (% of DM) after 91 days of experimental feeding.**

| ID | Dose | Ash | Phosphorus |
|---|---|---|---|
| P | Control -0 | 21.2 ± 3.4b | 3.77 ± 0.66b |
| Q | phytase 1000 FYT/kg | 28.6 ± 1.4a | 5.16 ± 0.26a |
| R | phytase 2000 FYT/kg | 28.6 ± 3.0 a | 5.17 ± 0.58a |
| S | phytase 3000 FYT/kg | 28.9 ± 1.0a | 5.26 ± 0.19a |
| | sign | p = 0.0116 | p = 0.013 |

| | | | |
|---|---|---|---|
| *Newman-Keuls test: Means within a row, not sharing a common superscript, are significantly different (p<0.05). Digestibility were calculated with n=3 tanks per treatment.* | | | |

### Conclusion

The supplementation of the reference diet (0.74 total phosphorus) with the phytase at 1000, 2000 and 3000 FYT/kg showed a dose-dependent and significant increase in growth, plasma phosphorus and plasma apparent digestibility after 91 days of experimental feeding. The inclusion of phytase at any level increased the retention of phosphorus in whole body and vertebrae of the animals.

The phytase of the invention is efficient in releasing phosphorus from phytate and the invention is directed in part to the use of the phytase when fish meal in the diets of salmonid fish is replaced by vegetable raw materials rich in this antinutritional factor.

### Example 11: Effect of graded supplemental levels of phytase on the growth performance, whole-body nutrient retention and nutrient digestibility in European seabass (Dicentrarchus labrax) fed a plant-protein rich diet

The trial comprised five dietary treatments: a control diet (CTRL) with a total dietary phosphorus (P) level of 0.7%, in which a significant fraction of P was present in the form of phytate-bound P (0.3%). Three other diets, based on the CTRL formulation, were supplemented with phytase (var400) at graded doses (500, 1000 and 2000 FTY/kg feed) (diets PHY500, PHY1000, PHY2000). Phytase was applied post-extrusion by coating. A fifth diet (MCP), also based on the CTRL formulation, was supplemented with monocalcium phosphate, to a total P level of 0.9% and an available P level higher than the known requirements of the species. All diets were isonitrogenous, isolipidic and isoenergetic. Quadruplicate groups of 38 European seabass, with a mean initial body weight of 57.6 ± 3.8 g were fed one of the five experimental diets during 94 days, with a water temperature profile of 22.1 ± 0.4°C.

**Table 19. Formulation of experimental diets and measured phytase activity**

| **Ingredients, %** | **CTRL** | **PHY500** | **PHY1000** | **PHY2000** | **MCP** |
|---|---|---|---|---|---|
| Fishmeal Super Prime | 7.50 | 7.50 | 7.50 | 7.50 | 7.50 |
| Fish protein hydrolysate | 2.50 | 2.50 | 2.50 | 2.50 | 2.50 |
| Wheat gluten | 10.10 | 10.10 | 10.10 | 10.10 | 10.50 |
| Corn gluten meal | 20.00 | 20.00 | 20.00 | 20.00 | 20.00 |
| Soybean meal | 24.00 | 24.00 | 24.00 | 24.00 | 24.00 |
| Rapeseed meal | 12.00 | 12.00 | 12.00 | 12.00 | 12.00 |
| Sunflower meal | 5.00 | 5.00 | 5.00 | 5.00 | 5.00 |
| Wheat bran | 3.03 | 3.03 | 3.03 | 3.03 | 1.43 |
| Fish oil | 5.00 | 5.00 | 5.00 | 5.00 | 5.00 |
| Rapeseed oil | 9.60 | 9.60 | 9.60 | 9.60 | 9.60 |
| Vitamin and mineral premix | 1.00 | 1.00 | 1.00 | 1.00 | 1.00 |
| Antioxidant | 0.20 | 0.20 | 0.20 | 0.20 | 0.20 |
| Monocalcium phosphate | | | | | 1.20 |
| L-Tryptophan | 0.02 | 0.02 | 0.02 | 0.02 | 0.02 |
| DL-Methionine | 0.03 | 0.03 | 0.03 | 0.03 | 0.03 |
| Yttrium oxide | 0.02 | 0.02 | 0.02 | 0.02 | 0.02 |
| **phytase (FTY/kg)** | | **500** | **1000** | **2000** | |

### Result

At the end of the trial, the final body weight (FBW) ranged between 142.0 and 156.5 grams. Fish from the best performing treatment (PHY2000) showed a 2.7-fold increaseof initial body weight (IBW). At day 94, all supplemented diets resulted on a significantly higher FBW and SGR than those fed the CTRL diet (P<0.05). Fish fed the PHY500, PHY1000, PHY2000 and MCPdiets showed a significantly lower FCR than those fed the CTRL diet (P<0.05). The CTRL treatment was consistently associated to significantly lowest values among the various treatments for whole-body phosphorus retention and apparent digestibility of phosphorus. The higher phytase supplementation doses (1000 and 2000 FTY/kg) led to a significant increase of whole-body P content (P<0.05). Fish fed the MCP diet showed a significantly higher whole-body P retention than those fed the CTRL diet, although significantly lower than those fed the PHY1000 and PHY2000 diets. Significant enhancements of total P and phytate-P digestibility were associated to increasing dietary doses of phytase, with dietary treatment PHY2000 having a significantly higher P digestibility than PHY1000, and the later having a significantly higher P digestibility than PHY500. Additionally, the increase of phytase supplementation doses (0, 500, 1000 and 2000 FTY/kg) was also positively associated to a stepwise increase of whole-body phosphorus retention. Accordingly, based on the above results, an aspect of the invention is directed to method of feeding fish comprising adding the phytase, as defined herein, such as in an amount of 250 to 5000 FYT/kg. In typical embodiments, the amount of phytase is from 300 to 4000 FYT/kg, such as 500 to 3000 FYT/kg.

**Table 20. Growth performance after 94 days of feeding (end of the trial).**

| **I** | **CTRL** | **PHY500** | **PHY1000** | **PHY2000** | **MCP** | **P-value** |
|---|---|---|---|---|---|---|
| Survival, % | 99.3 ± 1.3 | 100.0 ± 0.0 | 100.0 ± 0.0 | 100.0 ± 0.0 | 100.0 ± 0.0 | 0.438 |
| IBW, g | 57.6 ± 0.1 | 57.5 ± 0.1 | 57.5 ± 0.1 | 57.6 ± 0.1 | 57.5 ± 0.1 | 0.572 |
| FBW, g | 142.0 ± 2.4 ^{a} | 148.6 ± 1.6 ^{b} | 153.0 ± 1.6 ^{c} | 156.5 ± 2.6 ^{c} | 152.5 ± 2.9 ^{c} | **<0.001** |
| SGR, %/d | 0.96 ± 0.02 ^{a} | 1.01 ± 0.01 ^{b} | 1.04 ± 0.01 ^{c} | 1.06 ± 0.02 ^{c} | 1.04 ± 0.02 ^{c} | **<0.001** |
| FCR | 1.35 ± 0.07 ^{b} | 1.25 ± 0.03 ^{a} | 1.24 ± 0.02 ^{a} | 1.19 ± 0.01 ^{a} | 1.24 ± 0.02 ^{a} | **<0.001** |
| FI, %ABW/d | 1.21 ± 0.05 | 1.18 ± 0.03 | 1.19 ± 0.02 | 1.17 ± 0.02 | 1.19 ± 0.03 | 0.412 |
| PER | 1.62 ± 0.08 ^{a} | 1.75 ± 0.05 ^{b} | 1.77 ± 0.03 ^{bc} | 1.83 ± 0.02 ^{c} | 1.74 ± 0.03 ^{b} | **<0.001** |

Values are means ± standard deviation (n=4).

Values within a row with different superscripts, denote a statistical difference (P<0.05).

**Table 21. Whole-body nutrient retention (% of intake).**

| | **CTRL** | **PHY500** | **PHY1000** | **PHY2000** | **MCP** | **P-value** |
|---|---|---|---|---|---|---|
| Protein, % | 21.2 ± 2.1 ^{a} | 23.3 ± 0.5 ^{ab} | 23.6 ± 2.0 ^{ab} | 24.6 ± 0.7 ^{b} | 22.2 ± 0.4 ^{ab} | ***0.027*** |
| Fat, % | 61.5 ± 2.1 | 67.7 ± 1.3 | 67.9 ± 3.7 | 66.4 ± 2.8 | 65.6 ± 5.7 | 0.118 |
| Energy, % | 31.5 ± 1.9 ^{ab} | 34.3 ± 1.6 ^{b} | 32.0 ± 1.8 ^{ab} | 31.4 ± 0.7 ^{ab} | 29.9 ± 1.0 ^{a} | ***0.013*** |
| Phosphorus, % | 40.3 ± 1.3 ^{a} | 44.9 ± 1.6 ^{b} | 51.8 ± 1.5 ^{c} | 62.0 ± 2.9 ^{d} | 46.3 ± 3.3 ^{b} | ***<0.001*** |
| Calcium, % | 36.9 ± 1.8 ^{b} | 40.7 ± 4.4 ^{b} | 47.0 ± 2.8 ^{c} | 48.5 ± 3.6 ^{c} | 30.1 ± 3.6 ^{a} | ***<0.001*** |

Values are average ± standard deviation (n=4).

Values within a row with different superscripts, denote a statistical difference (P<0.05).

**Table 22. Apparent digestibility coefficients (ADC, %) of nutrients.**

| | **CTRL** | **PHY500** | **PHY1000** | **PHY2000** | **MCP** | **P-value** |
|---|---|---|---|---|---|---|
| Dry matter, % | 66.9 ± 0.3 ^{b} | 70.4 ± 0.7 ^{d} | 69.0 ± 1.2 ^{cd} | 68.2 ± 1.1 ^{bc} | 64.1 ± 1.4^{a} | **<0.001** |
| Protein, % | 89.4 ± 0.1 ^{a} | 91.2 ± 0.6 ^{c} | 90.6 ± 0.3 ^{b} | 90.2 ± 0.5 ^{b} | 88.9 ± 0.3 ^{a} | **<0.001** |
| Phosphorus, % | 33.5 ± 1.3 ^{a} | 41.4 ± 3.0 ^{b} | 50.1 ± 2.3 ^{c} | 62.8 ± 0.7 ^{d} | 52.8 ± 2.3 ^{c} | **<0.001** |
| Phytate-P, % | 23.9 ± 0.8 ^{b} | 33.5 ± 1.7 ^{c} | 37.2 ± 3.0 ^{c} | 42.4 ± 1.8 ^{d} | 18.1 ± 4.4 ^{a} | **<0.001** |
| Calcium, % | 37.8 ± 1.1 ^{b} | 40.5 ± 0.7 ^{b} | 38.5 ± 2.4 ^{b} | 38.2 ± 3.6 ^{b} | 29.8 ± 2.7 ^{a} | **<0.001** |

Values are average ± standard deviation (n=4).

Values within a row with different superscripts, denote a statistical difference (P<0.05).

### Conclusion

The phytase at supplementation doses of 500 to 5000 FTY/kg feed, such as 500 to 3000 FTY/kg, such as 500 to 2500 FTY/kg, such as 500 to 2000 FTY/kg such as 1000 to 2000 FTY/kg feed is an effective strategy to enhance the growth rate, phosphorus digestibility, whole-body phosphorus retention and reduce FCR in European seabass fed plant protein-rich diets.

### Example 12: Efficacy of a phytase on the growth performance, whole-body nutrient retention and nutrient digestibility in Nile tilapia (Oreochromis niloticus)fed plant-protein rich diets

The trial comprised five dietary treatments: a control diet (CTRL) with a total dietary phosphorus (P) level of 0.96%, in which a significant fraction of P was present in the form of phytate-bound P (0.6%). Three other diets, based on the CTRL formulation, were supplemented with phytase (var400) at graded doses (500, 1000 and 2000 FTY/kg feed) (diets PHY500, PHY1000, PHY2000). Phytase was applied post-extrusion by coating. A fifth diet (DCP), also based on the CTRL formulation, was supplemented with dicalcium phosphate, to a total P level of 1.2% and an available P level higher than the known requirements of the species. All diets were isonitrogenous, isolipidic and isoenergetic. Quadruplicate groups of 30 tilapia, with a mean initial body weight of 39.5 ± 1.4 g were fed one of the fiveexperimental diets during 93 days, with a water temperature profile of 25.5 ± 0.4°C.

**Table 23. Formulation of experimental diets**

| **Ingredients, %** | **CTRL** | **PHY500** | **PHY1000** | **PHY2000** | **DCP** |
|---|---|---|---|---|---|
| Fishmeal 60¹ | 2.50 | 2.50 | 2.50 | 2.50 | 2.50 |
| Guar korma meal² | 4.50 | 4.50 | 4.50 | 4.50 | 4.80 |
| Soybean meal | 32.50 | 32.50 | 32.50 | 32.50 | 32.50 |
| Rapeseed meal | 20.00 | 20.00 | 20.00 | 20.00 | 20.00 |
| Wheat bran | 14.00 | 14.00 | 14.00 | 14.00 | 14.00 |
| Rice bran | 14.00 | 14.00 | 14.00 | 14.00 | 14.00 |
| Corn meal | 8.20 | 8.20 | 8.20 | 8.20 | 6.53 |
| Fish oil | 0.50 | 0.50 | 0.50 | 0.50 | 0.50 |
| Rapeseed oil | 1.90 | 1.90 | 1.90 | 1.90 | 2.00 |
| Vit & Min premix | 1.00 | 1.00 | 1.00 | 1.00 | 1.00 |
| Antioxidant | 0.20 | 0.20 | 0.20 | 0.20 | 0.20 |
| Dicalcium phosphate | | | | | 1.60 |
| Calcium carbonate | 0.33 | 0.33 | 0.33 | 0.33 | |
| L-Lysine HCl | 0.10 | 0.10 | 0.10 | 0.10 | 0.10 |
| L-Tryptophan | 0.05 | 0.05 | 0.05 | 0.05 | 0.05 |
| DL-Methionine | 0.20 | 0.20 | 0.20 | 0.20 | 0.20 |
| Yttrium oxide | 0.02 | 0.02 | 0.02 | 0.02 | 0.02 |
| **phytase (FTY/kg)** | | **500** | **1000** | **2000** | |

| | | | | | |
|---|---|---|---|---|---|
| ¹ COFACO 60: 62.3% crude protein (CP), 8.4% crude fat (CF), COFACO, Portugal; ² Guar Korma: 55.3% CP, 7.8% CF, KFEED Ltd, Bulgaria. | | | | | |

### Result

At the end of the trial, fish from the best performing treatment (PHY2000) showed a 4.7-fold increase of initial body weight. Fish fed the CTRL diet showed a significantly lower FBW, SGR, PER, and a higher FCR than those fed all other diets (P<0.05). Moreover, fish fed the PHY1000, PHY2000 and DCP diets showed a significantlyhigher FBW and SGR than those fed the PHY500 and CTRL diets (P<0.05). Fish fed the PHY2000 diet showed a significantly lower FCR than those fed the PHY500 diet (P<0.05). Feed intake (FI) varied between 1.75 and 1.82 %ABW per day and was not significantly affected by dietary treatments (P>0.05). Fish fed the CTRL diet showed a significantly lower whole-body phosphorus (P) content than those fedall other diets (P<0.05). The graded increase of phytase supplementation doses resulted on significant increases of whole-body P content (P<0.05). The graded increase of dietary phytase resulted on significant increases of whole-body P retention (P<0.05). Fish fed the DCP diet showed a significantly higher whole-body P retention than those fed the CTRL and PHY500 diets (P<0.05), although significantly lower than those fed the PHY1000 and PHY2000 diets (P<0.05). Fish fed the CTRL diet showed a significantly lower P digestibility than those fed all other diets (P<0.05). Significant enhancements (P<0.05) of P digestibility were associated to increasing dietary doses of the phytase, with dietary treatment PHY2000 having a significantly higher P digestibility than PHY1000 (P<0.05), and the later having a significantly higher P digestibility than PHY500 (P<0.05). Phosphorus digestibility of the DCP diet was significantly lower than that of diet PHY2000 (P<0.05) and significantly higher than that of PHY500 and CTRL diets (P<0.05).

**Table 24. Growth performance after 93 days of feeding (end of the trial).**

| | **CTRL** | **PHY500** | **PHY1000** | **PHY2000** | **DCP** | **P-value** |
|---|---|---|---|---|---|---|
| Survival, % | 99.2 ± 1.7 | 99.2 ± 1.7 | 99.2 ± 1.7 | 100.0 ± 0.0 | 100.0 ± 0.0 | 0.736 |
| IBW, g | 39.7 ± 0.1 | 39.5 ± 0.3 | 39.7 ± 0.2 | 39.2 ± 0.3 | 39.6 ± 0.4 | 0.187 |
| FBW, g | 144.1 ± 8.0 ^{a} | 162.6 ± 3.3 ^{b} | 175.3 ± 3.1^{c} | 183.7 ± 6.4^{c} | 174.6 ± 4.6^{c} | ***<0.001*** |
| SGR, %/d | 1.39 ± 0.06 ^{a} | 1.52 ± 0.03 ^{b} | 1.60 ± 0.02 ^{c} | 1.66 ± 0.03 ^{c} | 1.60 ± 0.03 ^{c} | ***<0.001*** |
| FCR | 1.45 ± 0.06 ^{c} | 1.36 ± 0.05^{b} | 1.31 ± 0.04 ^{ab} | 1.26 ± 0.05 ^{a} | 1.34 ± 0.02 ^{ab} | ***<0.001*** |
| FI, %ABW/d | 1.79 ± 0.06 | 1.78 ± 0.08 | 1.78 ± 0.04 | 1.75 ± 0.07 | 1.82 ± 0.02 | 0.672 |
| PER | 2.28 ± 0.10^{a} | 2.43 ± 0.10^{b} | 2.54 ± 0.08 ^{bc} | 2.63 ± 0.10^{c} | 2.47 ± 0.03^{b} | ***<0.001*** |

Values are means ± standard deviation (n=4).

Values within a row with different superscripts, denote a statistical difference (P<0.05).

**Table 25. Whole-body nutrient retention (% of intake)**

| | **CTRL** | **PHY500** | **PHY1000** | **PHY2000** | **DCP** | **P-value** |
|---|---|---|---|---|---|---|
| Protein, % | 36.7 ± 3.6 ^{a} | 40.6 ± 1.8 ^{b} | 42.7 ± 2.5 ^{b} | 44.0 ± 1.7^{b} | 41.6 ± 1.7^{b} | ***0.007*** |
| Fat, % | 77.2 ± 10.4 | 83.5 ± 6.1 | 87.1 ± 7.1 | 81.5 ± 2.0 | 80.1 ± 5.0 | 0.324 |
| Energy, % | 28.1 ± 2.2 ^{a} | 30.4 ± 1.7 ^{ab} | 32.2 ± 2.0 ^{b} | 31.6 ± 1.0^{b} | 30.5 ± 0.6 ^{ab} | ***0.025*** |
| Phosphorus, % | 26.2 ± 1.5 ^{a} | 35.5 ± 0.7 ^{b} | 45.2 ± 0.3 ^{d} | 50.7 ± 1.3 ^{e} | 38.7 ± 1.1 ^{c} | ***<0.001*** |
| Calcium, % | 40.9 ± 2.7 ^{b} | 43.5 ± 1.9 ^{b} | 48.1 ± 2.5 ^{c} | 48.8 ± 3.0^{c} | 33.8 ± 1.3 ^{a} | ***<0.000*** |

Values are average ± standard deviation (n=4).

Values within a row with different superscripts, denote a statistical difference (P<0.05).

**Table 26. Apparent digestibility coefficients (ADC, %) of nutrients.**

| | **CTRL** | **PHY500** | **PHY1000** | **PHY2000** | **DCP** | **P-value** |
|---|---|---|---|---|---|---|
| Dry matter, % | 68.3 ± 1.7 ^{a} | 71.5 ± 0.5 ^{b} | 71.3 ± 0.1 ^{b} | 72.9 ± 0.1 ^{c} | 71.4 ± 0.5 ^{b} | **<0.001** |
| Protein, % | 83.6 ± 0.9 ^{a} | 84.4 ± 0.4 ^{ab} | 84.2 ± 0.3 ^{ab} | 84.9 ± 0.3 ^{b} | 84.0 ± 0.3 ^{ab} | **0.020** |
| Phosphorus, % | 27.7 ± 2.2 ^{a} | 37.4 ± 1.6 ^{b} | 46.8 ± 1.5 ^{c} | 64.3 ± 1.2 ^{d} | 47.5 ± 3.0 ^{c} | **<0.001** |
| Phytate-P, % | 21.2 ± 3.8 ^{a} | 36.5 ± 3.6 ^{b} | 46.6 ± 1.8 ^{c} | 51.3 ± 1.5 ^{d} | 21.5 ± 1.5 ^{a} | **<0.001** |
| Calcium, % | 50.8 ± 3.7 ^{ab} | 52.4 ± 1.8 ^{ab} | 53.1 ± 2.0 ^{ab} | 54.9 ± 1.7 ^{b} | 48.0 ± 3.0 ^{a} | 0.019 |

Values are average ± standard deviation (n=4).

Values within a row with different superscripts, denote a statistical difference (P<0.05).

### Conclusion

The phytase at supplementation doses of 500, 1000 and 2000 FTY/kg feed, suc as 500 to 200 F Y T / k g is an effective strategy to enhance the growth rate, phosphorus digestibility, whole-body phosphorus retention and reduce FCR in Nile tilapia fed plant protein-rich diets.

### Example 13: Effect of graded supplemental levels of phytase on the growth performance, whole-body nutrient retention and nutrient digestibility in gilthead seabream (Sparus aurata) fed a plant-protein rich diet

The trial comprised five dietary treatments: a control diet (CTRL) with a total dietary phosphorus (P) level of 0.78%, in which a significant fraction of P was present in the form of phytate-bound P (0.4%). Three other diets, based on the CTRL formulation, were supplemented with phytase (var400) at graded doses (500, 1000 and 2000 FTY/kg feed) (diets PHY500, PHY1000, PHY2000). Phytase was applied post-extrusion by coating. A fifth diet (MCP), also based on the CTRL formulation,was supplemented with monocalcium phosphate, to a total P level of 1.1% and an available P level higher than the known requirements of the species. All diets were isonitrogenous, isolipidic and isoenergetic. Quadruplicate groups of 37 gilthead seabream, with a mean initial body weight of 55.3 ±4.1 g were fed one of the five experimental diets during 94 days, with a water temperature profile of 22.4 ± 0.2°C.

**Table 27. Formulation of experimental diets.**

| **Ingredients, %** | **CTRL** | **PHY500** | **PHY1000** | **PHY2000** | **MCP** |
|---|---|---|---|---|---|
| Fishmeal Supre Prime | 7.50 | 7.50 | 7.50 | 7.50 | 7.50 |
| Fish protein hydrolysate | 2.50 | 2.50 | 2.50 | 2.50 | 2.50 |
| Wheat gluten | 7.40 | 7.40 | 7.40 | 7.40 | 7.70 |
| Corn gluten meal | 19.00 | 19.00 | 19.00 | 19.00 | 19.00 |
| Soybean meal | 24.00 | 24.00 | 24.00 | 24.00 | 24.00 |
| Rapeseed meal | 8.50 | 8.50 | 8.50 | 8.50 | 8.50 |
| Sunflower meal | 14.00 | 14.00 | 14.00 | 14.00 | 14.00 |
| Wheat bran | 3.33 | 3.33 | 3.33 | 3.33 | 1.83 |
| Fish oil | 5.00 | 5.00 | 5.00 | 5.00 | 5.00 |
| Rapeseed oil | 7.50 | 7.50 | 7.50 | 7.50 | 7.50 |
| Vitamin and mineral premix | 1.00 | 1.00 | 1.00 | 1.00 | 1.00 |
| Antioxidant | 0.20 | 0.20 | 0.20 | 0.20 | 0.20 |
| Monocalcium phosphate | | | | | 1.20 |
| L-Tryptophan | 0.02 | 0.02 | 0.02 | 0.02 | 0.02 |
| DL-Methionine | 0.03 | 0.03 | 0.03 | 0.03 | 0.03 |
| Yttrium oxide | 0.02 | 0.02 | 0.02 | 0.02 | 0.02 |
| **Phytase (FTY/kg)** | | **500** | **1000** | **2000** | |

### Result

At the end of the trial, the final body weight (FBW) ranged between 142.8 and 157.5 grams. Fish with the highest weight gain showed a 2.8-fold increase of their initial body weight (IBW). The specific growthrate (SGR) varied between 1.01 and 1.11 %/day. At day 94, all supplemented diets resulted on a significantly higher FBW and SGR than those fed the CTRL diet (P<0.05). Fish fed the PHY500, PHY1000, PHY2000 and MCP diets showed a significantly lower FCR than those fed the CTRL diet (P<0.05). The CTRL treatment was consistently associated to significantly lowest values among the various treatments for whole-body phosphorus retention and apparent digestibility of phosphorus. The higher phytase supplementation doses (1000 and 2000 FTY/kg) led to a significant whole-body P retention in comparison to both CTRL and MCP treatments (P<0.05). Significant enhancements of total P and phytate-P digestibility were associated to increasing dietary doses of phytase, with dietary treatment PHY2000 having a significantly higher P digestibility than PHY500 (P<0.05). Additionally, theincrease of phytase supplementation doses (0, 500, 1000 and 2000 FTY/kg) was also positively associated to an increase of whole-body phosphorus retention.

**Table 28. Growth performance after 94 days of feeding.**

| | **CTRL** | **PHY500** | **PHY1000** | **PHY2000** | **MCP** | **P-value** |
|---|---|---|---|---|---|---|
| Survival, % | 100.0 ± 0.0 | 100.0 ± 0.0 | 100.0 ± 0.0 | 100.0 ± 0.0 | 100.0 ± 0.0 | - |
| IBW, g | 55.4 ± 0.6 | 55.3 ± 0.3 | 55.2 ± 0.5 | 55.6 ± 0.2 | 55.4 ± 0.4 | 0.791 |
| FBW, g | 142.8 ± 2.4 ^{a} | 148.4 ± 0.7 ^{b} | 156.2 ± 1.8 ^{c} | 156.7 ± 1.2 ^{c} | 157.5 ± 1.5 ^{c} | **<0.001** |
| SGR, %/d | 1.01 ± 0.01 ^{a} | 1.05 ± 0.01 ^{b} | 1.11 ± 0.01 ^{c} | 1.10 ± 0.01 ^{c} | 1.11 ± 0.01 ^{c} | **<0.001** |
| FCR | 1.29 ± 0.04 ^{c} | 1.21 ± 0.03 ^{b} | 1.12 ± 0.03 ^{a} | 1.14 ± 0.03 ^{ab} | 1.16 ± 0.05 ^{ab} | **<0.001** |
| FI, %ABW/d | 1.21 ± 0.03 ^{b} | 1.17 ± 0.03 ^{ab} | 1.14 ± 0.02 ^{a} | 1.15 ± 0.02 ^{ab} | 1.18 ± 0.04 ^{ab} | **0.048** |
| PER | 1.74 ± 0.05 ^{a} | 1.86 ± 0.05 ^{b} | 2.01 ± 0.06 ^{c} | 1.97 ± 0.05 ^{bc} | 1.95 ± 0.09 ^{bc} | **<0.001** |

Values are means ± standard deviation (n=4).

Values within a row with different superscripts, denote a statistical difference (P<0.05).

**Table 29. Whole-body nutrient and energy retention (% of intake).**

| | **CTRL** | **PHY500** | **PHY1000** | **PHY2000** | **MCP** | **P-value** |
|---|---|---|---|---|---|---|
| Protein, % | 26.5 ± 1.9 ^{a} | 28.2 ± 1.5^{ab} | 30.7 ± 0.8^{b} | 30.6 ± 2.3^{b} | 28.7 ± 1.9 ^{ab} | ***0.021*** |
| Fat, % | 60.4 ± 4.5 | 63.1 ± 7.5 | 63.7 ± 3.4 | 69.5 ± 6.2 | 63.6 ± 3.3 | 0.210 |
| Energy, % | 29.9 ± 2.5 ^{a} | 33.3 ± 2.6 ^{abc} | 35.2 ± 1.2 ^{bc} | 35.9 ± 2.4 ^{c} | 31.2 ± 1.9 ^{ab} | ***0.006*** |
| Phosphorus, % | 45.9 ± 2.6 ^{a} | 53.5 ± 2.9 ^{a} | 62.6 ± 5.3 ^{b} | 59.9 ± 3.6 ^{b} | 48.0 ± 5.7 ^{a} | ***<0.001*** |
| Calcium, % | 44.7 ± 2.0 ^{b} | 52.9 ± 4.4^{c} | 59.9 ± 3.4^{d} | 60.7 ± 5.0 ^{d} | 37.2 ± 1.1 ^{a} | ***<0.001*** |

Values are average ± standard deviation (n=4).

Values within a row with different superscripts, denote a statistical difference (P<0.05).

**Table 30. Apparent digestibility coefficients (ADC, %) of nutrients.**

| | **CTRL** | **PHY500** | **PHY1000** | **PHY2000** | **MCP** | **P-value** |
|---|---|---|---|---|---|---|
| Dry matter, % | 63.4 ± 1.0^{a} | 66.3 ± 0.8 ^{b} | 66.5 ± 2.2 ^{b} | 65.1 ± 1.7 ^{ab} | 62.5 ± 0.8 ^{a} | ***0.004*** |
| Protein, % | 86.3 ± 0.9 | 87.5 ± 0.7 | 88.0 ± 1.3 | 87.0 ± 1.1 | 86.3 ± 0.8 | 0.117 |
| Phosphorus, % | 34.5 ± 1.1 ^{a} | 49.4 ± 1.5 ^{b} | 57.2 ± 4.3 ^{c} | 60.4 ± 3.4 ^{cd} | 62.6 ± 1.2 ^{d} | ***<0.001*** |
| Phytate-P, % | 32.4 ± 1.0 ^{a} | 39.1 ± 3.2 ^{b} | 39.8 ± 3.8 ^{b} | 45.7 ± 4.4 ^{c} | 30.8 ± 2.3 | ***<0.001*** |
| Calcium, % | 35.0 ± 1.7 ^{b} | 36.1 ± 4.7 ^{b} | 42.7 ± 5.0 ^{c} | 43.1 ± 2.0 ^{c} | 29.9 ± 2.3 | ***<0.001*** |

Values are average ± standard deviation (n=4).

Values within a row with different superscripts, denote a statistical difference (P<0.05).

### Conclusion

The phytase at supplementation doses of 500, 1000 and 2000 FTY/kg feed is an effective strategy to enhance the growth rate and phosphorus digestibility. Phytase at supplementation dosesof 1000 and 2000 FTY/kg feed significantly increase whole-body phosphorus retention in gilthead seabream fed plant protein-rich diets.

### Example 14: Efficacy of a phytase with a protease in broilers

The effect on the growth performance of broilers using feed with 1,500 FYT/kg of phytase (var400) and 30,000 U/kg of protease (Ronozyme^{®}ProAct 360) was investigated. Male broilers (Ross 308) were fed one of the four diets as described in Table 31 from hatch to day 28 post-hatch. Each treatment had 8 replicate cages of 6 birds per cage. The basal diets used in the trial were diet with standard Ca, standard crude protein diet or a low Ca, high crude protein diet (Table 32).

**Table 31. Treatment designation**

| **Treatment** | **Basal** | **Protease, U/kg¹** | **Phytase, U/kg²** |
|---|---|---|---|
| | **Diet** | | |
| 1 | Standard Ca and CP | 0 | 500 |
| 2 | | 30,000 | 1,500 |
| 3 | Low Ca and high CP | 0 | 500 |
| 4 | | 30,000 | 1,500 |

| | | | |
|---|---|---|---|
| ¹ Protease was included in the diets over the top (e.g. no further amino acid or protein matrix was applied). ² 500 U/kg of phytase was included in the diets to provide 0.15% avP, 0.17% Ca and 0.018% Na. No extra nutrients were removed when the additional 1,000 U/kg was included. | | | |

**Table 32. Starter and grower experimental diets**

| **Ingredient** | **Treatment 1, 2 (fed from day 0-14)** | **Treatment 3, 4 (fed from day 0-14)** | **Treatment 1, 2 (fed from day 15-28)** | **Treatment 3, 4 (fed from day 15-28)** |
|---|---|---|---|---|
| Corn | 59.10% | 56.19% | 61.52% | 58.61% |
| Soybean meal | 34.98% | 38.43% | 31.79% | 35.26% |
| Soy oil | 1.86% | 1.93% | 3.03% | 3.10% |
| Salt | 0.13% | 0.12% | 0.13% | 0.12% |
| Limestone | 0.77% | 0.12% | 0.66% | |
| Dicalcium phosphate | 1.53% | 1.48% | 1.33% | 1.28% |
| Lysine HCl | 0.23% | 0.24% | 0.17% | 0.18% |
| DL-Methionine | 0.32% | 0.36% | 0.26% | 0.30% |
| Threonine | 0.09% | 0.10% | 0.05% | 0.060% |
| Valine | 0.038% | 0.059% | | 0.013% |
| Vitamin and mineral premix | 1.00% | 1.00% | 1.00% | 1.00% |
| TiO Marker | | | 0.10% | 0.10% |
| TOTAL | 100.0% | 100.0% | 100.0% | 100.0% |

| **Nutrients** | | | | |
|---|---|---|---|---|
| Crude protein % | 22.50 | 24.00 | 21.00 | 22.50 |
| Poult ME kcal/kg | 3000.00 | 3000.00 | 3100.00 | 3100.00 |
| DM, % | 87.07 | 87.04 | 87.04 | 87.00 |
| Calcium % | 1.00 | 0.75 | 0.90 | 0.65 |
| Phosphorus % | 0.68 | 0.69 | 0.63 | 0.64 |
| Available P % | 0.35 | 0.35 | 0.31 | 0.31 |
| Phytate P % | 0.26 | 0.27 | 0.26 | 0.26 |
| Crude Fibre % | 2.23 | 2.28 | 2.18 | 2.23 |
| D Met+Cys % | 0.95 | 1.02 | 0.85 | 0.93 |
| D Lys % | 1.28 | 1.38 | 1.15 | 1.25 |
| D Thr % | 0.83 | 0.90 | 0.75 | 0.81 |
| Available P % | 0.35 | 0.35 | 0.31 | 0.31 |
| Na % | 0.18 | 0.18 | 0.18 | 0.18 |
| Cl % | 0.36 | 0.36 | 0.35 | 0.35 |

### Results

**Table 33. Growth performance of broilers fed standard Ca and crude protein or low Ca and high crude protein diets without or with protease and an extra 1,000 U/kg of phytase from hatch to day 28 post-hatch**

| **Dietary Ca** | **Dietary crude protein** | **Protease, U/kg** | **Phytase, U/kg** | **Feed intake, g/bird** | **BW gain, g/bird** | **FCR, g:g** |
|---|---|---|---|---|---|---|
| **Standard Ca** | **Standard CP** | **0** | **500** | 2,376 | 1,683 | 1.427 |
| | | **30,000** | **1,500** | 2,341 | 1,722 | 1.362 |
| **Percent delta** | | | | -1.5% | +2.3% | -4.6% |
| **Low Ca** | **High CP** | **0** | **500** | 2,349 | 1,795 | 1.318 |
| | | **30,000** | **1,500** | 2,370 | 1,861 | 1.281 |
| **Percent delta** | | | | +0.9% | +3.7% | -2.8% |

**Table 34. Apparent nutrient digestibility of broilers fed standard Ca and crude protein or low Ca and high crude protein diets without or with protease and an extra 1,000 U/kg of phytase from hatch to day 28 post-hatch**

| **Dietary Ca** | **Dietary crude protein** | **Protease, U/kg** | **Phytase, U/kg** | **Ileal protein digestibility, %** | **Fecal protein retention, %** | **Fecal energy retention, %** |
|---|---|---|---|---|---|---|
| **Standard Ca** | **Standard CP** | **0** | **500** | **82.5** | **63.4** | **77.0** |
| | | **30,000** | **1,500** | **83.8** | **66.2** | **78.0** |
| **Percent delta** | | | | **+1.6%** | **+4.4%** | **+1.3%** |
| **Low Ca** | **High CP** | **0** | **500** | **84.8** | **66.5** | **77.8** |
| | | **30,000** | **1,500** | **85.4** | **67.1** | **78.3** |
| **Percent delta** | | | | **+0.7%** | **+0.9%** | **+0.6%** |

### Conclusion

Feeding broilers protease in combination with an extra 1,000 FYT/kg of phytase increased protein digestibility by 0.7 to 1.6%, protein retention by 0.9 to 4.4% and energy retention by 0.6 to 1.3%. This resulted in an improvement in body weight gain (BWG) by 2.3 or 3.7% and feed conversion ratio by 4.6 or 2.8%.

## Claims

1. A phytase variant which has at least 70%, at least 75%, at least 80%, at least 85%, at least 90%, at least 95%, at least 97%, at least 98%, or at least 99% identity to SEQ ID NO: 2 and which comprises the alterations N31C/G52C/A99C/K141C/T177C/V199C/N203L as compared to SEQ ID NO: 2 and further comprises a substitution in one or more position(s) selected from the following: 30, 36, 43, 46, 57, 60, 64, 73, 79, 119, 121, 123, 130, 134, 138, 151, 155, 161, 162, 168, 176, 180, 184, 190, 207, 224, 230, 243, 273, 286, 336, 340, 358 and 375 using SEQ ID NO: 2 for numbering.

2. The variant of claim 1, comprising substitutions in the positions: 57, 73, 121, 134, 155, 207 and 273.

3. The variant of claim 2, further comprising substitutions in the positions: 36, 60, 64, 73, 119, 130, 138,161, 162, 168, 176, 180, 184, 190, 224, 230, 243, 336 and 340.

4. The variant according to any of claims 1-3, where the substitutions are selected among: 30Q, 36A, 43C, 46C, 57Y, 60H, 64Q, 73P, 79Q, 119P, 121P, 123C, 130T,C, 134Q, 138A, 151S, 155F, 161T, 162A, 168R 176P, 180N, 184Q, 190T, 207T, 224Q, 230E, 243N, 273L, 286S, 336R, 340L,P, 358Q and 375K.

5. The variant according to any of the claim 1-4, comprising the substitutions 31C/52C/57Y/ 73P/99C/121 P/134Q/141C/155F/177C/199C/203L/207T/273L.

6. The variant according to claim 5, selected among variants comprising the substitutions selected from the group consisting of:
31C/ 52C/ 57Y/ 73P/ 99C/ 121P/ 134Q/ 141C/ 155F/ 177C/ 199C/ 203L/ 207T/ 273L;
31C/ 36A/ 52C/ 57Y/ 60H/ 64Q/ 73P/ 99C/ 119S/ 121P/ 130T/ 134Q/ 138A/ 141C/ 155F/ 161T/ 162A/ 176P/ 177C/ 180N/ 184Q/ 190T/ 199C/ 203L/ 207T/ 224Q/ 230E/ 243N/ 273L/ 336R/ 340L;
31C/ 36A/ 43C/ 46C/ 52C/ 57Y/ 60H/ 64Q/ 73P/ 99C/ 119S/ 121P/ 130T/ 134Q/ 138A/ 141C/ 155F/ 161T/ 162A/ 176P/ 177C/ 180N/ 184Q/ 190T/ 199C/ 203L/ 207T/ 224Q/ 230E/ 243N/ 273L/ 286S/ 336R/ 340L;
31C/ 36A/ 52C/ 57Y/ 60H/ 64Q/ 73P/ 99C/ 119S/ 121P/ 123C/ 130C/ 134Q/ 138A/ 141C/ 151S/ 155F/ 161T/ 162A/ 176P/ 177C/ 180N/ 184Q/ 190T/ 199C/ 203L/ 207T/ 224Q/ 230E/ 243N/ 273L/ 336R/ 340L; and
31C/ 36A/ 43C/ 46C/ 52C/ 57Y/ 60H/ 64Q/ 73P/ 99C/ 119S/ 121P/ 123C/ 130C/ 134Q/ 138A/ 141C/ 155F/ 161T/ 162A/ 176P/ 177C/ 180N/ 184Q/ 190T/ 199C/ 203L/ 207T/ 224Q/ 230E/ 243N/ 273L/ 286S/ 336R/ 340L.

7. The variant of claim 6, having the amino acid sequence of SEQ ID NO: 2 with the substitutions selected from the group consisting of:
N31C/ G52C/ E57Y/ N73P/ A99C/ N121P/ S134Q/ K141C/ Y155F/ T177C/ V199C/ N203L/ P207T/ M273L;
N31C/ Q36A/ G52C/ E57Y/ Q60H/ L64Q/ N73P/ A99C/ E119S/ N121P/ M130T/ S134Q/ L138A/ K141C/ Y155F/ S161T/ S162A/ E176P/ T177C/ T180N/ S184Q/ P190T/ V199C/ N203L/ P207T/ E224Q/ Q230E/ R243N/ M273L/ K336R/ T340L;
N31C/ Q36A/ P43C/ W46C/ G52C/ E57Y/ Q60H/ L64Q/ N73P/ A99C/ E119S/ N121P/ M130T/ S134Q/ L138A/ K141C/ Y155F/ S161T/ S162A/ E176P/ T177C/ T180N/ S184Q/ P190T/ V199C/ N203L/ P207T/ E224Q/ Q230E/ R243N/ M273L/ N286S/ K336R/ T340L;
N31C/ Q36A/ G52C/ E57Y/ Q60H/ L64Q/ N73P/ A99C/ E119S/ N121P/ P123C/ M130C/ S134Q/ L138A/ K141C/ N151S/ Y155F/ S161T/ S162A/ E176P/ T177C/ T180N/ S184Q/ P190T/ V199C/ N203L/ P207T/ E224Q/ Q230E/ R243N/ M273L/ K336R/ T340L; or
N31C/ Q36A/ P43C/ W46C/ G52C/ E57Y/ Q60H/ L64Q/ N73P/ A99C/ E119S/ N121P/ P123C/ M130C/ S134Q/ L138A/ K141C/ Y155F/ S161T/ S162A/ E176P/ T177C/ T180N/ S184Q/ P190T/ V199C/ N203L/ P207T/ E224Q/ Q230E/ R243N/ M273L/ N286S/ K336R/ T340L.

8. The variant of any of claims 1-7, which has an improved thermostability in comparison with the phytase having the amino acid sequence of SEQ ID NO: 2 and the substitutions N31C/ G52C/ A99C/ K141C/ T177C/ V199C/ N203L.

9. A polynucleotide encoding the variant of any of claims 1-8.

10. A nucleic acid construct or an expression vector comprising the polynucleotide of claim 9.

11. A host cell comprising the polynucleotide of claim 9.

12. A method of producing a phytase variant according to any of claims 1-8, comprising:
cultivating the host cell of claim 11 under conditions suitable for expression of the variant; and recovering the variant.

13. A plant comprising the phytase variant of any of claims 1-8 and/or the polynucleotide of claim 9.

14. A composition comprising at least one phytase variant according to any of claims 1-8.

15. The composition of claim 14 further comprising
at least one fat soluble vitamin;
at least one water soluble vitamin; and/or
at least one trace mineral.

16. The composition of claim 14 or 15, further comprising at least one enzyme selected from the following group of enzymes: amylase, phytase, phosphatase, xylanase, galactanase, alpha-galactosidase, protease, phospholipase and/or beta-glucanase.

17. The composition of any of claims 14-16, which is an animal feed additive.

18. An animal feed composition having a crude protein content of 50 to 800 g/kg and comprising the phytase variant of any of claims 1-8, or the composition of any of claims 14-17.

19. A method of improving the nutritional value of an animal feed, wherein the phytase variant of any of claims 1-8, or the composition of any of claims 14-17 is added to the feed.

20. A method for the treatment of vegetable proteins, comprising the step of adding the phytase variant of any of claims 1-8, or the composition of any of claims 14-17 to at least one vegetable protein or protein source.

21. A method for increasing weight gain and/or improving Feed Conversion Ratio of an animal, the method comprising the step of applying to the animal a feed with an efficient amount of the phytase variant of any of claims 1-8 or the composition of any of claims 14-17.

22. Use of the phytase variant of any of claims 1-8, or the composition of any of claims 14-17 in animal feed; in the preparation of animal feed; for improving the nutritional value of animal feed; for reducing phytate levels in animal manure; for the treatment of vegetable proteins; for liberating phosphorous from a phytate substrate; for increasing weight gain, improving specific growth rate and/or improving Feed Conversion Ratio of an animal; or for improving nutrient retention, and/or nutrient digestibility in an animal.

## Patentansprüche

1. Phytase-Variante, die mindestens 70 %, mindestens 75 %, mindestens 80 %, mindestens 85 %, mindestens 90 %, mindestens 95 %, mindestens 97 %, mindestens 98 % oder mindestens 99 % Identität zu SEQ ID NO: 2 aufweist und die die Veränderungen N31C/G52C/A99C/K141C/T177C/V199C/N203L im Vergleich zu SEQ ID NO: 2 umfasst und ferner eine Substitution in einer oder mehreren Position(en) umfasst, die aus den Folgenden ausgewählt sind: 30, 36, 43, 46, 57, 60, 64, 73, 79, 119, 121, 123, 130, 134, 138, 151, 155, 161, 162, 168, 176, 180, 184, 190, 207, 224, 230, 243, 273, 286, 336, 340, 358 und 375 unter Verwendung von SEQ ID NO: 2 zur Nummerierung.

2. Variante nach Anspruch 1, umfassend Substitutionen in den Positionen: 57, 73, 121, 134, 155, 207 und 273.

3. Variante nach Anspruch 2, ferner umfassend Substitutionen in den Positionen: 36, 60, 64, 73, 119, 130, 138,161, 162, 168, 176, 180, 184, 190, 224, 230, 243, 336 und 340.

4. Variante nach einem der Ansprüche 1-3, wobei die Substitutionen ausgewählt sind aus: 30Q, 36A, 43C, 46C, 57Y, 60H, 64Q, 73P, 79Q, 119P, 121P, 123C, 130T,C, 134Q, 138A, 151S, 155F, 161T, 162A, 168R 176P, 180N, 184Q, 190T, 207T, 224Q, 230E, 243N, 273L, 286S, 336R, 340L,P, 358Q und 375K.

5. Variante nach einem der Ansprüche 1-4, umfassend die Substitutionen 31C/52C/57Y/73P/99C/121P/134Q/141C/155F/177C/199C/203L/ 207T/273L.

6. Variante nach Anspruch 5, ausgewählt aus Varianten, umfassend die Substitutionen, die ausgewählt sind aus der Gruppe bestehend aus:
31C/ 52C/ 57Y/ 73P/ 99C/ 121P/ 134Q/ 141C/ 155F/ 177C/ 199C/ 203L/ 207T/ 273L;
31C/ 36A/ 52C/ 57Y/ 60H/ 64Q/ 73P/ 99C/ 119S/ 121P/ 130T/ 134Q/ 138A/ 141C/ 155F/ 161T/ 162A/ 176P/ 177C/ 180N/ 184Q/ 190T/ 199C/ 203L/ 207T/ 224Q/ 230E/ 243N/ 273L/ 336R/ 340L;
31C/ 36A/ 43C/ 46C/ 52C/ 57Y/ 60H/ 64Q/ 73P/ 99C/ 119S/ 121P/ 130T/ 134Q/ 138A/ 141C/ 155F/ 161T/ 162A/ 176P/ 177C/ 180N/ 184Q/ 190T/ 199C/ 203L/ 207T/ 224Q/ 230E/ 243N/ 273L/ 286S/ 336R/ 340L;
31C/ 36A/ 52C/ 57Y/ 60H/ 64Q/ 73P/ 99C/ 119S/ 121P/ 123C/ 130C/ 134Q/ 138A/ 141C/ 151S/ 155F/ 161T/ 162A/ 176P/ 177C/ 180N/ 184Q/ 190T/ 199C/ 203L/ 207T/ 224Q/ 230E/ 243N/ 273L/ 336R/ 340L; und
31C/ 36A/ 43C/ 46C/ 52C/ 57Y/ 60H/ 64Q/ 73P/ 99C/ 119S/ 121P/ 123C/ 130C/ 134Q/ 138A/ 141C/ 155F/ 161T/ 162A/ 176P/ 177C/ 180N/ 184Q/ 190T/ 199C/ 203L/ 207T/ 224Q/ 230E/ 243N/ 273L/ 286S/ 336R/ 340L.

7. Variante nach Anspruch 6, aufweisend die Aminosäuresequenz von SEQ ID NO: 2 mit den Substitutionen, die ausgewählt sind aus der Gruppe bestehend aus:
N31C/ G52C/ E57Y/ N73P/ A99C/ N121P/ S134Q/ K141C/ Y155F/ T177C/ V199C/ N203L/ P207T/ M273L;
N31C/ Q36A/ G52C/ E57Y/ Q60H/ L64Q/ N73P/ A99C/ E119S/ N121P/ M130T/ S134Q/ L138A/ K141C/ Y155F/ S161T/ S162A/ E176P/ T177C/ T180N/ S184Q/ P190T/ V199C/ N203L/ P207T/ E224Q/ Q230E/ R243N/ M273L/ K336R/ T340L;
N31C/ Q36A/ P43C/ W46C/ G52C/ E57Y/ Q60H/ L64Q/ N73P/ A99C/ E119S/ N121P/ M130T/ S134Q/ L138A/ K141C/ Y155F/ S161T/ S162A/ E176P/ T177C/ T180N/ S184Q/ P190T/ V199C/ N203L/ P207T/ E224Q/ Q230E/ R243N/ M273L/ N286S/ K336R/ T340L;
N31C/ Q36A/ G52C/ E57Y/ Q60H/ L64Q/ N73P/ A99C/ E119S/ N121P/ P123C/ M130C/ S134Q/ L138A/ K141C/ N151S/ Y155F/ S161T/ S162A/ E176P/ T177C/ T180N/ S184Q/ P190T/ V199C/ N203L/ P207T/ E224Q/ Q230E/ R243N/ M273L/ K336R/ T340L; oder
N31C/ Q36A/ P43C/ W46C/ G52C/ E57Y/ Q60H/ L64Q/ N73P/ A99C/ E119S/ N121P/ P123C/ M130C/ S134Q/ L138A/ K141C/ Y155F/ S161T/ S162A/ E176P/ T177C/ T180N/ S184Q/ P190T/ V199C/ N203L/ P207T/ E224Q/ Q230E/ R243N/ M273L/ N286S/ K336R/ T340L.

8. Variante nach einem der Ansprüche 1-7, die eine verbesserte Thermostabilität im Vergleich zu der Phytase mit der Aminosäuresequenz von SEQ ID NO: 2 und den Substitutionen N31C/ G52C/ A99C/ K141C/ T177C/ V199C/ N203L aufweist.

9. Polynukleotid, codierend die Variante nach einem der Ansprüche 1-8.

10. Nukleinsäurekonstrukt oder Expressionsvektor, umfassend das Polynukleotid nach Anspruch 9.

11. Wirtszelle, umfassend das Polynukleotid nach Anspruch 9.

12. Verfahren zum Herstellen einer Phytase-Variante nach einem der Ansprüche 1-8, umfassend:
Kultivieren der Wirtszelle nach Anspruch 11 unter Bedingungen, die für die Expression der Variante geeignet sind; und Gewinnen der Variante.

13. Pflanze, umfassend die Phytase-Variante nach einem der Ansprüche 1-8 und/oder das Polynukleotid nach Anspruch 9.

14. Zusammensetzung, umfassend mindestens eine Phytase-Variante nach einem der Ansprüche 1-8.

15. Zusammensetzung nach Anspruch 14, ferner umfassend
mindestens ein fettlösliches Vitamin;
mindestens ein wasserlösliches Vitamin; und/oder
mindestens ein Spurenmineral.

16. Zusammensetzung nach Anspruch 14 oder 15, ferner umfassend mindestens ein Enzym, ausgewählt aus der folgenden Gruppe von Enzymen: Amylase, Phytase, Phosphatase, Xylanase, Galactanase, Alpha-Galactosidase, Protease, Phospholipase und/oder Beta-Glucanase.

17. Zusammensetzung nach einem der Ansprüche 14-16, bei der es sich um ein Tierfutteradditiv handelt.

18. Tierfutterzusammensetzung, aufweisend einen Rohproteingehalt von 50 bis 800 g/kg und umfassend die Phytase-Variante nach einem der Ansprüche 1-8 oder die Zusammensetzung nach einem der Ansprüche 14-17.

19. Verfahren zur Verbesserung des Nährwerts eines Tierfutters, wobei die Phytase-Variante nach einem der Ansprüche 1-8 oder die Zusammensetzung nach einem der Ansprüche 14-17 dem Futter hinzugefügt wird.

20. Verfahren zur Behandlung von pflanzlichen Proteinen, umfassend den Schritt des Hinzufügens der Phytase-Variante nach einem der Ansprüche 1-8 oder der Zusammensetzung nach einem der Ansprüche 14-17 zu mindestens einem pflanzlichen Protein oder einer pflanzlichen Proteinquelle.

21. Verfahren zum Erhöhen der Gewichtszunahme und/oder Verbessern des Futterumwandlungsverhältnisses (Feed Conversion Ratio) eines Tieres, wobei das Verfahren den Schritt des Anwendens eines Futters mit einer wirksamen Menge der Phytase-Variante nach einem der Ansprüche 1-8 oder der Zusammensetzung nach einem der Ansprüche 14-17 auf das Tier umfasst.

22. Verwendung der Phytase-Variante nach einem der Ansprüche 1-8 oder der Zusammensetzung nach einem der Ansprüche 14-17 in Tierfutter; bei der Herstellung von Tierfutter; zur Verbesserung des Nährwerts von Tierfutter; zur Verringerung des Phytatspiegels in Tiermist; zur Behandlung von pflanzlichen Proteinen; zur Freisetzung von Phosphor aus einem Phytatsubstrat; zur Erhöhung der Gewichtszunahme, zur Verbesserung der spezifischen Wachstumsrate und/oder zur Verbesserung des Futterumwandlungsverhältnisses eines Tieres; oder zur Verbesserung der Nährstoffretention und/oder Nährstoffverdaulichkeit bei einem Tier.

## Revendications

1. Variant de phytase qui a au moins 70 %, au moins 75 %, au moins 80 %, au moins 85 %, au moins 90 %, au moins 95 %, au moins 97 %, au moins 98 %, ou au moins 99 % d'identité avec SEQ ID NO: 2 et qui comprend les altérations N31C/G52C/A99C/K141C/T177C/V199C/N203L comparé à SEQ ID NO: 2 et comprend en outre une substitution dans une ou plusieurs position(s) sélectionnée(s) parmi les suivantes : 30, 36, 43, 46, 57, 60, 64, 73, 79, 119, 121, 123, 130, 134, 138, 151, 155, 161, 162, 168, 176, 180, 184, 190, 207, 224, 230, 243, 273, 286, 336, 340, 358 et 375 en utilisant SEQ ID NO: 2 pour la numérotation.

2. Variant selon la revendication 1, comprenant des substitutions dans les positions : 57, 73, 121, 134, 155, 207 et 273.

3. Variant selon la revendication 2, comprenant en outre des substitutions dans les positions : 36, 60, 64, 73, 119, 130, 138, 161, 162, 168, 176, 180, 184, 190, 224, 230, 243, 336 et 340.

4. Variant selon l'une quelconque des revendications 1 à 3, où les substitutions sont sélectionnées parmi : 30Q, 36A, 43C, 46C, 57Y, 60H, 64Q, 73P, 79Q, 119P, 121P, 123C, 130T,C, 134Q, 138A, 151S, 155F, 161T, 162A, 168R 176P, 180N, 184Q, 190T, 207T, 224Q, 230E, 243N, 273L, 286S, 336R, 340L,P, 358Q et 375K.

5. Variant selon l'une quelconque des revendications 1 à 4, comprenant les substitutions 31C/52C/57Y/73P/99C/121P/134Q/141C/155F/177C/199C/203L/ 207T/273L.

6. Variant selon la revendication 5, sélectionné parmi des variants comprenant les substitutions sélectionnées dans le groupe constitué de :
31C/ 52C/ 57Y/ 73P/ 99C/ 121P/ 134Q/ 141C/ 155F/ 177C/ 199C/ 203L/ 207T/ 273L ;
31C/ 36A/ 52C/ 57Y/ 60H/ 64Q/ 73P/ 99C/ 119S/ 121P/ 130T/ 134Q/ 138A/ 141C/ 155F/ 161T/ 162A/ 176P/ 177C/ 180N/ 184Q/ 190T/ 199C/ 203L/ 207T/ 224Q/ 230E/ 243N/ 273L/ 336R/ 340L ;
31C/ 36A/ 43C/ 46C/ 52C/ 57Y/ 60H/ 64Q/ 73P/ 99C/ 119S/ 121P/ 130T/ 134Q/ 138A/ 141C/ 155F/ 161T/ 162A/ 176P/ 177C/ 180N/ 184Q/ 190T/ 199C/ 203L/ 207T/ 224Q/ 230E/ 243N/ 273L/ 286S/ 336R/ 340L ;
31C/ 36A/ 52C/ 57Y/ 60H/ 64Q/ 73P/ 99C/ 119S/ 121P/ 123C/ 130C/ 134Q/ 138A/ 141C/ 151S/ 155F/ 161T/ 162A/ 176P/ 177C/ 180N/ 184Q/ 190T/ 199C/ 203L/ 207T/ 224Q/ 230E/ 243N/ 273L/ 336R/ 340L ; et
31C/ 36A/ 43C/ 46C/ 52C/ 57Y/ 60H/ 64Q/ 73P/ 99C/ 119S/ 121P/ 123C/ 130C/ 134Q/ 138A/ 141C/ 155F/ 161T/ 162A/ 176P/ 177C/ 180N/ 184Q/ 190T/ 199C/ 203L/ 207T/ 224Q/ 230E/ 243N/ 273L/ 286S/ 336R/ 340L.

7. Variant selon la revendication 6, ayant la séquence d'acides aminés de SEQ ID NO: 2 avec les substitutions sélectionnées dans le groupe constitué de :
N31C/ G52C/ E57Y/ N73P/ A99C/ N121P/ S134Q/ K141C/ Y155F/ T177C/ V199C/ N203L/ P207T/ M273L ;
N31C/ Q36A/ G52C/ E57Y/ Q60H/ L64Q/ N73P/ A99C/ E119S/ N121P/ M130T/ S134Q/ L138A/ K141C/ Y155F/ S161T/ S162A/ E176P/ T177C/ T180N/ S184Q/ P190T/ V199C/ N203L/ P207T/ E224Q/ Q230E/ R243N/ M273L/ K336R/ T340L ;
N31C/ Q36A/ P43C/ W46C/ G52C/ E57Y/ Q60H/ L64Q/ N73P/ A99C/ E119S/ N121P/ M130T/ S134Q/ L138A/ K141C/ Y155F/ S161T/ S162A/ E176P/ T177C/ T180N/ S184Q/ P190T/ V199C/ N203L/ P207T/ E224Q/ Q230E/ R243N/ M273L/ N286S/ K336R/ T340L ;
N31C/ Q36A/ G52C/ E57Y/ Q60H/ L64Q/ N73P/ A99C/ E119S/ N121P/ P123C/ M130C/ S134Q/ L138A/ K141C/ N151S/ Y155F/ S161T/ S162A/ E176P/ T177C/ T180N/ S184Q/ P190T/ V199C/ N203L/ P207T/ E224Q/ Q230E/ R243N/ M273L/ K336R/ T340L ; ou
N31C/ Q36A/ P43C/ W46C/ G52C/ E57Y/ Q60H/ L64Q/ N73P/ A99C/ E119S/ N121P/ P123C/ M130C/ S134Q/ L138A/ K141C/ Y155F/ S161T/ S162A/ E176P/ T177C/ T180N/ S184Q/ P190T/ V199C/ N203L/ P207T/ E224Q/ Q230E/ R243N/ M273L/ N286S/ K336R/ T340L.

8. Variant selon l'une quelconque des revendications 1 à 7, qui a une thermostabilité améliorée par comparaison avec la phytase ayant la séquence d'acides aminés de SEQ ID NO: 2 et les substitutions N31C/ G52C/ A99C/ K141C/ T177C/ V199C/ N203L.

9. Polynucléotide codant le variant selon l'une quelconque des revendications 1 à 8.

10. Construction d'acides nucléiques ou vecteur d'expression comprenant le polynucléotide selon la revendication 9.

11. Cellule hôte comprenant le polynucléotide selon la revendication 9.

12. Procédé de production d'un variant de phytase selon l'une quelconque des revendications 1 à 8, comprenant :
la culture de la cellule hôte selon la revendication 11 dans des conditions appropriées pour l'expression du variant ; et la récupération du variant.

13. Plante comprenant le variant de phytase selon l'une quelconque des revendications 1 à 8 et/ou le polynucléotide selon la revendication 9.

14. Composition comprenant au moins un variant de phytase selon l'une quelconque des revendications 1 à 8.

15. Composition selon la revendication 14 comprenant en outre
au moins une vitamine liposoluble ;
au moins une vitamine hydrosoluble ; et/ou
au moins un oligoélément.

16. Composition selon la revendication 14 ou la revendication 15, comprenant en outre au moins une enzyme sélectionnée dans le groupe d'enzymes suivant : amylase, phytase, phosphatase, xylanase, galactanase, alpha-galactosidase, protéase, phospholipase et/ou bêtaglucanase.

17. Composition selon l'une quelconque des revendications 14 à 16, qui est un additif d'aliment pour animaux.

18. Composition d'aliment pour animaux ayant une teneur en protéines brutes de 50 à 800 g/kg et comprenant le variant de phytase selon l'une quelconque des revendications 1 à 8, ou la composition selon l'une quelconque des revendications 14 à 17.

19. Procédé d'amélioration de la valeur nutritionnelle d'un aliment pour animaux, dans lequel le variant de phytase selon l'une quelconque des revendications 1 à 8, ou la composition selon l'une quelconque des revendications 14 à 17 est ajouté(e) à l'aliment.

20. Procédé pour le traitement de protéines végétales, comprenant l'étape consistant à ajouter le variant de phytase selon l'une quelconque des revendications 1 à 8, ou la composition selon l'une quelconque des revendications 14 à 17 à au moins une protéine végétale ou une source de protéines.

21. Procédé pour augmenter la prise de poids et/ou améliorer le taux de conversion alimentaire d'un animal, le procédé comprenant l'étape consistant à appliquer à l'animal un aliment avec une quantité efficace du variant de phytase selon l'une quelconque des revendications 1 à 8 ou de la composition selon l'une quelconque des revendications 14 à 17.

22. Utilisation du variant de phytase selon l'une quelconque des revendications 1 à 8, ou de la composition selon l'une quelconque des revendications 14 à 17 dans l'aliment pour animaux ; dans la préparation d'aliment pour animaux ; pour améliorer la valeur nutritionnelle de l'aliment pour animaux ; pour réduire les niveaux de phytate dans les déjections animales ; pour le traitement de protéines végétales ; pour libérer du phosphore d'un substrat phytate ; pour augmenter la prise de poids, améliorer le taux de croissance spécifique et/ou améliorer le taux de conversion alimentaire d'un animal ; ou pour améliorer la rétention des éléments nutritifs, et/ou la digestibilité des éléments nutritifs chez un animal.
